# EUROPEAN PATENT APPLICATION

(11) **EP 1 254 885 A1**
(43) Date of publication of application: **06.11.2002**
(21) Application number: 01902770.5
(22) Date of filing: 05.02.2001
(51) Int. Cl.: C07C 43/178, C07C 41/26, C07C 213/00, C07C 215/30, C07C 215/08, C07C 217/48, C07C 317/32, C07F 7/08, C07B 61/00, C07B 53/00

(54) **PROCESS FOR PREPARING OPTICALLY ACTIVE SECONDARY ALCOHOLS HAVING NITROGENOUS OR OXYGENIC FUNCTIONAL GROUPS**

(30) Priority: 08.02.2000 JP 2000000301
(71) Applicant: Asahi Kasei Kabushiki Kaisha, Osaka-shi, Osaka 530-8205 (JP)
(72) Inventor: NAKANO, Seiji, Fuji-shi, Shizuoka 419-0939 (JP); NOYORI, Ryoji, Nisshin-shi, Aichi 470-0132 (JP); OHKUMA, Takeshi, Nagakute-cho, Aichi-gun, Aichi 480-1124 (JP); ISHII, Dai, Fukui-shi, Fukui 910-0017 (JP)
(74) Representative: Forstmeyer, Dietmar, Dr. rer. nat., Dipl.-Chem.
(86) International application number: JP0100797
(87) International publication number: WO01058843

(57) **Abstract**

The present invention provides a process for the preparation of an optically active secondary alcohol represented by the general formula (3): 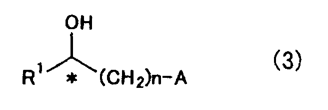 wherein R¹ represents a straight-chain lower alkyl group, an aromatic ring group, or the like; A represents CH₂NR²R³, or the like; n is an integer of from 0 to 2; and * represents an asymmetric carbon atom, which comprises asymmetrically hydrogenating with selectivity among the functional groups, a ketone compound represented by the general formula (1) having a nitrogenous or oxygenic functional group at any of the α-, β- and γ-positions: 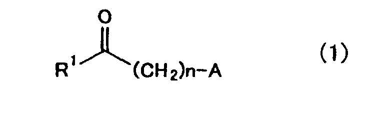 wherein R¹, A and n are as defined above, using a ruthenium/optically active bidentate phosphine/diamine complex as a catalyst in the presence of hydrogen or in the presence of hydrogen and a base.

The optically active secondary alcohols obtained by the present process are useful as medicaments and intermediates for producing medicaments.

## Description

### TITLE OF THE INVENTION

Process for the preparation of optically active secondary alcohols having nitrogenous or oxygenic functional groups

### FIELD OF THE INVENTION

The present invention relates to a novel process of excellent practical use for the preparation of optically active secondary alcohols having a nitrogenous or oxygenic functional group which comprises asymmetrically hydrogenating a ketone compound having a nitrogenous or oxygenic functional group at any of the α-, β- and γ-positions with a ruthenium/optically active bidentate phosphine/diamine complex as a catalyst in the presence of hydrogen or in the presence of hydrogen and a base with good selectivity for the functional groups, good enantioselectivity and high efficient.

### BACKGROUND OF THE INVENTION

Transition metal complexes have been reported as being a useful catalyst for a variety of homogeneous catalytic reactions or heterogeneous catalytic reactions. However, universally applicable, highly practical, highly efficient and highly selective catalysts for hydrogenation reaction of ketone compounds having a nitrogenous or oxygenic functional group have not been developed. A process for preparing alcohol compounds comprising hydrogenating carbonyl compounds with a homogeneous catalyst to yield the corresponding alcohol compounds has been well known. Examples of the process include, for example, the process using a ruthenium complex, described in (Literature 1) Eds. G. Wilkinson, F. G. A. Stone and E. W. Abel, Comprehensive Organometallic Chemistry, Vol. 4, p. 931 (1982); the processes using an rhodium complex, described in (Literature 2) Inorg. Nucl. Chem. Letters, Vol. 12, p. 865 (1976), J. Organomet. Chem., Vol. 129, p. 239 (1977), Chem. Letters, p. 261 (1982), and Tetrahedron Letters*,* Vol. 35, p. 4963 (1994); and the process using a iridium complex, described in (Literature 3) J. Am. Chem. Soc., Vol. 115, p. 3318 (1993).

When viewed from obtaining optically active alcohol compounds, some processes which include a process using an enzyme such as baker's yeast, and a process comprising asymmetrically hydrogenating a carbonyl compound with a metal complex catalyst have been known. With respect to the latter process, many examples of asymmetric catalytic reaction have been reported. The examples include, for example, the process for asymmetrically hydrogenating a functional group-containing carbonyl compound with an optically active ruthenium catalyst, described in detail in (Literature 4) Asymmetric Catalysis in Organic Synthesis, pp. 56-82 (1994) Ed. R. Noyori; the process comprising a hydrogen-transferring reductive reaction using a ruthenium-, rhodium- or iridium-based asymmetric complex catalyst, described in (Literature 5) Chem. Rev., Vol. 92, pp. 1051-1069 (1992); the processes comprising an asymmetric hydrogenation reaction using nickel catalyst modified with tartaric acid, described in (Literature 6) Oil Chemistry, pp. 822-831 (1980) and Ed. Y. Izumi, and Advances in Catalysis, Vol. 32, p. 215 (1983); the process comprising an asymmetric hydrosilylation reaction, described in (Literature 7) Asymmetric Synthesis, Vol. 5, Chap. 4 (1985) Ed. J. D. Morrison, and J. Organomet. Chem., Vol. 346, pp. 413-424 (1988); the processes comprising reducing borane in the presence of an asymmetric ligand, described in (Literature 8) J. Chem. Soc. Perkin Trans. 1, pp. 2039-2044 (1985) and J. Am. Chem. Soc., Vol. 109, pp. 5551-5553 (1987); the process comprising an asymmetric hydrogenation reaction using a ruthenium/optically active phosphine complex, described in (Literature 9) JP-A-4-187685; the process comprising an asymmetric hydrogenation reaction in the presence of phosphine and diamine asymmetric ligand, described in (Literature 10) J. Am. Chem. Soc., Vol. 117, pp. 2675-2676 (1995); the process comprising an asymmetric hydrogenation reaction using a rhodium complex, described in (Literature 11) Tetrahedron Letters, Vol. 40(24), pp. 4551-4554 (1999); and, the process using a cationic ruthenium/BINAP complex, such as [RuI((S)-BINAP) (p-cymene)]⁺I⁻, described in (Literature 12) J. Org. Chem., Vol. 59, pp. 3064-3076 (1994).

However, these conventional processes have a problem that they are not necessarily practically useful due to the facts that they use a noble metal complex catalyst based on a relatively costly metal such as rhodium, iridium, palladium or platinum; have a low hydrogenating activity; and require a relatively high reaction temperature or hydrogen pressure. The process using an enzyme, which can afford an alcohol compound having a relatively high optical purity, has disadvantages that the nature of reaction substrates to be used is limited and that the absolute configuration of alcohol compounds obtained is limited to specific ones.

Further, the conventional process using an asymmetric hydrogenation catalyst based on a transition metal, which can produce optically active alcohol compounds with a high selectivity with respect to the reaction substrates such as β-keto acid derivatives, also has the following drawbacks relating to the catalytic activity. For example, the nature of reaction substrates to be used is limited; a relatively high hydrogen pressure is required when the process is carried out at normal temperature; and a heating treatment is required when the hydrogen pressure is not high. Further, although the process described in the abovementioned Literature 10, which is excellent in the selectivity and activity, it has the following drawbacks. That is, the process needs a complicated handling comprising mixing tree components which are ruthenium phosphine complex having poor long-term storage stability, diamine compound and base, on occasion. Further, when an aminoketone compound is used as the substrate, the decomposition thereof goes ahead of the reaction of interest. Further, the process described in Literature 12, which can reduce 1-dimethyl aminoacetone with a high enantioselectivity, is not necessarily suitable to industrial use from the viewpoint of the catalytic activity due to the following drawbacks. That is, for example, the kinds of substrate ketone compound to be used are limited; the process requires a high pressure of about 100 atm; and a relatively long reaction time is needed.

Consequently, there has been a need for a catalyst having universal applicability, high activity, high functional group-selectivity and high enantioselectivity which can be used to asymmetrically hydrogenate ketone compounds having a nitrogenous or oxygenic functional group at any of the α-, β- and γ-positions to directly produce the corresponding optically active secondary alcohols having a nitrogenous or oxygenic functional group at any of the α-, β- and γ-positions which are widely used as medicaments or an intermediate for producing especially medicaments, or to produce the corresponding optically active secondary alcohols via sequential steps of suitably removing the protecting groups and transforming the functional groups. There has been also a need for a practical process using the above catalyst. An object of the present invention is to provide such a catalyst and a process for producing optically active secondary alcohols having a nitrogenous or oxygenic functional group.

### DISCLOSURE OF THE INVENTION

In order to solve the above problems, the present inventors conducted a variety of studies. As a result, the present inventors have found a characteristic ruthenium/optically active bidentate phosphine/diamine complex which can reductively hydrogenate ketone compounds having a nitrogenous or oxygenic functional group at any of the α-, β- and γ-positions with high selectivity under a mild condition to afford the corresponding optically active secondary alcohols with good enantioselectivity and good yield, and thereby completed the present invention.

That is, the present invention relates to a process for the preparation of an optically active secondary alcohol represented by the general formula (3) having a nitrogenous or oxygenic functional group at any of the α-, β- and γ-positions: 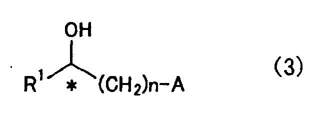 wherein
n is an integer of from 0 to 2;
R¹ represents:
   (a) a straight-chain lower alkyl group,
   (b) an optionally substituted monocyclic aromatic hydrocarbon ring group,
   (c) an optionally substituted fused bicyclic aromatic hydrocarbon ring group,
   (d) an optionally substituted fused tricyclic aromatic hydrocarbon ring group,
   (e) an optionally substituted monocyclic heteroaromatic ring group,
   (f) an optionally substituted fused bicyclic heteroaromatic ring group, or
   (g) an optionally substituted fused tricyclic heteroaromatic ring group;
A represents:
   (a) CH₂NR²R³,
   (b) CH₂OR⁴, or
   (c) CH(OR¹⁵)₂;
R² represents:
   (a) an acyl or alkyloxycarbonyl group,
   (b) an optionally substituted straight-chain alkyl group,
   (c) an optionally substituted branched-chain alkyl group,
   (d) an optionally substituted cyclic alkyl group,
   (e) an optionally substituted alkenyl group,
   (f) an optionally substituted aralkyl group,
   (g) an optionally substituted aryl group,
   (h) a heteroatom-containing saturated carbon chain group,
   (i) a heteroatom-containing unsaturated carbon chain group,
   (j) an optionally substituted heteromonocyclic group,
   (k) an optionally substituted heteropolycyclic group, or
   (l) a composite group consisting of members suitably selected from any of (b) to (k);
when R² is (a) an acyl or alkyloxycarbonyl group among the above groups, R³ represents:
   (a) hydrogen,
   (b) an optionally substituted straight-chain alkyl group,
   (c) an optionally substituted branched-chain alkyl group,
   (d) an optionally substituted cyclic alkyl group,
   (e) an optionally substituted alkenyl group,
   (f) an optionally substituted aralkyl group,
   (g) an optionally substituted aryl group,
   (h) a heteroatom-containing saturated carbon chain group,
   (i) a heteroatom-containing unsaturated carbon chain group,
   (j) an optionally substituted heteromonocyclic group,
   (k) an optionally substituted heteropolycyclic group, or
   (l) a composite group consisting of members suitably selected from any of (b) to (k);
alternatively, when R² is any group other than (a) an acyl or alkyloxycarbonyl group among the above groups, R³ represents:
   (a) an optionally substituted straight-chain alkyl group,
   (b) an optionally substituted branched-chain alkyl group,
   (c) an optionally substituted cyclic alkyl group,
   (d) an optionally substituted alkenyl group,
   (e) an optionally substituted aralkyl group,
   (f) an optionally substituted aryl group,
   (g) a heteroatom-containing saturated carbon chain group,
   (h) a heteroatom-containing unsaturated carbon chain group,
   (i) an optionally substituted heteromonocyclic group,
   (j) an optionally substituted heteropolycyclic group, or
   (k) a composite group consisting of members suitably selected from any of (a) to (j); and R² and R³ may be linked to each other to form a heterocyclic group;
R⁴ represents:
   (a) an optionally substituted straight-chain alkyl group,
   (b) an optionally substituted branched-chain alkyl group,
   (c) an optionally substituted cyclic alkyl group,
   (d) an optionally substituted benzyl group,
   (e) an optionally substituted aralkyl group,
   (f) an optionally substituted aryl group,
   (g) a heteroatom-containing saturated carbon chain group,
   (h) a heteroatom-containing unsaturated carbon chain group,
   (i) an optionally substituted heteromonocyclic group
   (j) an optionally substituted heteropolycyclic group,
   (k) a composite group consisting of members suitably selected from any of (a) to (j), or
   (l) an organosilicon group represented by SIR⁵R⁶R⁷;
R⁵, R⁶ and R⁷ each independently represent:
   (a) a straight-chain or branched-chain lower alkyl group, or
   (b) a phenyl group;
R¹⁵ represents:
   (a) a straight-chain lower alkyl group,
   (b) a branched-chain lower alkyl group,
   (c) a cyclic lower alkyl group,
   (d) an optionally substituted phenyl group,
   (e) an optionally substituted benzyl group, or
   (f) alternatively, two R¹⁵ are bonded to each other to form a cyclic ketal group; and
* represents an asymmetric carbon atom,
which comprises asymmetrically hydrogenating a ketone compound represented by the general formula (1) having a nitrogenous or oxygenic functional group at any of the α-, β- and γ-positions: 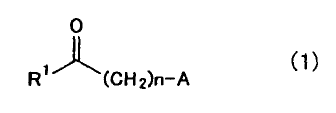 wherein R¹, A and n are as defined above, or a mineral or organic acid salt thereof, with a catalyst in the presence of hydrogen or in the presence of hydrogen and a base, the catalyst being a ruthenium/optically active bidentate phosphine/diamine complex represented by the general formula (2): 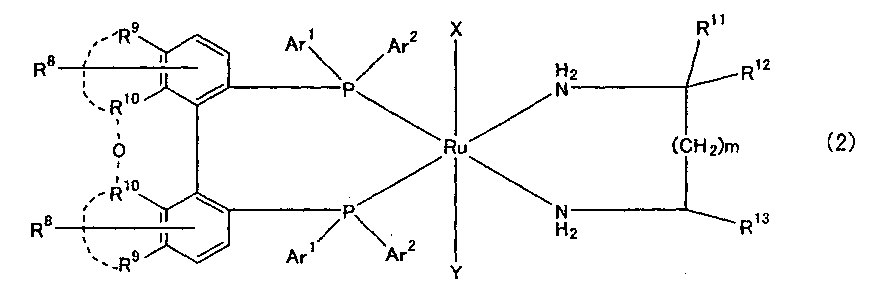 wherein
m is an integer of from 0 to 2;
X and Y may be covalently or ionically bonded to the ruthenium metal, and they independently represent:
   (a) hydrogen,
   (b) halogen,
   (c) an alkoxy group,
   (d) a carboxyl group, or
   (e) other anion radical;
Ar¹ and Ar² independently represent a phenyl group substituted with from zero to five substituents selected from straight-chain or branched-chain lower alkyl group, halogen or lower alkoxy group;
R⁸ represents:
   (a) hydrogen,
   (b) a lower alkyl group,
   (c) a lower alkoxy group, or
   (d) N(R¹⁴)₂ wherein R¹⁴ represents a lower alkyl group;
R⁹ represents:
   (a) hydrogen,
   (b) a lower alkyl group, or
   (c) a lower alkoxy group;
R¹⁰ represents:
   (a) a lower alkyl group, or
   (b) a lower alkoxy group;
   the dashed line linking one R¹⁰ to the other R¹⁰ means that one R¹⁰ may be bonded to the other R¹⁰ via an oxygen atom; one dashed line linking R⁹ to R¹⁰, and the other dashed line linking R⁹ to R¹⁰ independently mean that each pair of R⁹ and R¹⁰ taken together with the benzene ring to which they are attached may form a ring selected from the following rings:
   (a) an optionally substituted tetralin ring,
   (b) an optionally substituted naphthalene ring, and
   (c) an optionally substituted 1,3-benzodioxole ring;
R¹¹ represents:
   (a) hydrogen,
   (b) a straight-chain lower alkyl group,
   (c) a branched-chain lower alkyl group,
   (d) a cyclic lower alkyl group,
   (e) a phenyl group substituted with from zero to five substituents selected from lower alkyl groups or lower alkoxy groups,
   (f) a 1-naphthyl group substituted with from zero to seven substituents selected from lower alkyl groups or lower alkoxy groups, or
   (g) a 2-naphthyl group substituted with from zero to seven substituents selected from lower alkyl groups or lower alkoxy groups;
provided that (I) when R¹¹ is hydrogen, then R¹² and R¹³ are independently represent:
   (a) hydrogen,
   (b) a straight-chain lower alkyl group,
   (c) a branched-chain lower alkyl group,
   (d) a cyclic lower alkyl group,
   (e) a phenyl group substituted with from zero to five substituents selected from lower alkyl groups or lower alkoxy groups,
   (f) a 1-naphthyl group substituted with from zero to seven substituents selected from lower alkyl groups or lower alkoxy groups,
   (g) a 2-naphthyl group substituted with from zero to seven substituents selected from lower alkyl groups or lower alkoxy groups, or
   (h) alternatively, R¹² and R¹³ may be bonded to each other to form a ring selected from:
      (h-1) a cycloalkyl ring, or
      (h-2) a heteroatom-containing heterocyclic ring; or
(II) when R¹¹ is other than hydrogen, then R¹² represents:
   (a) a straight-chain lower alkyl group,
   (b) a branched-chain lower alkyl group,
   (c) a cyclic lower alkyl group,
   (d) a phenyl group substituted with from zero to five substituents selected from lower alkyl groups or lower alkoxy groups,
   (e) a 1-naphthyl group substituted with from zero to seven substituents selected from lower alkyl groups or lower alkoxy groups, or
   (f) a 2-naphthyl group substituted with from zero to seven substituents selected from lower alkyl groups or lower alkoxy groups; and
R¹³ represents:
   (a) hydrogen,
   (b) a lower alkyl group, or
   (c) a benzyl group.

Further, the present invention is also a process for the preparation of an optically active secondary alcohol, wherein R¹ and A in the general formula (1) set forth above comprise no acidic substituents.

Further, the present invention is also a process for the preparation of an optically active secondary alcohol, wherein Ar¹ and Ar² in the general formula (2) set forth above independently represent a phenyl group having from two to five substituents selected from the group consisting of straight-chain or branched-chain lower alkyl groups, halogens, or lower alkoxy groups provided that at least two of the said substituents are straight-chain or branched-chain lower alkyl groups.

Further, the present invention is also a process for the preparation of an optically active secondary alcohol, wherein in the general formula (1) set forth above, n is 0; A is CH₂NR²R³; R¹ is a phenyl group having at 3-position a nitro group, an amino group, or N(CH₂C₆H₅)SO₂R¹⁶ wherein R¹⁶ is a methyl group or a benzyl group, and at 4-position hydrogen, halogen, or a benzyloxy group; R² is an acyl group, an alkyloxycarbonyl group, or a benzyl group; and R³ is an ethyl group which is bonded at its end to the fused tricyclic ring group via an oxygen atom.

Further, the present invention provides a practical process for the preparation of optically active secondary alcohols containing an amino group, optically active diols and optically active hydroxyaldehydes which are useful in a variety of applications, for example, as an intermediate for producing medicaments and pesticides or as medicaments and pesticides per se, wherein the process comprises sequentially or simultaneously removing the nitrogen- and oxygen-protecting groups of the optically active secondary alcohols obtained by the preparing process set forth above.

Further, the present invention also provides a process for the preparation of an optically active secondary alcohol containing an amino group, wherein the process comprises converting a terminal primary hydroxyl group of an optically active diol into a leaving group by a conventional method; when the diol is an optically active 1,2-diol, optionally converting it into an optically active epoxy compound; and reacting the reaction product with a primary or secondary amine compound.

Further, the present invention also provides a short process for the preparation of an optically active secondary alcohol containing an amino group, wherein the process comprises converting an optically active secondary alcohol having a terminal aldehyde group protected as ketal into the corresponding optically active imino alcohol by deprotecting the aldehyde group followed by a reaction with a primary amine compound; and subjecting the thus obtained imino alcohol to a conventional imine-reducing reaction.

Examples of such an optically active secondary alcohol containing an amino group which is particularly important as an medicament, may be mentioned as follows. For example, examples of the alcohol compound having an amino group at the α-position include adrenaline derivatives having an activity as a sympathetic receptor agonist or antagonist, such as an antiasthmatic agent acting on the bronchial smooth muscle and a vasopressor acting on the heart. Compounds disclosed in WO 97/25311 and WO 99/01431 are described therein as being very useful for treating and preventing diabetes, obesity, hyperlipidemia and the like. Examples of the alcohol compound having an amino group at the β-position include compounds acting on the central nervous system, such as trihexyphenidyl hydrochloride which is an antiparkinson agent. Examples of the alcohol compound having an amino group at the γ-position include nonsedating histamine H₁-receptor antagonists (allergic disease-treating agents) such as Terfenadine, antipsychotic agents such as BMS 181100 (described in the literature: J. Med. Chem., Vol. 35, 4516-4525 (1992)), and drugs acting on the central nervous system.

The disclosures in the text of specification and/or drawings of Japanese Patent Application No. 2000-30127, from which the present application claims the priority right, is incorporated herein.

### PREFERRED EMBODIMENTS OF THE INVENTION

The present invention has the technical features set forth above. Preferred embodiments of the present invention will be described below in more detail. As a reaction substrate, ketone compounds having a nitrogenous or oxygenic functional group at any of the α-, β- and γ-positions are represented by the general formula (1) wherein n represents an integer of from 0 to 2, with 0 being preferred.

R¹ represents:
(a) a straight-chain lower alkyl group,
(b) an optionally substituted monocyclic aromatic hydrocarbon ring group,
(c) an optionally substituted fused bicyclic aromatic hydrocarbon ring group,
(d) an optionally substituted fused tricyclic aromatic hydrocarbon ring group,
(e) an optionally substituted monocyclic heteroaromatic ring group,
(f) an optionally substituted fused bicyclic heteroaromatic ring group, or
(g) an optionally substituted fused tricyclic heteroaromatic ring group.

With respect to the groups represented by R¹, (a) a straight-chain lower alkyl group means a straight-chain alkyl group having from one to six carbon atoms. "Optionally substituted group" referred to in (b) to (g) means a group optionally substituted with from one to nine substituents which may be the same or different from each other.

The substituents include alkyl group, alkoxy group, halogen, alkenyl group, nitro group, amino group, acylated amino group, alkylsulfonylated amino group, and cyano group. The substituents preferably comprise no acidic functional groups such as phenolic hydroxyl group and carboxylic acid group.

Examples of (a) a straight-chain lower alkyl group include methyl group, ethyl group, n-propyl group, n-butyl group, n-pentyl group, and n-hexyl group, with methyl group being preferred.

Examples of (b) an optionally substituted monocyclic aromatic hydrocarbon ring group include 3-aminophenyl group, 3-nitrophenyl group, 3-[(benzyl)(methylsulfonyl)amino]phenyl group, 3-[(benzyl)(benzylsulfonyl)amino]phenyl group, 4-methoxyphenyl group, 4-nitrophenyl group, 4-chlorophenyl group, 4-bromophenyl group, 4-benzyloxyphenyl group, 3,4-dichlorophenyl group, 3,4-dibromophenyl group, 4-chloro-3-aminophenyl group, 4-chloro-3-[(benzyl) (methylsulfonyl)amino]phenyl group, 4-chloro-3-[(benzyl](benzylsulfonyl)amino]phenyl group, 4-chloro-3-nitrophenyl group, 4-bromo-3-aminophenyl group, 4-bromo-3-[(benzyl)(methylsulfonyl)-amino]phenyl group, 4-bromo-3-[(benzyl)(benzylsulfonyl)amino]phenyl group, 4-bromo-3-nitrophenyl group, 4-benzyloxy-3-nitrophenyl group, 4-benzyloxy-3-aminophenyl group, 4-benzyloxy-3-[(benzyl) (methylsulfonyl)amino]phenyl group, 4-benzyloxy-3-[(benzyl)(benzylsulfonyl)amino]phenyl group, 4-benzyloxy-3-[(hydroxy)methyl]phenyl group, and xylyl group.

More preferred examples of (b) include 3-aminophenyl group, 3-nitrophenyl group, 3-[(benzyl)(methylsulfonyl)amino]phenyl group, 4-methoxyphenyl group, 4-benzyloxyphenyl group, 4-chloro-3-aminophenyl group, 4-chloro-3-[(benzyl) (methylsulfonyl)amino]phenyl group, 4-chloro-3-nitrophenyl group, 4-bromo-3-aminophenyl group, 4-bromo-3-[(benzyl) (methylsulfonyl)amino]phenyl group, 4-bromo-3-nitrophenyl group, 4-benzyloxy-3-nitrophenyl group, 4-benzyloxy-3-aminophenyl group, 4-benzyloxy-3-[(benzyl) (methylsulfonyl)amino]phenyl group, and 4-benzyloxy-3-[(hydroxy)methyl]phenyl group.

Examples of (c) an optionally substituted fused bicyclic aromatic hydrocarbon ring group include 1-naphthyl group, 2-naphthyl group, 2-indenyl group, 3-indenyl group, 4-indenyl group, 5-indenyl group, 6-indenyl group, 7-indenyl group and the like, with 1-naphthyl group and 2-naphthyl group being more preferred.

Examples of (d) an optionally substituted fused tricyclic aromatic hydrocarbon ring group include anthracenyl group, phenanthrenyl group, fluorenyl group and the like.

Examples of (e) an optionally substituted monocyclic heteroaromatic ring group include 2-thienyl group, 3-thienyl group, 2-furyl group, 3-furyl group, 3-pyrrolyl group, 3-pyrazolyl group, 4-pyrazolyl group, 4-imidazolyl group, oxazolyl group, isoxazolyl group, thiazolyl group, 3-pyranyl group, 4-pyranyl group, 5-pyranyl group, 6-pyranyl group, pyridyl group, pyridazinyl group, pyrimidinyl group, pyrazinyl group, oxazinyl group, triazinyl group and the like, with 3-furyl group and pyridyl group being more preferred.

Examples of (f) an optionally substituted fused bicyclic heteroaromatic ring group include xanthenyl group, 3-indolyl group, 4-indolyl group, 5-indolyl group, 6-indolyl group, 7-indolyl group, 4-isoindolyl group, 5-isoindolyl group, 6-isoindolyl group, 7-isoindolyl group, 4-benzimidazolyl group, 5-benzimidazolyl group, 6-benzimidazolyl group, 7-benzimidazolyl group, benzofuranyl group, 4-dihydrobenzofuranyl group, 5-dihydrobenzofuranyl group, 6-dihydrobenzofuranyl group, 7-dihydrobenzofuranyl group, thionaphthenyl group, benzoxazolyl group, quinolyl group, isoquinolyl group, quinolizinyl group, quinoxalinyl group, phthalazinyl group, naphthyridinyl group and the like.

Preferred examples include 3-indolyl group, 4-indolyl group, 6-indolyl group, 4-benzimidazolyl group, 5-benzimidazolyl group, 6-benzimidazolyl group, 7-benzimidazolyl group, 4-benzofuranyl group, 5-benzofuranyl group, 6-benzofuranyl group, 4-dihydrobenzofuranyl group, 5-dihydrobenzofuranyl group, 6-dihydrobenzofuranyl group, 4-benzoxazolyl group, 5-benzoxazolyl group, 6-benzoxazolyl group, 7-benzoxazolyl group, and quinoxalinyl group.

More preferred examples include 4-indolyl group, 6-indolyl group, 4-benzimidazolyl group, 5-benzimidazolyl group, 6-benzimidazolyl group, 4-benzoxazolyl group, and 6-benzoxazolyl group.

Examples of (g) an optionally substituted fused tricyclic heteroaromatic ring group include acridinyl group, thianthracenyl group, phenoxazinyl group, phenothiazinyl group, carbazolyl group, dibenzofuranyl group, and dibenzothiophenyl group, with acridinyl group and phenoxazinyl group being preferred.

Among the abovementioned groups, R¹ is preferably:
(b) an optionally substituted monocyclic aromatic hydrocarbon ring group,
(c) an optionally substituted fused bicyclic aromatic hydrocarbon ring group,
(e) an optionally substituted monocyclic heteroaromatic ring group, or
(f) an optionally substituted fused bicyclic heteroaromatic ring group;
more preferably:
(b) an optionally substituted monocyclic aromatic hydrocarbon ring group,
(c) an optionally substituted fused bicyclic aromatic hydrocarbon fused ring group, or
(f) an optionally substituted fused bicyclic heteroaromatic ring group; and
still more preferably:
(b) an optionally substituted monocyclic aromatic hydrocarbon ring group, or
(f) an optionally substituted fused bicyclic heteroaromatic fused ring group.

A represents (a) CH₂NR²R³, (b) CH₂OR⁴, or (c) CH(OR¹⁵)₂, with (a) CH₂NR²R³ being preferred.
R² represents:
(a) an acyl or alkyloxycarbonyl group,
(b) an optionally substituted straight-chain alkyl group,
(c) an optionally substituted branched-chain alkyl group,
(d) an optionally substituted cyclic alkyl group,
(e) an optionally substituted alkenyl group,
(f) an optionally substituted aralkyl group,
(g) an optionally substituted aryl group,
(h) a heteroatom-containing saturated carbon chain group,
(i) a heteroatom-containing unsaturated carbon chain group,
(j) an optionally substituted heteromonocyclic group,
(k) an optionally substituted heteropolycyclic group, or
(l) a composite group consisting of members suitably selected from any of (b) to (k).

With respect to the groups represented by R², "optionally substituted group" referred to in (b) to (g), (j) and (k) means a group optionally substituted with from one to twenty substituents which may be the same or different from each other, preferably a group optionally substituted with from one to nine substituents which may be the same or different from each other, and more preferably a group optionally substituted with from one to five substituents which may be the same or different from each other.

The substituents may include alkyl group, alkoxy group, halogen, alkenyl group, nitro group, amino group, acylated amino group, alkylsulfonylated amino group, cyano group, hydroxyl group protected with silyl group, and aryloxy group. The substituents preferably comprise no acidic functional groups such as phenolic hydroxyl group and carboxylic acid group.

Members of (a) an acyl group and alkyloxycarbonyl group are not limited as long as they are those commonly used, and preferred examples include acyl groups derived from alkylcarboxylic acid, such as formyl group, acetyl group, propionyl group, butyryl group, pentanoyl group, hexanoyl group, trifluoroacetyl group, and pivaloyl group; acyl groups derived from aromatic carboxylic acid, such as benzoyl group, 4-methoxybenzoyl group, 2,4,6-trimethylbenzoyl group, naphthoyl group and the like; and carbamate type protecting groups, such as tert-butoxycarbonyl group, benzyloxycarbonyl group, p-methoxybenzylcarbonyl group, 2,2,2-trichloroethoxycarbonyl group and the like. More preferred examples include acetyl group, benzoyl group, and tert-butoxycarbonyl group.

Examples of (b) an optionally substituted straight-chain alkyl group include methyl group, ethyl group, propyl group, butyl group, pentyl group, hexyl group and the like, with methyl group being preferred.

Examples of (c) an optionally substituted branched-chain alkyl group include isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group and the like, with isopropyl group, tert-butyl group, cyclopentylmethyl group, and cyclohexylmethyl group being preferred.

Examples of (d) an optionally substituted cyclic alkyl group include cyclopropyl group, cyclobutyl group, cyclopentyl group, cyclohexyl group, cycloheptyl group and the like, with cyclopropyl group being preferred.

Examples of (e) an optionally substituted alkenyl group include allyl group and vinyl group, with allyl group being preferred.

Examples of (f) an optionally substituted aralkyl group include benzyl group, p-methoxybenzyl group, p-nitrobenzyl group and the like, with benzyl group and p-methoxybenzyl group being preferred.

Examples of (g) an optionally substituted aryl group include phenyl group, substituted phenyl group, naphthyl group, substituted naphthyl group, anthryl group, substituted anthryl group, phenanthryl group, substituted phenanthryl group, indenyl group, substituted indenyl group, fluorenyl group, substituted fluorenyl group and the like, with phenyl group, substituted phenyl group, naphthyl group, and substituted naphthyl group being preferred.

Examples of (h) an heteroatom-containing saturated carbon chain group include:
(h-1) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group, and the like which are substituted directly with an optionally substituted heteroaromatic ring group;
(h-2) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group, and the like which are substituted with a heteroatom existing as a substituent on an optionally substituted heteroaromatic ring group;
(h-3) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group, and the like which are substituted directly with an optionally substituted saturated heterocyclic ring group; and
(h-4) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentyl-methyl group, cyclopentylethyl group, cyclohexylmethyl group, cyclohexylethyl group, and the like which are substituted with a heteroatom existing as a substituent on an optionally substituted saturated heterocyclic ring group.

A heteroatom-containing saturated carbon chain group as (h) is preferably selected from (h-1) and (h-2), and more preferably from (h-2).

In (h-2), the heteroatom-containing saturated carbon chain group is preferably methyl, ethyl, propyl, butyl, pentyl or hexyl group substituted with a heteroatom existing as a substituent on an optionally substituted heteroaromatic ring group, and more preferably methyl or ethyl group substituted with a heteroatom existing as a substituent on an optionally substituted hetero-aromatic ring group. In addition, "heteroatom existing as a substituent" is preferably oxygen atom or nitrogen atom, and more preferably oxygen atom.

With respect to (h-1) and (h-2), examples of "optionally substituted heteroaromatic ring group" include thienyl group, furyl group, pyrrolyl group, pyrazolyl group, imidazolyl group, oxazolyl group, isoxazolyl group, thiazolyl group, pyridyl group, pyridazinyl group, pyrimidinyl group, pyrazinyl group, oxazinyl group, triazinyl group, indolyl group, isoindolyl group, benzimidazolyl group, benzofuranyl group, dihydrobenzofuranyl group, thionaphthenyl group, dihydrothionaphthenyl group, benzoxazolyl group, quinolyl group, isoquinolyl group, quinolizinyl group, quinoxalinyl group, phthalazinyl group, naphthyridinyl group, acridinyl group, phenoxazinyl group, phenothiazinyl group, purinyl group, xanthinyl group, pteridinyl group, carbazolyl group, dibenzofuranyl group, dibenzothiophenyl group, tetrahydrocarbazolyl group, tetrahydrodibenzofuranyl group, tetrahydrodibenzothiophenyl group and the like. Preferred examples include indolyl group, benzimidazolyl group, benzofuranyl group, benzoxazolyl group, quinolyl group, isoquinolyl group, phenoxazinyl group, phenothiazinyl group, carbazolyl group, dibenzofuranyl group, dibenzothiophenyl group, tetrahydro-carbazolyl group, tetrahydrodibenzofuranyl group, and tetrahydrodibenzothiophenyl group. More preferred examples include carbazolyl group, dibenzofuranyl group, dibenzothiophenyl group, tetrahydrocarbazolyl group, tetrahydrodibenzofuranyl group, and tetrahydrodibenzothiophenyl group.

With respect to (h-3) and (h-4), examples of "optionally substituted saturated heterocyclic ring group" include tetrahydrofuranyl group, tetrahydrothienyl group, pyrrolidinyl group, pyrazolonyl group, isoimidazolyl group, imidazolinyl group, imidazolidinyl group, oxazolinyl group, oxazolidinyl group, pyranyl group, thiazinyl group, orthooxazinyl group, oxazinyl group, piperidyl group, piperazinyl group, homopiperazinyl group, dioxanyl group, morpholinyl group, thioxanyl group, thiomorpholinyl group and the like, with pyrrolidinyl group, piperazinyl group, homopiperazinyl group, dioxanyl group, and morpholinyl group being preferred.

In addition, with respect to (h-2) and (h-4), "heteroatom existing as a substituent" may be oxygen atom, nitrogen atom, or sulfur atom, with oxygen atom and nitrogen atom being more preferred.

Examples of (i) a heteroatom-containing unsaturated carbon chain group include:
(i-1) ethylene group, propylene group, butenyl group, pentenyl group, hexenyl group, and the like which are substituted directly with an optionally substituted heteroaromatic ring group;
(i-2) ethylene group, propylene group, butenyl group, pentenyl group, hexenyl group, and the like which are substituted with a heteroatom existing as a substituent on an optionally substituted hetero-aromatic ring group;
(i-3) ethylene group, propylene group, butenyl group, pentenyl group, hexenyl group, and the like which are substituted directly with an optionally substituted saturated heterocyclic ring group; and
(i-4) ethylene group, propylene group, butenyl group, pentenyl group, hexenyl group, and the like which are substituted with a heteroatom existing as a substituent on an optionally substituted saturated heterocyclic ring group.

A heteroatom-containing unsaturated carbon chain group as (i) is preferably selected from (i-1) and (i-3), and more preferably from (i-1).

In (i-1), the heteroatom-containing unsaturated carbon chain group is preferably ethylene or propylene group substituted directly with an optionally substituted heteroaromatic ring group. With respect to (i-1) and (i-2), examples of "optionally substituted heteroaromatic ring group" include thienyl group, furyl group, pyrrolyl group, pyrazolyl group, imidazolyl group, oxazolyl group, isoxazolyl group, thiazolyl group, pyridyl group, pyridazinyl group, pyrimidinyl group, pyrazinyl group, oxazinyl group, triazinyl group, indolyl group, isoindolyl group, benzimidazolyl group, benzofuranyl group, dihydrobenzofuranyl group, thionaphthenyl group, dihydrothionaphthenyl group, benzoxazolyl group, quinolyl group, isoquinolyl group, quinolizinyl group, quinoxalinyl group, phthalazinyl group, naphthyridinyl group, acridinyl group, phenoxazinyl group, phenothiazinyl group, purinyl group, xanthinyl group, pteridinyl group, carbazolyl group, dibenzofuranyl group, dibenzothiophenyl group, tetrahydrocarbazolyl group, tetrahydrodibenzofuranyl group, tetrahydrodibenzothiophenyl group and the like. Preferred examples include indolyl group, benzimidazolyl group, benzofuranyl group, benzoxazolyl group, quinolyl group, isoquinolyl group, phenoxazinyl group, phenothiazinyl group, carbazolyl group, dibenzofuranyl group, dibenzothiophenyl group, tetrahydro-carbazolyl group, tetrahydrodibenzofuranyl group, and tetrahydrodibenzothiophenyl group. More preferred examples include carbazolyl group, dibenzofuranyl group, dibenzothiophenyl group, tetrahydrocarbazolyl group, tetrahydrodibenzofuranyl group, and tetrahydrodibenzothiophenyl group.

With respect to (i-3) and (i-4), examples of "optionally substituted saturated heterocyclic ring group" include tetrahydrofuranyl group, tetrahydrothienyl group, pyrrolidinyl group, pyrazolonyl group, isoimidazolyl group, imidazolinyl group, imidazolidinyl group, oxazolinyl group, oxazolidinyl group, pyranyl group, thiazinyl group, orthooxazinyl group, oxazinyl group, piperidyl group, piperazinyl group, homopiperazinyl group, dioxanyl group, morpholinyl group, thioxanyl group, thiomorpholinyl group and the like, with pyrrolidinyl group, piperazinyl group, homopiperazinyl group, dioxanyl group, and morpholinyl group being preferred.

In addition, with respect to (i-2) and (i-4), "heteroatom existing as a substituent" may be oxygen atom, nitrogen atom, or sulfur atom, with oxygen atom and nitrogen atom being more preferred.

Examples of (j) an optionally substituted heteromonocyclic group include thienyl group, furyl group, pyrrolyl group, pyrazolyl group, imidazolyl group, oxazolyl group, isoxazolyl group, thiazolyl group, pyridyl group, pyridazinyl group, pyrimidinyl group, pyrazinyl group, oxazinyl group, triazinyl group, tetrahydrofuranyl group, tetrahydrothienyl group, pyrrolidinyl group, pyrazolonyl group, isoimidazolyl group, imidazolinyl group, imidazolidinyl group, oxazolinyl group, oxazolidinyl group, pyranyl group, thiazinyl group, orthooxazinyl group, oxazinyl group, piperidyl group, piperazinyl group, homopiperazinyl group, dioxanyl group, morpholinyl group, thioxanyl group, thiomorpholinyl group and the like, with furyl group, pyrrolyl group, and pyridyl group being preferred.

Examples of (k) an optionally substituted heteropolycyclic group include indolyl group, isoindolyl group, benzimidazolyl group, benzofuranyl group, dihydrobenzofuranyl group, thionaphthenyl group, dihydrothionaphthenyl group, benzoxazolyl group, quinolyl group, isoquinolyl group, quinolizinyl group, quinoxalinyl group, phthalazinyl group, naphthyridinyl group, acridinyl group, phenoxazinyl group, phenothiazinyl group, purinyl group, xanthinyl group, pteridinyl group, carbazolyl group, dibenzofuranyl group, dibenzothiophenyl group, tetrahydrocarbazolyl group, tetrahydrodibenzofuranyl group, tetrahydrodibenzothiophenyl group and the like. Preferred examples include indolyl group, benzimidazolyl group, benzofuranyl group, benzoxazolyl group, quinolyl group, isoquinolyl group, phenoxazinyl group, phenothiazinyl group, carbazolyl group, dibenzofuranyl group, dibenzothiophenyl group, tetrahydrocarbazolyl group, tetrahydrodibenzofuranyl group, and tetrahydrodibenzothiophenyl group.

Among the abovementioned groups, R² is preferably:
(a) an acyl or alkyloxycarbonyl group,
(b) an optionally substituted straight-chain alkyl group,
(c) an optionally substituted branched-chain alkyl group,
(d) an optionally substituted cyclic alkyl group,
(e) an optionally substituted alkenyl group,
(f) an optionally substituted aralkyl group,
(g) an optionally substituted aryl group, or
(h) a heteroatom-containing saturated carbon chain group;
more preferably:
(a) an acyl or alkyloxycarbonyl group,
(b) an optionally substituted straight-chain alkyl group,
(c) an optionally substituted branched-chain alkyl group,
(e) an optionally substituted alkenyl group, or
(f) an optionally substituted aralkyl group; and
still more preferably:
(a) an acyl or alkyloxycarbonyl group, or
(f) an optionally substituted aralkyl group.
When (I) R² is (a) an acyl or alkyloxycarbonyl group among the above groups, R³ represents:
(Ia) hydrogen,
(Ib) an optionally substituted straight-chain alkyl group,
(Ic) an optionally substituted branched-chain alkyl group,
(Id) an optionally substituted cyclic alkyl group,
(Ie) an optionally substituted alkenyl group,
(If) an optionally substituted aralkyl group,
(Ig) an optionally substituted aryl group,
(Ih) a heteroatom-containing saturated carbon chain group,
(Ii) a heteroatom-containing unsaturated carbon chain group,
(Ij) an optionally substituted heteromonocyclic group,
(Ik) an optionally substituted heteropolycyclic group, or
(Il) a composite group consisting of members suitably selected from any of (Ib) to (Ik); or
alternatively, when (II) R² is any group other than (a) an acyl or alkyloxycarbonyl group among the above groups, R³ represents:
(IIb) an optionally substituted straight-chain alkyl group,
(IIc) an optionally substituted branched-chain alkyl group,
(IId) an optionally substituted cyclic alkyl group,
(IIe) an optionally substituted alkenyl group,
(IIf) an optionally substituted aralkyl group,
(IIg) an optionally substituted aryl group,
(IIh) a heteroatom-containing saturated carbon chain group,
(IIi) a heteroatom-containing unsaturated carbon chain group,
(IIj) an optionally substituted heteromonocyclic group,
(IIk) an optionally substituted heteropolycyclic group, or
(IIl) a composite group consisting of members suitably selected from any of (IIb) to (IIk); and R² and R³ may be linked to each other to form a heterocyclic group. The substituents set forth above preferably comprise no acidic functional groups such as phenolic hydroxyl group and carboxylic acid group.

With respect to the groups represented by R³, "optionally substituted group" referred to in (Ib) to (Ig), (Ij), (Ik), (IIb) to (IIg), (IIj) and (IIk) means a group optionally substituted with from one to twenty substituents which may be the same or different from each other, preferably a group optionally substituted with from one to nine substituents which may be the same or different from each other, and more preferably a group optionally substituted with from one to five substituents which may be the same or different from each other.

The substituents may include alkyl group, alkoxy group, halogen, alkenyl group, nitro group, amino group, acylated amino group, alkylsulfonylated amino group, cyano group, hydroxyl group protected with silyl group, aryloxy group and the like. The substituents preferably comprise no acidic functional groups such as phenolic hydroxyl group and carboxylic acid group.

Examples of (Ib) and (IIb) an optionally substituted straight-chain alkyl group include optionally substituted methyl, ethyl, propyl, butyl, pentyl, hexyl groups and the like, with optionally substituted methyl and ethyl groups being preferred. Unsubstituted methyl and ethyl groups are also preferred.

Examples of (Ic) and (IIc) an optionally substituted branched-chain alkyl group include isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group and the like, with isopropyl group, tert-butyl group, cyclopentylmethyl group, and cyclohexylmethyl group being preferred.

Examples of (Id) and (IId) an optionally substituted cyclic alkyl group include cyclopropyl group, cyclobutyl group, cyclopentyl group, cyclohexyl group, cycloheptyl group and the like, with cyclopropyl group being preferred.

Examples of (Ie) and (IIe) an optionally substituted alkenyl group include allyl group, vinyl group and the like, with allyl group being preferred.

Examples of (If) and (IIf) an optionally substituted aralkyl group include benzyl group, p-methoxybenzyl group, p-nitrobenzyl group and the like, with benzyl group and p-methoxybenzyl group being preferred.

Examples of (Ig) and (IIg) an optionally substituted aryl group include phenyl group, substituted phenyl group, naphthyl group, substituted naphthyl group, anthryl group, substituted anthryl group, phenanthryl group, substituted phenanthryl group, indenyl group, substituted indenyl group, fluorenyl group, substituted fluorenyl group and the like, with phenyl group, substituted phenyl group, naphthyl group, and substituted naphthyl group being preferred.

Examples of (Ih) and (IIh) a heteroatom-containing saturated carbon chain group include:
(h-1) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group and the like which are substituted directly with an optionally substituted heteroaromatic ring group;
(h-2) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group and the like which are substituted with a heteroatom existing as a substituent on an optionally substituted heteroaromatic ring group;
(h-3) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, cyclohexylethyl group and the like which are substituted directly with an optionally substituted saturated heterocyclic ring group; and
(h-4) methyl group, ethyl group, propyl group, butyl group, pentyl group, hexyl group, isopropyl group, isobutyl group, sec-butyl group, tert-butyl group, cyclopropylmethyl group, cyclopropylethyl group, cyclobutylmethyl group, cyclobutylethyl group, cyclopentylmethyl group, cyclopentylethyl group, cyclohexylmethyl group, and cyclohexylethyl group and the like which are substituted with a heteroatom existing as a substituent on an optionally substituted saturated heterocyclic ring.

With respect to (h-1) and (h-2), examples of "optionally substituted heteroaromatic ring group" include thienyl group, furyl group, pyrrolyl group, pyrazolyl group, imidazolyl group, oxazolyl group, isoxazolyl group, thiazolyl group, pyridyl group, pyridazinyl group, pyrimidinyl group, pyrazinyl group, oxazinyl group, triazinyl group, indolyl group, isoindolyl group, benzimidazolyl group, benzofuranyl group, dihydrobenzofuranyl group, thionaphthenyl group, dihydrothionaphthenyl group, benzoxazolyl group, quinolyl group, isoquinolyl group, quinolizinyl group, quinoxalinyl group, phthalazinyl group, naphthyridinyl group, acridinyl group, phenoxazinyl group, phenothiazinyl group, purinyl group, xanthinyl group, pteridinyl group, carbazolyl group, dibenzofuranyl group, dibenzothiophenyl group, tetrahydrocarbazolyl group, tetrahydrodibenzofuranyl group, tetrahydrodibenzothiophenyl group and the like. Preferred examples include indolyl group, benzimidazolyl group, benzofuranyl group, benzoxazolyl group, quinolyl group, isoquinolyl group, phenoxazinyl group, phenothiazinyl group, carbazolyl group, dibenzofuranyl group, dibenzothiophenyl group, tetrahydrocarbazolyl group, tetrahydrodibenzofuranyl group, and tetrahydrodibenzothiophenyl group.

With respect to (h-3) and (h-4), examples of "optionally substituted saturated heterocyclic ring group" include tetrahydrofuranyl group, tetrahydrothienyl group, pyrrolidinyl group, pyrazolonyl group, isoimidazolyl group, imidazolinyl group, imidazolidinyl group, oxazolinyl group, oxazolidinyl group, pyranyl group, thiazinyl group, orthooxazinyl group, oxazinyl group, piperidyl group, piperazinyl group, homopiperazinyl group, dioxanyl group, morpholinyl group, thioxanyl group, thiomorpholinyl group and the like, with pyrrolidinyl group, piperazinyl group, homopiperazinyl group, dioxanyl group, and morpholinyl group being preferred.

With respect to (h-2) and (h-4), "heteroatom existing as a substituent" may be oxygen atom, nitrogen atom, or sulfur atom, with oxygen atom and nitrogen atom being preferred.

A heteroatom-containing saturated carbon chain group as (Ih) and (IIh) is preferably selected from (h-1) and (h-2), and more preferably from (h-2).

In (h-2), the optionally substituted heteroaromatic ring group may be particularly preferably carbazolyl group, dibenzofuranyl group, dibenzothiophenyl group, tetrahydrocarbazolyl group, tetrahydrodibenzofuranyl group, or tetrahydrodibenzothiophenyl group. In addition, preferred examples of the saturated carbon chain group substituted with a heteroatom existing as a substituent on an optionally substituted heteroaromatic ring group include methyl group, ethyl group, propyl group, butyl group, pentyl group, and hexyl group, with methyl group and ethyl group being more preferred. Most preferably, the saturated carbon chain group is ethyl group. Further, "heteroatom existing as a substituent" is preferably oxygen atom or nitrogen atom, and more preferably oxygen atom.

Examples of (Ii) and (IIi) a heteroatom-containing unsaturated carbon chain group include:
(i-1) ethylene group, propylene group, butenyl group, pentenyl group, hexenyl group and the like which are substituted directly with an optionally substituted heteroaromatic ring group;
(i-2) ethylene group, propylene group, butenyl group, pentenyl group, hexenyl group and the like which are substituted with a heteroatom existing as a substituent on an optionally substituted heteroaromatic ring group;
(i-3) ethylene group, propylene group, butenyl group, pentenyl group, hexenyl group and the like which are substituted directly with an optionally substituted saturated heterocyclic ring group; and
(i-4) ethylene group, propylene group, butenyl group, pentenyl group, hexenyl group and the like which are substituted with a heteroatom existing as a substituent on an optionally substituted saturated heterocyclic ring.

A heteroatom-containing unsaturated carbon chain group as (Ii) and (IIi) is preferably selected from (i-1) and (i-3), and more preferably from (i-1).

In (i-1), the heteroatom-containing unsaturated carbon chain group is preferably ethylene or propylene group substituted directly with an optionally substituted heteroaromatic ring group.

With respect to (i-1) and (i-2), examples of "optionally substituted heteroaromatic ring group" include thienyl group, furyl group, pyrrolyl group, pyrazolyl group, imidazolyl group, oxazolyl group, isoxazolyl group, thiazolyl group, pyridyl group, pyridazinyl group, pyrimidinyl group, pyrazinyl group, oxazinyl group, triazinyl group, indolyl group, isoindolyl group, benzimidazolyl group, benzofuranyl group, dihydrobenzofuranyl group, thionaphthenyl group, dihydrothionaphthenyl group, benzoxazolyl group, quinolyl group, isoquinolyl group, quinolizinyl group, quinoxalinyl group, phthalazinyl group, naphthyridinyl group, acridinyl group, phenoxazinyl group, phenothiazinyl group, purinyl group, xanthinyl group, pteridinyl group, carbazolyl group, dibenzofuranyl group, dibenzothiophenyl group, tetrahydrocarbazolyl group, tetrahydrodibenzofuranyl group, tetrahydrodibenzothiophenyl group and the like. Preferred examples of "optionally substituted heteroaromatic ring group" include indolyl group, benzimidazolyl group, benzofuranyl group, benzoxazolyl group, quinolyl group, isoquinolyl group, phenoxazinyl group, phenothiazinyl group, carbazolyl group, dibenzofuranyl group, dibenzothiophenyl group, tetrahydrocarbazolyl group, tetrahydrodibenzofuranyl group, and tetrahydrodibenzothiophenyl group. More preferred examples include carbazolyl group, dibenzofuranyl group, dibenzothiophenyl group, tetrahydrocarbazolyl group, tetrahydrodibenzofuranyl group, and tetrahydrodibenzothiophenyl group.

With respect to (i-3) and (i-4), examples of "optionally substituted saturated heterocyclic ring group" include tetrahydrofuranyl group, tetrahydrothienyl group, pyrrolidinyl group, pyrazolonyl group, isoimidazolyl group, imidazolinyl group, imidazolidinyl group, oxazolinyl group, oxazolidinyl group, pyranyl group, thiazinyl group, orthooxazinyl group, oxazinyl group, piperidyl group, piperazinyl group, homopiperazinyl group, dioxanyl group, morpholinyl group, thioxanyl group, thiomorpholinyl group and the like, with pyrrolidinyl group, piperazinyl group, homopiperazinyl group, dioxanyl group, and morpholinyl group being preferred.

With respect to (i-2) and (i-4), "heteroatom existing as a substituent" may be oxygen atom, nitrogen atom, or sulfur atom, with oxygen atom and nitrogen atom being preferred.

Examples of (Ij) and (IIj) "optionally substituted heteromonocyclic group" include thienyl group, furyl group, pyrrolyl group, pyrazolyl group, imidazolyl group, oxazolyl group, isoxazolyl group, thiazolyl group, pyridyl group, pyridazinyl group, pyrimidinyl group, pyrazinyl group, oxazinyl group, triazinyl group, tetrahydrofuranyl group, tetrahydrothienyl group, pyrrolidinyl group, pyrazolonyl group, isoimidazolyl group, imidazolinyl group, imidazolidinyl group, oxazolinyl group, oxazolidinyl group, pyranyl group, thiazinyl group, orthooxazinyl group, oxazinyl group, piperidyl group, piperazinyl group, homopiperazinyl group, dioxanyl group, morpholinyl group, thioxanyl group, thiomorpholinyl group and the like, with furyl group, pyrrolyl group, and pyridyl group being preferred.

Examples of (Ik) and (IIk) "optionally substituted heteropolycyclic group" include indolyl group, isoindolyl group, benzimidazolyl group, benzofuranyl group, dihydrobenzofuranyl group, thionaphthenyl group, dihydrothionaphthenyl group, benzoxazolyl group, quinolyl group, isoquinolyl group, quinolizinyl group, quinoxalinyl group, phthalazinyl group, naphthyridinyl group, acridinyl group, phenoxazinyl group, phenothiazinyl group, purinyl group, xanthinyl group, pteridinyl group, carbazolyl group, dibenzofuranyl group, dibenzothiophenyl group, tetrahydrocarbazolyl group, tetrahydrodibenzofuranyl group, tetrahydrodibenzothiophenyl group and the like. Further, preferred examples include indolyl group, benzimidazolyl group, benzofuranyl group, benzoxazolyl group, quinolyl group, isoquinolyl group, phenoxazinyl group, phenothiazinyl group, carbazolyl group, dibenzofuranyl group, dibenzothiophenyl group, tetrahydrocarbazolyl group, tetrahydrodibenzofuranyl group, and tetrahydro-dibenzothiophenyl group.

With respect to (IIl) examples of nitrogen atom-containing heterocyclic ring group formed by R² and R³ which are bonded to each other include aziridine ring, azetidine ring, pyrrole ring, pyrrolidine ring, pyrazole ring, pyrazolone ring, imidazole ring, isoimidazole ring, imidazoline ring, imidazolidine ring, oxazolidine ring, piperidine ring, piperazine ring, homopiperazine ring, morpholine ring, thiomorpholine ring, tetrahydroquinoline ring, tetrahydroisoquinoline ring, indole ring, isoindole ring, benzimidazole ring, phenoxazine ring, phenothiazine ring, purine ring, xanthine ring, carbazole ring, tetrahydrocarbazole ring and the like. Further, preferred examples include aziridine ring, pyrrolidine ring, piperidine ring, piperazine ring, homopiperazine ring, morpholine ring, and thiomorpholine ring.

Among the abovementioned groups, R³ is preferably:
(a) hydrogen,
(b) an optionally substituted straight-chain alkyl group,
(c) an optionally substituted branched-chain alkyl group,
(d) an optionally substituted cyclic alkyl group,
(e) an optionally substituted alkenyl group,
(f) an optionally substituted aralkyl group,
(g) an optionally substituted aryl group,
(h) a heteroatom-containing saturated carbon chain group, or
(i) a heteroatom-containing unsaturated carbon chain group;
more preferably:
(b) an optionally substituted straight-chain alkyl group,
(c) an optionally substituted branched-chain alkyl group,
(d) an optionally substituted cyclic alkyl group,
(e) an optionally substituted alkenyl group,
(f) an optionally substituted aralkyl group,
(g) an optionally substituted aryl group,
(h) a heteroatom-containing saturated carbon chain group, or
(i) a heteroatom-containing unsaturated carbon chain group;
still more preferably:
(b) an optionally substituted straight-chain alkyl group,
(f) an optionally substituted aralkyl group, or
(h) a heteroatom-containing saturated carbon chain group; and
particularly preferably:
(h) a heteroatom-containing saturated carbon chain group.

Compounds represented by the general formula (1) set forth above wherein A is CH₂NR²R³, and R² is neither an acyl group nor an alkyloxycarbonyl group, which are ketone compounds having an amine type nitrogenous functional group, may be used not only in the form of free amine but also as a salt with commonly used mineral or organic acid. The commonly used mineral acid is not limited as long as it has no oxidizing property. Examples thereof include hydrochloric acid, hydrobromic acid, hydroiodic acid, sulfuric acid and the like, with hydrochloric acid and hydrobromic acid being more preferred. The commonly used organic acid is not limited as long as it has no oxidizing property, and may be a carboxylic acid, such as formic acid, acetic acid, butyric acid, or trifluoroacetic acid, with acetic acid and trifluoroacetic acid being more preferred.

Among ketone compounds represented by the general formula (1), compounds of the formula (1) in which n is 0 and A is CH₂NR²R³ include compounds useful as an important intermediate for the preparation of medicaments which are described in WO 97/25311 and WO 99/01431 as being very useful for treating and preventing diabetes, obesity, hyperlipidemia and the like.
Such compounds may be specified as follows.

That is, R¹ represents a substituted phenyl group, such as 3-aminophenyl group, 3-nitrophenyl group, 3-[(benzyl) (methylsulfonyl)amino]phenyl group, 4-chloro-3-aminophenyl group, 4-chloro-3-[(benzyl)(methylsulfonyl)amino]phenyl group, 4-chloro-3-nitrophenyl group, 4-bromo-3-aminophenyl group, 4-bromo-3-[(benzyl)(methylsulfonyl)amino]phenyl group, 4-bromo-3-nitrophenyl group, 4-benzyloxy-3-nitrophenyl group, 4-benzyloxy-3-aminophenyl group, 4-benzyloxy-3-[(benzyl)(methylsulfonyl)amino]phenylgroup, 4-benzyloxy-3-[(benzyl)(benzylsulfonyl)amino]phenyl group, or 4-benzyloxy-3-[(hydroxy)methyl]phenylgroup. R¹ may be preferably 4-chloro-3-[(benzyl)(methylsulfonyl)amino]phenyl group, 4-bromo-3-[(benzyl)(methylsulfonyl)amino]phenylgroup,3-[(benzyl)-(methylsulfonyl)amino]phenyl group, or 4-benzyloxy-3-[(benzyl)-(methylsulfonyl)amino]phenyl group. Further, R² represents an amino-protecting group which can be removed under a mild condition. Specific examples thereof include an acyl group, such as acetyl group, propionyl group, butyryl group, pentanoyl group, hexanoyl group, trifluoroacetyl group, pivaloyl group, benzoyl group, 4-methoxybenzoyl group, 2,4,6-trimethylbenzoyl group, or naphthoyl group; a carbamate type protecting group, such as tert-butoxycarbonyl group, benzyloxycarbonyl group, p-methoxybenzyloxycarbonyl group, or 2,2,2-trichloroethoxycarbonyl group; and an aralkyl group, such as benzyl group, p-methoxybenzyl group, or p-nitrobenzyl group, with benzoyl group and benzyl group being preferred. Further, R³ represents an ethyl group which is bonded at its end to a fused tricyclic ring group via an oxygen atom. Specific examples thereof include 2-(dibenzofuran-3-yloxy)ethyl group, 2-(dibenzo-thiophen-3-yloxy)ethyl group, 2-(9H-carbazol-2-yloxy)ethyl group, 2-(1,2,3,4-tetrahydrodibenzofuran-6-yloxy)ethyl group, 2-(1,2,3,4-tetrahydrodibenzothiophen-6-yloxy)ethyl group, and 2-(1,2,3,4-tetrahydro-9H-carbazol-7-yloxy)ethyl group, with 2-(9H-carbazol-2-yloxy)ethyl group being preferred.

Particularly preferred examples of the ketone compounds having the abovementioned substituents are set forth below. That is, such particularly preferred examples may be the compounds having a benzyl group as R², such as:
1-[4-benzyloxy-3-[(benzyl)(methylsulfonyl)amino]phenyl]-2-[[2-(dibenzothiophen-3-yloxy)ethyl]benzylamino]ethanone;
1-[4-benzyloxy-3-[(benzyl) (methylsulfonyl)amino]phenyl]-2-[[2-(dibenzofuran-3-yloxy)ethyl]benzylamino]ethanone;
1-[4-benzyloxy-3-[(benzyl) (methylsulfonyl)amino]phenyl]-2-[[2-(9H-carbazol-2-yloxy)ethyl]benzylamino]ethanone;
1-(4-benzyloxy-3-[(benzyl)(methylsulfonyl)amino]phenyl]-2-[[2-(1,2,3,4-tetrahydrodibenzothiophen-6-yloxy)ethyl]-benzylamino]ethanone;
1-[4-benzyloxy-3-[(benzyl)(methylsulfonyl)amino]phenyl]-2-[[2-(1,2,3,4-tetrahydrodibenzofuran-6-yloxy)ethyl]benzylamino]-ethanone;
1-[4-benzyloxy-3-[(benzyl)(methylsulfonyl)amino]phenyl]-2-[[2-(1,2,3,4-tetrahydro-9H-carbazol-7-yloxy)ethyl]benzylamino]-ethanone;
1-[4-benzyloxy-3-[(benzyl) (benzylsulfonyl)amino]phenyl]-2-[[2-(dibenzothiophen-3-yloxy]ethyl]benzylamino]ethanone;
1-[4-benzyloxy-3-[(benzyl)(benzylsulfonyl)amino]phenyl]-2-[[2-(dibenzofuran-3-yloxy)ethyl]benzylamino]ethanone;
1-[4-benzyloxy-3-[(benzyl) (benzylsulfonyl)amino]phenyl]-2-[[2-(9H-carbazol-2-yloxy)ethyl]benzylamino]ethanone;
1-[4-benzyloxy-3-[(benzyl) (benzylsulfonyl)amino]phenyl]-2-[[2-(1,2,3,4-tetrahydrodibenzothiophen-6-yloxy)ethyl]-benzylamino]ethanone;
1-[4-benzyloxy-3-[(benzyl)(benzylsulfonyl)amino]phenyl]-2-[[2-(1,2,3,4-tetrahydrodibenzofuran-6-yloxy)ethyl]benzylamino]-ethanone;
1-[4-benzyloxy-3-[(benzyl)(benzylsulfonyl)amino]phenyl]-2-[[2-(1,2,3,4-tetrahydro-9H-carbazol-7-yloxy)ethyl]benzylamino]-ethanone;
1-[4-chloro-3-[(benzyl)(methylsulfonyl)amino]phenyl]-2-[[2-(dibenzothiophen-3-yloxy)ethyl]benzylamino]ethanone;
1-[4-chloro-3-[(benzyl)(methylsulfonyl)amino]phenyl]-2-[[2-(dibenzofuran-3-yloxy)ethyl]benzylamino]ethanone;
1-[4-chloro-3-[(benzyl)(methylsulfonyl)amino]phenyl]-2-[[2-(9H-carbazol-2-yloxy)ethyl]benzylamino]ethanone;
1-[4-chloro-3-[(benzyl)(methylsulfonyl)amino]phenyl]-2-[[2-(1,2,3,4-tetrahydrodibenzothiophen-6-yloxy)ethyl]-benzylamino]ethanone;
1-[4-chloro-3-[(benzyl)(methylsulfonyl)amino]phenyl]-2-[[2-(1,2,3,9-tetrahydrodibenzofuran-6-yloxy)ethyl]benzylamlno]-ethanone;
1- [4-chloro-3-[(benzyl)(methylsulfonyl)amino]phenyl]-2-[[2-(1,2,3,4-tetrahydro-9H-carbazol-7-yloxy)ethyl]benzylamino]-ethanone;
1-[4-bromo-3-[(benzyl)(methylsulfonyl)amino]phenyl]-2-[[2-(dibenzothiophen-3-yloxy)ethyl]benzylamino]ethanone;
1- [4-bromo-3-[(benzyl)(methylsulfonyl)amino]phenyl]-2-[[2-(dibenzofuran-3-yloxy)ethyl]benzylamino]ethanone;
1-[4-bromo-3-[(benzyl)(methylsulfonyl)amino]phenyl]-2-[[2-(9H-carbazol-2-yloxy)ethyl]benzylamino]ethanone;
1-[4-bromo-3-[(benzyl)(methylsulfonyl)amino]phenyl]-2-[[2-(1,2,3,4-tetrahydrodibenzothiophen-6-yloxy)ethyl]benzylamino]-ethanone;
1-[4-bromo-3-[(benzyl)(methylsulfonyl)amino]phenyl]-2-[[2-(1,2,3,4-tetrahydrodibenzofuran-6-yloxy)ethyl]benzylamino]-ethanone;
1-[4-bromo-3-[(benzyl) (methylsulfonyl)amino]phenyl]-2-[[2-(1,2,3,4-tetrahydro-9H-carbazol-7-yloxy)ethyl]benzylamino]-ethanone;
1-[3-[(benzyl) (methylsulfonyl)amino]phenyl]-2-[[2-(dibenzothiophen-3-yloxy)ethyl]benzylamino]ethanone;
1-[3-[(benzyl) (methylsulfonyl)amino]phenyl]-2-[[2-(dibenzofuran-3-yloxy)ethyl]benzylamino]ethanone;
1-[3-[(benzyl) (methylsulfonyl)amino]phenyl]-2-[[2-(9H-carbazol-2-yloxy)ethyl]benzylamino]ethanone;
1-[3-[(benzyl)(methylsulfonyl)amino]phenyl]-2-[[2-(1,2,3,4-tetrahydrodibenzothiophen-6-yloxy)ethyl]benzylamino]-ethanone;
1-[3-[(benzyl)(methylsulfonyl)amino]phenyl]-2-[[2-(1,2,3,4-tetrahydrodibenzofuran-6-yloxy)ethyl]benzylamino]-ethanone;
1-[3-[(benzyl) (methylsulfonyl)amino]phenyl]-2-[[2-(1,2,3,4-tetrahydro-9H-carbazol-7-yloxy)ethyl]benzylamino]-ethanone;
1-(4-benzyloxy-3-aminophenyl)-2-[[2-(dibenzothiophen-3-yloxy)ethyl]benzylamino]ethanone;
1-(4-benzyloxy-3-aminophenyl)-2-[[2-(dibenzofuran-3-yloxy)ethyl]benzylamino]ethanone;
1-(4-benzyloxy-3-aminophenyl)-2-[[2-(9H-carbazol-2-yloxy)-ethyl]benzylamino]ethanone;
1-(4-benzyloxy-3-aminophenyl)-2-[[2-(1,2,3,4-tetrahydro-dibenzothiophen-6-yloxy)ethyl]benzylamino]ethanone;
1-(4-benzyloxy-3-aminophenyl)-2-[[2-(1,2,3,4-tetrahydrodibenzofuran-6-yloxy)ethyl]benzylamino]ethanone;
1-(4-benzyloxy-3-aminophenyl)-2-[[2-(1,2,3,4-tetrahydro-9H-carbazol-7-yloxy)ethyl]benzylamino]ethanone;
1-(4-chloro-3-aminophenyl)-2-[[2-(dibenzothiophen-3-yloxy)ethyl]benzylamino]ethanone;
1-(4-chloro-3-aminophenyl)-2-[[2-(dibenzofuran-3-yloxy)-ethyl]benzylamino]ethanone;
1-(4-chloro-3-aminophenyl)-2-[[2-(9H-carbazol-2-yloxy)-ethyl]benzylamino]ethanone;
1-(4-chloro-3-aminophenyl)-2-[[2-(1,2,3,4-tetrahydrodibenzothiophen-6-yloxy)ethyl]benzylamino]ethanone;
1-(4-chloro-3-aminophenyl)-2-[[2-(1,2,3,4-tetrahydrodibenzofuran-6-yloxy)ethyl]benzylamino]ethanone;
1-(4-chloro-3-aminophenyl)-2-[[2-(1,2,3,4-tetrahydro-9H-carbazol-7-yloxy)ethyl]benzylamino]ethanone;
1-(4-bromo-3-aminophenyl)-2-[[2-(dibenzothiophen-3-yloxy)-ethyl]benzylamino]ethanone
1-(4-bromo-3-aminophenyl)-2-[[2-(dibenzofuran-3-yloxy)-ethyl]benzylamino]ethanone;
1-(4-bromo-3-aminophenyl)-2-[[2-(9H-carbazol-2-yloxy)-ethyl]benzylamino]ethanone;
1-(4-bromo-3-aminophenyl)-2-[[2-(1,2,3,4-tetrahydrodibenzothiophen-6-yloxy)ethyl]benzylamino]ethanone;
1-(4-bromo-3-aminophenyl)-2-[[2-(l,2,3,4-tetrahydrodibenzofuran-6-yloxy)ethyl]benzylamino]ethanone;
1-(4-bromo-3-aminophenyl)-2-[[2-(1,2,3,4-tetrahydro-9H-carbazol-7-yloxy)ethyl]benzylamino]ethanone;
1-(3-aminophenyl)-2-[[2-(dibenzothiophen-3-yloxy)ethyl]-benzylamino]ethanone;
1-(3-aminophenyl)-2-[[2-(dibenzofuran-3-yloxy)ethyl]-benzylamino]ethanone;
1-(3-aminophenyl)-2-[[2-(9H-carbazol-2-yloxy)ethyl]-benzylamino]ethanone;
1-(3-aminophenyl)-2-[[2-(1,2,3,4-tetrahydrodibenzothiophen-6-yloxy)ethyl]benzylamino]ethanone;
1-(3-aminophenyl)-2-[[2-(1,2,3,4-tetrahydrodibenzofuran-6-yloxy)ethyl]benzylamino]ethanone;
1-(3-aminophenyl)-2-[[2-(1,2,3,4-tetrahydro-9H-carbazol-7-yloxy)ethyl]benzylamino]ethanone;
1-(4-benzyloxy-3-nitrophenyl)-2-[[2-(dibenzothiophen-3-yloxy)ethyl]benzylamino]ethanone;
1-(4-benzyloxy-3-nitrophenyl)-2-[[2-(dibenzofuran-3-yloxy) ethyl]benzylamino]ethanone;
1-(4-benzyloxy-3-nitrophenyl)-2-[[2-(9H-carbazol-2-yloxy)-ethyl]benzylamino]ethanone;
1-(4-benzyloxy-3-nitrophenyl)-2-[[2-(1,2,3,4-tetrahydrodibenzothiophen-6-yloxy)ethyl]benzylamino]ethanone;
1-(4-benzyloxy-3-nitrophenyl)-2-[[2-(1,2,3,4-tetrahydrodibenzofuran-6-yloxy)ethyl]benzylamino]ethanone;
1-(4-benzyloxy-3-nitrophenyl)-2-[[2-(1,2,3,4-tetrahydro-9H-carbazol-7-yloxy)ethyl]benzylamino]ethanone;
1-(4-chloro-3-nitrophenyl)-2-[[2-(dibenzothiophen-3-yloxy)ethyl]benzylamino]ethanone;
1-(4-chloro-3-nitrophenyl)-2-[[2-(dibenzofuran-3-yloxy)-ethyl]benzylamino]ethanone;
1-(4-chloro-3-nitrophenyl)-2-[[2-(9H-carbazol-2-yloxy)-ethyl]benzylamino]ethanone;
1-(4-chloro-3-nitrophenyl)-2-[[2-(1,2,3,4-tetrahydrodibenzothiophen-6-yloxy)ethyl]benzylamino]ethanone;
1-(4-chloro-3-nitrophenyl)-2-[[2-(1,2,3,4-tetrahydrodibenzofuran-6-yloxy)ethyl]benzylamino]ethanone;
1-(4-chloro-3-nitrophenyl)-2-[[2-(1,2,3,4-tetrahydro-9H-carbazol-7-yloxy)ethyl]benzylamino]ethanone;
1-(4-bromo-3-nitrophenyl)-2-[[2-(dibenzothiophen-3-yloxy)-ethyl]benzylamino]ethanone;
1-(4-bromo-3-nitrophenyl)-2-[[2-(dibenzofuran-3-yloxy)-ethyl]benzylamino]ethanone;
1-(4-bromo-3-nitrophenyl)-2-[[2-(9H-carbazol-2-yloxy)-ethyl]benzylamino]ethanone;
1-(4-bromo-3-nitrophenyl)-2-[[2-(l,2,3,4-tetrahydrodibenzothiophen-6-yloxy)ethyl]benzylamino]ethanone;
1-(4-bromo-3-nitrophenyl)-2-[[2-(1,2,3,4-tetrahydrodibenzofuran-6-yloxy)ethyl]benzylamino]ethanone;
1-(4-bromo-3-nitrophenyl)-2-[[2-(1,2,3,4-tetrahydro-9H-carbazol-7-yloxy)ethyl]benzylamino]ethanone;
1-(3-nitrophenyl)-2-[[2-(dibenzothiophen-3-yloxy) ethyl]-benzylamino]ethanone;
1-(3-nitrophenyl)-2-[[2-(dibenzofuran-3-yloxy)ethyl]-benzylamino]ethanone;
1-(3-nitrophenyl)-2-[[2-(9H-carbazol-2-yloxy)ethyl]-benzylamino]ethanone;
1-(3-nitrophenyl)-2-[[2-(1,2,3,4-tetrahydrodibenzothiophen-6-yloxy)ethyl]benzylamino]ethanone;
1-(3-nitrophenyl)-2-[[2-(1,2,3,4-tetrahydrodibenzofuran-6-yloxy)ethyl]benzylamino]ethanone; and
1-(3-nitrophenyl)-2-[[2-(1,2,3,4-tetrahydro-9H-carbazol-7-yloxy)ethyl]benzylamino]ethanone;
and acceptable salts thereof, such as hydrochloride, acetate, hydrobromide, and trifluoroacetate.

Further, the compounds having a benzoyl group as R² may be also particularly preferred. Specific examples thereof include:
1-[4-benzyloxy-3-[(benzyl)(methylsulfonyl)amino]phenyl]-2-[[2-(dibenzothiophen-3-yloxy)ethyl]benzoylamino]ethanone;
1-[4-benzyloxy-3-[(benzyl)(methylsulfonyl)amino]phenyl]-2-[[2-(dibenzofuran-3-yloxy)ethyl]benzoylamino]ethanone;
1-[4-benzyloxy-3-[(benzyl)(methylsulfonyl)amino]phenyl]-2-[[2-(9H-carbazol-2-yloxy)ethyl]benzoylamino]ethanone;
1-[4-benzyloxy-3-[(benzyl) (methylsulfonyl)amino]phenyl]-2-[[2-(1,2,3,4-tetrahydrodibenzothiophen-6-yloxy)ethyl] benzoylamino]ethanone;
1-[4-benzyloxy-3-[(benzyl)(methylsulfonyl)amino]phenyl]-2-[[2-(1,2,3,4-tetrahydrodibenzofuran-6-yloxy)ethyl] benzoylamino] ethanone;
1-[4-benzyloxy-3-[(benzyl)(methylsulfonyl)amino]phenyl]-2-[[2-(1,2,3,4-tetrahydro-9H-carbazol-7-yloxy)ethyl]benzoylamino]ethanone;
1-[4-benzyloxy-3-[(benzyl) (benzylsulfonyl)amino]phenyl]-2-[[2-(dibenzothiophen-3-yloxy)ethyl]benzoylamino]ethanone;
1-[4-benzyloxy-3-[(benzyl)(benzylsulfonyl)amino]phenyl]-2-[[2-(dibenzofuran-3-yloxy)ethyl]benzoylamino]ethanone;
1-[4-benzyloxy-3-[(benzyl) (benzylsulfonyl)amino]phenyl]-2-[[2-(9H-carbazol-2-yloxy)ethyl]benzoylamino]ethanone;
1-[4-benzyloxy-3-[(benzyl)(benzylsulfonyl)amino]phenyl]-2-[[2-(1,2,3,4-tetrahydrodibenzothiophen-6-yloxy)ethyl]benzoyl-amino]ethanone;
1-[4-benzyloxy-3-[(benzyl)(benzylsulfonyl)amino]phenyl]-2-[[2-(1,2,3,4-tetrahydrodibenzofuran-6-yloxy)ethyl]benzoyl-amino]ethanone;
1-[4-benzyloxy-3-[(benzyl)(benzylsulfonyl)amino]phenyl]-2-[[2-(1,2,3,4-tetrahydro-9H-carbazol-7-yloxy)ethyl]benzoylamino]ethanone;
1-[4-chloro-3-[(benzyl](methylsulfonyl)amino]phenyl]-2-[[2-(dibenzothiophen-3-yloxy)ethyl]benzoylamino]ethanone;
1-[4-chloro-3-[(benzyl)(methylsulfonyl)amino]phenyl]-2-[[2-(dibenzofuran-3-yloxy)ethyl]benzoylamino]ethanone;
1-[4-chloro-3-[(benzyl)(methylsulfonyl)amino]phenyl]-2-[[2-(9H-carbazol-2-yloxy)ethyl]benzoylamino]ethanone;
1-[4-chloro-3-[(benzyl)(methylsulfonyl)amino]phenyl]-2-[[2-(1,2,3,4-tetrahydrodibenzothiophen-6-yloxy)ethyl]benzoyl-amino]ethanone;
1-[4-chloro-3-[(benzyl)(methylsulfonyl)amino]phenyl]-2-[[2-(1,2,3,4-tetrahydrodibenzofuran-6-yloxy)ethyl]benzoyl-amino]ethanone;
1-[4-chloro-3-[(benzyl)(methylsulfonyl)amino]phenyl]-2-[[2-(1,2,3,4-tetrahydro-9H-carbazol-7-yloxy)ethyl]benzoylamino]ethanone;
1-[4-bromo-3-[(benzyl)(methylsulfonyl)amino]phenyl]-2-[[2-(dibenzothiophen-3-yloxy)ethyl]benzoylamino]ethanone;
1-[4-bromo-3-[(benzyl)(methylsulfonyl)amino]phenyl]-2-[[2-(dibenzofuran-3-yloxy)ethyl]benzoylamino]ethanone;
1-[4-bromo-3-[(benzyl)(methylsulfonyl)amino]phenyl]-2-[[2-(9H-carbazol-2-yloxy)ethyl]benzoylamino]ethanone;
1-[4-bromo-3-[(benzyl)(methylsulfonyl)amino]phenyl]-2-[[2-(1,2,3,4-tetrahydrodibenzothiophen-6-yloxy)ethyl]benzoylamino]ethanone;
1-[4-bromo-3-[(benzyl) (methylsulfonyl)amino]phenyl]-2-[[2-(1,2,3,4-tetrahydrodibenzofuran-6-yloxy)ethyl]benzoylamino]-ethanone;
1-[4-bromo-3-[(benzyl)(methylsulfonyl)amino]phenyl]-2-[[2-(1,2,3, 4-tetrahydro-9H-carbazol-7-yloxy)ethyl]benzoylamino]-ethanone;
1-[3-[(benzyl)(methylsulfonyl)amino]phenyl]-2-[[2-(dibenzothiophen-3-yloxy)ethyl]benzoylamino]ethanone;
1-[3-[(benzyl) (methylsulfonyl)amino]phenyl]-2-[[2-(dibenzofuran-3-yloxy)ethyl]benzoylamino]ethanone;
1-[3-[(benzyl)(methylsulfonyl)amino]phenyl]-2-[[2-(9H-carbazol-2-yloxy)ethyl]benzoylamino]ethanone;
1-[3-[(benzyl)(methylsulfonyl)amino]phenyl]-2-[[2-(1,2,3,4-tetrahydrodibenzothiophen-6-yloxy)ethyl]benzoylamino]ethanone;
1-[3-[(benzyl)(methylsulfonyl)amino]phenyl]-2-[[2-(1,2,3,4-tetrahydrodibenzofuran-6-yloxy)ethyl]benzoylamino]ethanone;
1-[3-[(benzyl)(methylsulfonyl)amino]phenyl]-2-[[2-(1,2,3,4-tetrahydro-9H-carbazol-7-yloxy)ethyl]benzoylamino]ethanone;
1-(4-benzyloxy-3-aminophenyl)-2-[[2-(dibenzothiophen-3-yloxy)ethyl]benzoylamino]ethanone;
1-(4-benzyloxy-3-aminophenyl)-2-[[2-(dibenzofuran-3-yloxy)ethyl]benzoylamino]ethanone;
1-(4-benzyloxy-3-aminophenyl)-2-[[2-(9H-carbazol-2-yloxy)-ethyl]benzoylamino]ethanone;
1- (4-benzyloxy-3-aminophenyl)-2-[[2-(1,2,3,4-tetrahydro-dibenzothiophen-6-yloxy)ethyl]benzoylamino]ethanone;
1-(4-benzyloxy-3-aminophenyl)-2-[[2-(1,2,3,4-tetrahydrodibenzofuran-6-yloxy)ethyl]benzoylamino]ethanone;
1-(4-benzyloxy-3-aminophenyl)-2-[[2-(1,2,3,4-tetrahydro-9H-carbazol-7-yloxy)ethyl]benzoylamino]ethanone;
1-(4-chloro-3-aminophenyl)-2-[[2-(dibenzothiophen-3-yloxy)ethyl]benzoylamino]ethanone;
1-(4-chloro-3-aminophenyl)-2-[[2-(dibenzofuran-3-yloxy)-ethyl]benzoylamino]ethanone;
1-(4-chloro-3-aminophenyl)-2-[[2-(9H-carbazol-2-yloxy)-ethyl]benzoylamino]ethanone;
1-(4-chloro-3-aminophenyl)-2-[[2-(1,2,3,4-tetrahydro-dibenzothiophen-6-yloxy)ethyl]benzoylamino]ethanone;
1-(4-chloro-3-aminophenyl)-2-[[2-(1,2,3,4-tetrahydrodibenzofuran-6-yloxy)ethyl]benzoylamino]ethanone;
1-(4-chloro-3-aminophenyl)-2-[[2-(1,2,3,4-tetrahydro-9H-carbazol-7-yloxy]ethyl]benzoylamino]ethanone;
1-(4-bromo-3-aminophenyl)-2-[[2-(dibenzothiophen-3-yloxy)-ethyl]benzoylamino]ethanone;
1-(4-bromo-3-aminophenyl)-2-[[2-(dibenzofuran-3-yloxy)-ethyl]benzoylamino]ethanone;
1-(4-bromo-3-aminophenyl)-2-[[2-(9H-carbazol-2-yloxy)-ethyl]benzoylamino]ethanone;
1-(4-bromo-3-aminophenyl)-2-[[2-(1,2,3,4-tetrahydrodibenzothiophen-6-yloxy)ethyl]benzoylamino]ethanone;
1-(4-bromo-3-aminophenyl)-2-[[2-(1,2,3,4-tetrahydrodibenzofuran-6-yloxy)ethyl]benzoylamino]ethanone;
1-(4-bromo-3-aminophenyl)-2-[[2-(1,2,3,4-tetrahydro-9H-carbazol-7-yloxy)ethyl]benzoylamino]ethanone;
1-(3-aminophenyl)-2-[[2-(dibenzothiophen-3-yloxy)ethyl]-benzoylamino]ethanone;
1-(3-aminophenyl)-2-[[2-(dibenzofuran-3-yloxy)ethyl]-benzoylamino]ethanone;
1-(3-aminophenyl)-2-[[2-(9H-carbazol-2-yloxy)ethyl]-benzoylamino]ethanone;
1-(3-aminophenyl)-2-[[2-(1,2,3,4-tetrahydrodibenzothiophen-6-yloxy)ethyl]benzoylamino]ethanone;
1-(3-aminophenyl)-2-[[2-(1,2,3,4-tetrahydrodibenzofuran-6-yloxy)ethyl]benzoylamino]-ethanone;
1-(3-aminophenyl)-2-[[2-(1,2,3,4-tetrahydro-9H-carbazol-7-yloxy)ethyl]benzoylamino]ethanone;
1-(4-benzyloxy-3-nitrophenyl)-2-[[2-(dibenzothiophen-3-yloxy)ethyl]benzoylamino]ethanone;
1-(4-benzyloxy-3-nitrophenyl)-2-[[2-(dibenzofuran-3-yloxy)ethyl]benzoylamino]ethanone;
1-(4-benzyloxy-3-nitrophenyl)-2-[[2-(9H-carbazol-2-yloxy)ethyl]benzoylamino]ethanone;
1-(4-benzyloxy-3-nitrophenyl)-2-[[2-(1,2,3,4-tetrahydrodibenzothiophen-6-yloxy)ethyl]benzoylamino]ethanone;
1-(4-benzyloxy-3-nitrophenyl)-2-[[2-(1,2,3,4-tetrahydrodibenzofuran-6-yloxy)ethyl]benzoylamino]ethanone;
1-(4-benzyloxy-3-nitrophenyl)-2-[[2-(1,2,3,4-tetrahydro-9H-carbazol-7-yloxy)ethyl]benzoylamino]ethanone;
1-(4-chloro-3-nitrophenyl)-2-[[2-(dibenzothiophen-3-yloxy)ethyl]benzoylamino]ethanone;
1-(4-chloro-3-nitrophenyl)-2-[[2-(dibenzofuran-3-yloxy)-ethyl]benzoylamino]ethanone;
1-(4-chloro-3-nitrophenyl)-2-[[2-(9H-carbazol-2-yloxy)-ethyl]benzoylamino]ethanone;
1-(4-chloro-3-nitrophenyl)-2-[[2-(1,2,3,4-tetrahydrodibenzothiophen-6-yloxy)ethyl]benzoylamino]ethanone;
1-(4-chloro-3-nitrophenyl)-2-[[2-(1,2,3,4-tetrahydrodibenzofuran-6-yloxy)ethyl]benzoylamino]ethanone;
1-(4-chloro-3-nitrophenyl)-2-[[2-(1,2,3,4-tetrahydro-9H-carbazol-7-yloxy)ethyl]benzoylamino]ethanone;
1-(4-bromo-3-nitrophenyl)-2-[[2-(dibenzothiophen-3-yloxy)-ethyl]benzoylamino]ethanone;
1-(4-bromo-3-nitrophenyl)-2-[[2-(dibenzofuran-3-yloxy)-ethyl]benzoylamino]ethanone;
1-(4-bromo-3-nitrophenyl)-2-[[2-(9H-carbazol-2-yloxy)-ethyl]benzoylamino]ethanone;
1-(4-bromo-3-nitrophenyl)-2-[[2-(1,2,3,4-tetrahydrodibenzothiophen-6-yloxy)ethyl]benzoylamino]ethanone;
1-(4-bromo-3-nitrophenyl)-2-[[2-(1,2,3,4-tetrahydrdibenzofuran-6-yloxy)ethyl]benzoylamino]ethanone;
1-(4-bromo-3-nitrophenyl)-2-[[2-(1,2,3,4-tetrahydro-9H-carbazol-7-yloxy)ethyl]benzoylamino]ethanone;
1-(3-nitrophenyl)-2-[[2-(dibenzothiophen-3-yloxy)ethyl]-benzoylamino]ethanone;
1-(3-nitrophenyl)-2-[[2-(dibenzofuran-3-yloxy)ethyl]-benzoylamino]ethanone;
1-(3-nitrophenyl)-2-[[2-(9H-carbazol-2-yloxy)ethyl]-benzoylamino]ethanone;
1-(3-nitrophenyl)-2-[[2-(1,2,3,4-tetrahydrodibenzothiophen-6-yloxy)ethyl]benzoylamino]ethanone;
1-(3-nitrophenyl)-2-[[2-(1,2,3,4-tetrahydrodibenzofuran-6-yloxy)ethyl]benzoylamino]ethanone; and
1-(3-nitrophenyl)-2-[[2-(1,2,3,4-tetrahydro-9H-carbazol-7-yloxy)ethyl]benzoylamino]ethanone.

Ketone compounds having a nitrogenous or oxygenic functional group at any of the α-, β- and γ-positions which are the reaction substrates represented by the general formula (1) may be synthesized as follows.

A process for the preparation of compounds of the general formula (1) in which A is CH₂NR²R³ and R² is neither an acyl group nor an alkyloxycarbonyl group, or ketone compounds having an amine type nitrogenous functional group, may comprising condense a leaving group-containing ketone compound [4-a] with a secondary amine compound [4-b] as indicated in the reaction scheme (4): 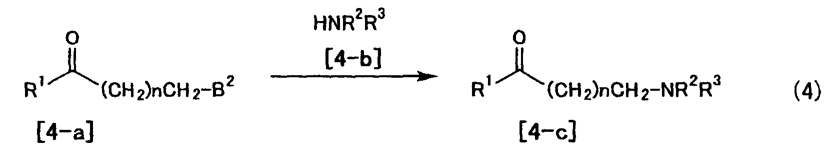 wherein R¹, R³ and n are as defined above; R² is neither an acyl group nor an alkyloxycarbonyl group; and
B² represents
(a) halogen, such as chlorine, bromine or iodine;
(b) a sulfonate group, such as mesylate or tosylate group; or
(c) a leaving group, such as quaternary ammonium salt.

This condensation reaction may be carried out by methods described in literatures [for example, as regards synthesis of α-aminoketone compounds: Larsen, A. A., et al., Journal of Medicinal Chemistry, 10, p. 462 (1967), and Larsen, A. A., et al., Journal of Medicinal Chemistry, 9, pp. 88-97 (1966); as regards synthesis of β-aminoketone compounds: Maxwell, C. E., Organic Synthesis Collective Vol. 3, pp. 305-306 (1955); as regards synthesis of γ-aminoketone compounds: Yevich, J. P. et al., Journal of Medicinal Chemistry, 35, pp. 4516-4525 (1992)]. Generally, the process may be carried out in the presence of aromatic hydrocarbon type solvent (such as toluene or xylene), ether type solvent (such as diethyl ether, tetrahydrofuran (THF), 1,4-dioxane, diisopropyl ether (IPE), or tert-butyl methyl ether (MTBE)), polar solvent (such as acetonitrile, acetone, methyl ethyl ketone (MEK), dimethylformamide (DMF), dimethylacetamide (DMA), N-methylpyrrolidone (NMP), or dimethylsulfoxide (DMSO)), or alcohol type solvent (such as methanol, ethanol, 2-propanol, 1-butanol, or 2-butanol). The process may be also carried out in the absence of the abovementioned solvents when [4-b] is used in an excess amount and also acts as a solvent.

This reaction is generally carried out using a base catalyst as an acid scavenger, such as alkali metal salt (such as potassium carbonate or sodium carbonate) or organic base (such as triethylamine, Hunig's base, pyridine or collidine), in an amount of from 1 to 10 mol, preferably from 1 to 5 mol, more preferably from 1 to 2 mol for 1 mol of a leaving group B². The reaction may be also carried out without these acid scavengers, however, when [4-b] is used in an excess amount. Further, when the leaving group B² is chlorine or bromine, the reaction time can be shortened by adding iodide such as sodium iodide or potassium iodide as a halogen exchange catalyst in an amount of from 0.01 to 10 times by mole, preferably from 0.1 to 0.3 times by mole. This reaction can be generally completed at temperatures ranging from -20°C to 200°C for from 1 to 48 hours, and is more preferably carried out at temperatures ranging from 20°C to 100°C for from 1 to 24 hours.

Alternatively, as indicated in the reaction scheme (5): 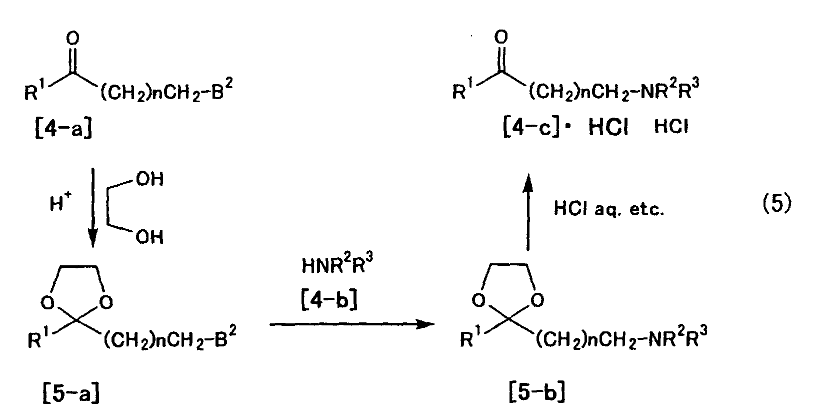 wherein R¹, R², R³, n and B² are as defined for the reaction scheme (4), this condensation reaction may be a process comprising heating a leaving group B²-containing ketone compound at temperatures ranging from 80°C to 100°C in the presence of an acid catalyst such as p-toluenesulfonic acid in 1,2-ethanediol to protect the carbonyl group of the ketone compound as ketal; condensing the protected ketone compound with a secondary amine compound [4-b]; and then deprotecting the ketal group of [5-b] to give a salt of desired aminoketone compound [4-c]. This reaction of [5-a] with [4-b] may be carried out according to the procedure indicated in the reaction scheme (4). The ketal-deprotecting method is not limited as long as it is a method commonly used. For example, the deprotection may be carried out with good yield by using from 0.1 to 10 times by mole, preferably 0.1 to 2 times by mole of dilute hydrochloric acid in THF as a solvent at temperatures ranging from 20°C to 40°C for from 1 to 24 hours. Further, aminoketone compounds generated may be simultaneously solidified as hydrochloride salt and may be also easily purified by recrystallization.

Compounds of the general formula (1) in which A is CH₂NR²R³ and R² is an acyl group or an alkyloxycarbonyl group, or ketone compounds having an amide type nitrogenous functional group, may be synthesized by known processes described in literatures [for example, Ikezaki, et al., YAKUGAKU ZASSHI, 106(1), pp. 80-89 (1986); and Takayuki Kawaguchi, et al., Chemical & Pharmaceutical Bulletin, 41(4), pp. 639-642 (1993)]. That is, as indicated in the reaction scheme (6): 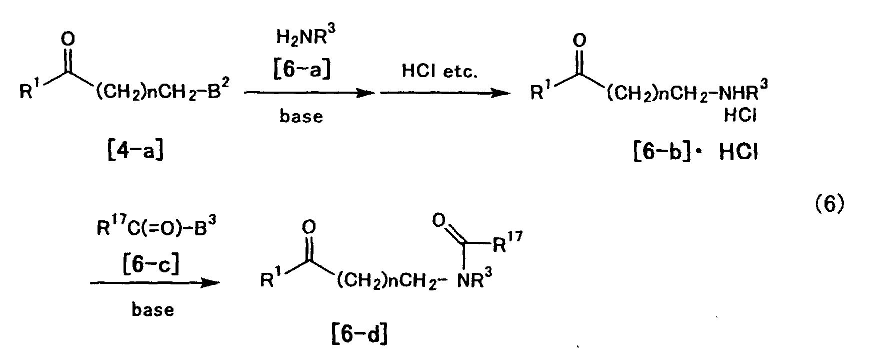 wherein R¹, R³, n and B² are as defined above;
R¹⁷ represents
   (a) an alkyl group, or
   (b) an alkoxy group; and
B³ represents
   (a) halogen, such as chlorine or bromine,
   (b) a carboxylic ester, such as formyloxy group or acetoxy group, or
   (c) a leaving group such as mesylate, tosylate or trifluoromethanesulfonate group,
a ketone compound having an amide type nitrogenous functional group [6-d] may be synthesized by condensing a leaving group B²-containing ketone compound [4-a] with a primary amine compound [6-a] to give a secondary aminoketone compound [6-b]; and protecting the amino group of [6-b] with an acylating or alkyloxycarbonylating agent [6-c].

Alternatively, as indicated in the reaction scheme (7): 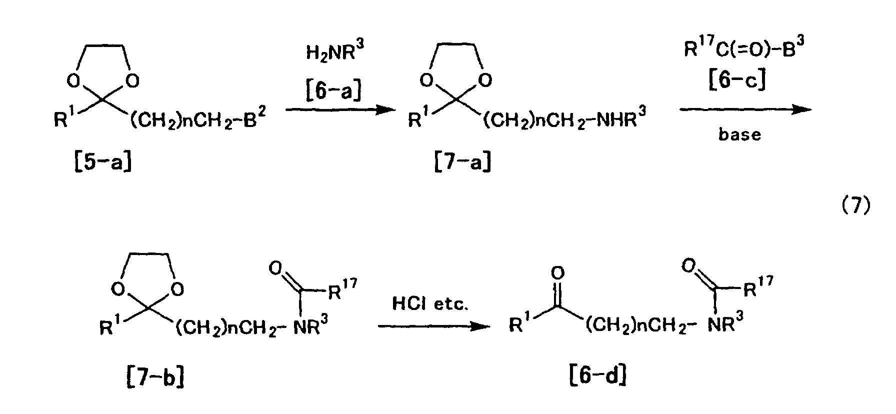 wherein R¹, R¹⁷, R³, n, B² and B³ are as defined above,
a ketal compound containing an amide type nitrogenous functional group [7-b] may be synthesized by condensing a leaving group B²-containing ketal compound [5-a] with a primary amine compound [6-a] to give a secondary aminoketal compound [7-a]; and protecting the secondary amino group with an acylating or alkyloxycarbonylating agent [6-c]. Alternative process may be a process comprising deprotecting [7-b] with hydrochloric acid or the like to synthesize [6-d]. This condensation reaction of [4-a] or [5-a] with primary amine compound [6-a] may be generally carried out in the presence of aromatic hydrocarbon type solvent (such as toluene or xylene), ether type solvent (such as diethyl ether, tetrahydrofuran (THF), 1,4-dioxane, diisopropyl ether (IPE), or tert-butyl methyl ether (MTBE)), polar solvent (such as acetonitrile, acetone, methyl ethyl ketone (MEK), dimethylformamide (DMF), dimethylacetamide (DMA), N-methylpyrrolidone (NMP), or dimethylsulfoxide (DMSO)), or alcohol type solvent (such as methanol, ethanol, 2-propanol, 1-butanol, or 2-butanol). The condensation reaction may also be carried out in the absence of the abovementioned solvent when [6-a] is used in an excess amount and also acts as a solvent.

According to this condensation reaction, a base catalyst as an acid scavenger, such as alkali metal salt (such as potassium carbonate or sodium carbonate) or organic base (such as triethyl-amine, Hunig's base, pyridine or collidine) may be used in an amount of from 1 to 10 mol, preferably from 1 to 5 mol, more preferably from 1 to 2 mol for 1 mol of the leaving group B². The process using a large excess amount of [6-a] in the absence of these acid scavengers may also be preferred in the viewpoint of inhibiting generating the dialkylated form. Further, when the leaving group B² is chlorine or bromine, the reaction time can be shortened by adding iodide such as sodium iodide or potassium iodide as a halogen exchange catalyst in an amount of from 0.01 to 10 times by mole, preferably from 0.1 to 0.3 times by mole. This reaction can be generally completed at temperatures ranging from -20°C to 100°C for from 0.1 to 48 hours, and may be more preferably carried out at temperatures ranging from 20°C to 50°C for from 0.1 to 1 hour. With respect to this condensation reaction, using a biphasic solvent system consisting of water and organic solvent is possible and may be particularly preferred. That is, the process for preparing the secondary aminoketone compound [6-b] may be a process comprising dissolving [6-a] in an amount of from 1 to 5 times by mole, preferably from 3 to 5 times by mole, more preferably from 3 to 3.5 times by mole, based on the leaving group B², in a water-immiscible solvent such as toluene at a relatively high concentration of from 0.1 to 5 mol/L, preferably from 0.5 to 2 mol/L; adding as an acid scavenger, from 1 to 5 L, preferably from 1 to 1.5 L of an aqueous sodium hydroxide solution having a concentration of from 5 to 50 w/v%, preferably from 10 to 20 w/v%, for 1 mol of the leaving group B²; adding portionwise [4-a] with vigorous stirring at temperatures ranging from 0°C to 10°C, preferably from 2°C to 7°C; and, while maintaining the reaction temperature in the range of from 2°C to 7°C, allowing the reaction to proceed for from 0.5 to 5 hours, preferably from 1 to 3 hours.

As occasion demands, a salt of [6-b] may be synthesized by using, for example, 10 w/v% hydrochloric acid at temperatures ranging from 0°C to 50°C, preferably from 10°C to 30°C, to form hydrochloride salt. Then, R² or an acyl or alkyloxycarbonyl group represented by R¹⁷C(=O) may be introduced by the conventional method described in the literature accepted in the art (Greene, T. W., et al., Protective Groups in Organic Synthesis (Wiley-Interscience Publication)). For example, a benzoyl group may be introduced into the secondary aminoketone compound [6-b] by treating the aminoketone compound with benzoyl chloride in an amount of from 1 to 3 mol, preferably from 1 to 1.2 mol for 1 mol of the aminoketone compound in a solvent such as dichloroethane or THF in the presence of an acid scavenger such as sodium hydrogencarbonate at temperatures ranging from 0°C to 30°C.

Next, when A of the general formula (1) is OR⁴ and R⁴ is SiR⁵R⁶R⁷, as indicated in the reaction scheme (8): 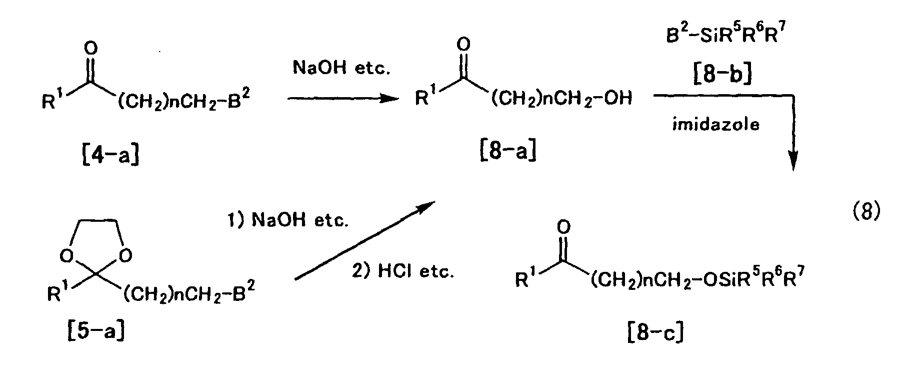 wherein R¹, B², R⁵, R⁶, R⁷ and n are as defined above, a silyloxyketone compound [8-c] may be synthesized by treating [4-a] or [5-a] with alkali such as NaOH or KOH; in case of [5-a], deprotecting ketal by an acid treatment; and reacting the thus obtained hydroxylketone compound [8-a] with B²-SiR⁵R⁶R⁷ [8-b] which is a sililating agent. This sililating reaction may be carried out by the conventional method for protecting a hydroxyl group with silyl described in the literature accepted in the art (Greene, T. W., et al., Protective Groups in Organic Synthesis (Wiley-Interscience Publication)).

Specifically, the process may be a process comprising dissolving [8-a] in a solvent such as THF, dichloromethane or DMF at a concentration of from 0.1 to 10 mol/L, preferably from 1 to 5 mol/L; adding an organic base such as imidazole, triethylamine or pyridine as an acid scavenger, preferably adding imidazole in an amount of from 1.0 to 1.5 times by mole, more preferably from 1.1 to 1.3 times by mole based on the sililating agent; and reacting [8-a] with a sililating agent such as tert-butyldiphenyl-chlorosilane in an amount of from 1.05 to 1.2 times by mole at temperatures ranging from 0°C to 30°C for from 0.5 to 12 hours.

Further, as indicated in the reaction scheme (9): 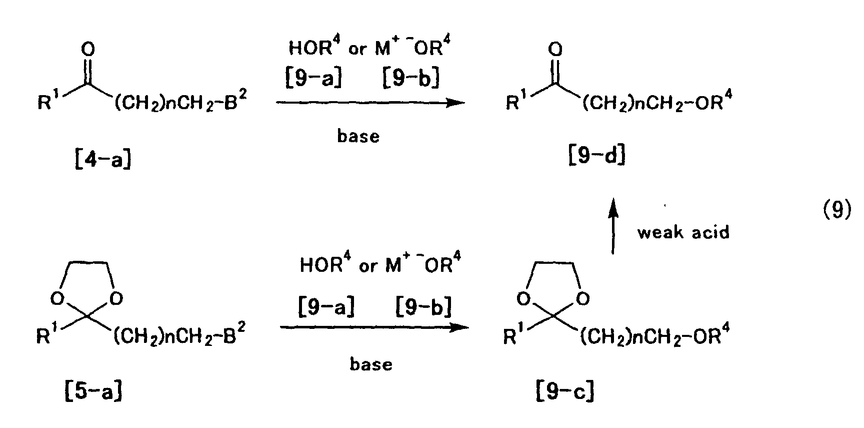 wherein R¹, B², R⁴ and n are as defined above, a ketone compound having an ether type oxygenic functional group may be synthesized by reacting [4-a] with an alcohol, phenol or silanol compound [9-a] represented by HOR⁴. An alternative process using [5-a] may be also used to synthesize an ether linkage-containing ketal compound [9-c]. The selective deprotection of ketal group afford the ketone compound [9-d]. Generally, this substitution reaction between HOR⁴ [9-a] and the leaving group B² may be carried out using [9-a] in an amount of from 1 to 10 mol, preferably from 1.1 to 2.0 mol for 1 mol of the leaving group B² in aromatic hydrocarbon solvent (such as toluene or xylene), ether type solvent (such as diethyl ether, tetrahydrofuran (THF), 1,4-dioxane, diisopropyl ether (IPE), or tert-butyl methyl ether (MTBE)), or polar solvent (such as acetonitrile, acetone, methyl ethyl ketone (MEK), dimethyl-formamide (DMF), dimethylacetamide (DMA), N-methylpyrrolidone (NMP), or dimethylsulfoxide (DMSO)).

Further, when [9-a] is a lower alcohol such as methanol, ethanol, 2-propanol, 1-butanol or 2-butanol, it may be also preferred to use [9-a] per se in a large excess amount as a solvent. According to this substitution reaction, a base catalyst, such as alkali metal salt (such as sodium hydroxide, potassium carbonate or sodium carbonate) or an,organic base (such as triethylamine, Hunig's base, pyridine or collidine) may be used as an acid scavenger in an amount of from 1 to 10 mol, preferably from 1 to 5 mol, more preferably from 1 to 2 mol for 1 mol of the leaving group B². It may be also preferred to previously convert the hydroxyl group of HOR⁴ [9-a] into its activated salt represented by M^{+ -}OR⁴ [9-b] wherein M⁺ represents a monovalent metal ion or quaternary ammonium ion, with a strong base such as a metal hydride (such as sodium hydride) or tetrabutylammonium hydroxide; and reacting [4-a] with the activated salt at relatively low temperatures ranging from -20°C to 5°C. Further, when the leaving group B² is chlorine or bromine, the reaction time can be shortened by adding iodide as a halogen exchange catalyst in an amount of from 0.01 to 10 times by mole, preferably from 0.1 to 0.3 times by mole as set forth above. This reaction can be generally completed at temperatures ranging from -20°C to 100°C for from 0.1 to 48 hours, and may be more preferably completed at temperatures ranging from 20°C to 50°C for from 0.1 to 1 hour.

When A of the general formula (1) is CH(OR¹⁵)₂**,** the process for synthesizing a ketal type oxygenic functional group-containing ketone compound wherein n is 0, 1 or 2 may vary depending the numerical value of n.

When n is 0, as indicated in the reaction scheme (10): 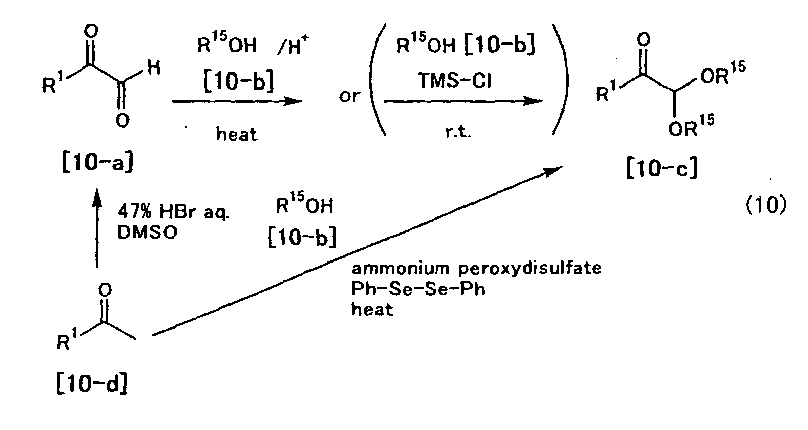 wherein R¹ and R¹⁵ are as defined above, the ketone compounds of interest may be synthesized by directly using a glyoxal compound [10-a] according to the known methods described in the literatures (Evans, et al., J. Chem. Soc., pp. 3324-3328 (1955); Henery-Logan, et al., Chem. Commun., 130 (1968); Trost, B. M., et al., J. Org. Chem., 45(14), pp. 2741-2746 (1980)). Alternatively, [10-a] obtained by oxidizing a variety of methyl ethyl ketone compounds [10-d] with 47% hydrobromic acid and dimethylsulfoxide according to the known methods described in the literatures (Bruce, M. J., et al., J. Chem. Soc. Perkin Trans.1, 14, pp. 1789-1796 (1995); and Tanaka, Yasuhiro, et al., Chem. Pharm. Bull., 44(5), pp. 885-891 (1996)) may be also used as the glyoxal compound set forth above. Specifically, an α-ketalketone compound [10-c] may be synthesized by heating/dehydrating a mixture of [10-a] with an alcohol compound [10-b] represented by R¹⁵OH or a diol compound such as 1,2-ethanediol or 1,3-propanediol in an amount of from 1 to 10 times by mole, preferably from 1 to 2 times by mole, in a solvent such as toluene in the presence of an acid catalyst such as hydrochloric acid, sulfuric acid or p-toluenesulfonic acid. Further, when the known method described in the literature (Chan, T. H., et al., Synthesis, 3, pp. 203-205(1983)) is used, [10-c] may be also synthesized by a process under a relatively mild condition wherein the process comprises reacting [10-a] with [10-b] in the presence of a Lewis acid catalyst such as trimethylsilyl chloride at room temperature for from 12 to 24 hours. An alternative process which does not use [10-a] may be a more abbreviated process for directly synthesizing [10-c] from a variety of methylketone compounds [10-d] according to the known method described in the literature (Tiecco, M., et al., J. Org. Chem., 55(15), pp. 4523-4528 (1990)) wherein the process comprises treating a methylketone compound [10-d] with ammonium peroxydisulfate and diphenyldiselenide in the presence of [10-b] for from 0.5 to 2 hours.

Next, when n is 1, as indicated in the reaction scheme (11): 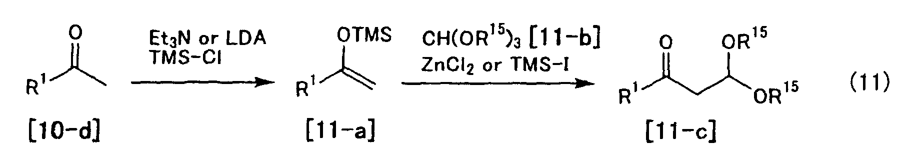 wherein R¹ and R¹⁵ are as defined above, a β-ketalketone compound [11-c] may be synthesized from a silyl enol ether [11-a] by the known methods described in the literatures (Makin, S. M., et al., J. Org. Chem. USSR (Engl. Transl.), 17(4), pp. 630-634 (1981); and Sakurai, Hideki, et al., Bull. Chem. Soc. Jpn., 56(10), pp. 3195-3196 (1983)). That is, the β-ketalketone compound [11-c] may be synthesized by reacting the silyl enol ether [11-a] with trialkoxymethane [11-b] such as trimethoxymethane in an aprotic solvent such as ethyl acetate or dichloromethane in the presence of a strong Lewis acid catalyst such as ZnCl₂ or trimethyliodosilane at temperatures ranging from -78°C to 25°C for from 1 to 3 hours. The silyl enol ether [11-a] may be easily prepared by treating a variety of methylketone compounds [10-d] with an organic base such as triethylamine or LDA (lithium diisopropylamide) in an amount of from 1 to 1.1 times by mole and trimethylchlorosilane in an amount of from 1 to 1.1 times by mole.

Further, when n is 2, as indicated in the reaction scheme (12): 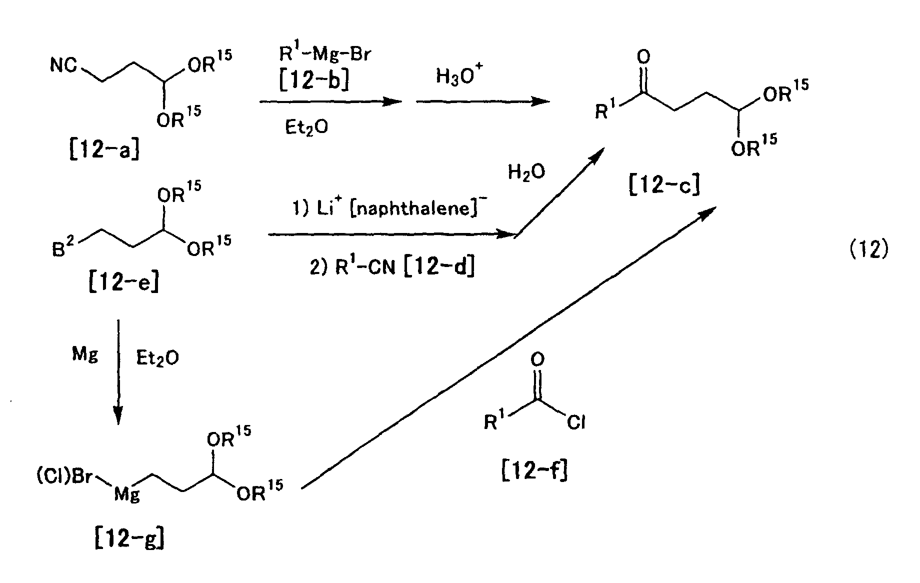 wherein R¹ and R¹⁵ are as defined above, and B² represents chlorine or bromine, a γ-ketalketone compound [12-c] may be synthesized according to the known method described in the literature (Kirrmann, et al., Bull. Soc. Chim. Fr., <5>2, p. 2150 (1935)) by reacting a commercially available γ-ketalnitrile compound [12-a] with a Grignard reagent [12-b] in an amount of from 1.0 to 1.05 times by mole in an ether type solvent such as diethyl ether or THF at relatively low temperatures ranging from -78°C to 0°C for from 1 to 6 hours followed by a hydrolysis reaction at normal temperature for from 1 to 2 hours. Further, [12-c] may be also synthesized according to the known method described in the literature (Barluenga, J., et al., J. Chem. Soc. Perkin Trans.1, pp. 3113-3118 (1988)) by reacting nitrile [12-d] or acid chloride [12-f] with a nucleophilic reagent (prepared from γ-ketal halide [12-e] and an organolithium compound such as lithium naphthalenide) in an amount of from 1.0 to 1.1 times by mole in an ether type anhydrous solvent such as diethyl ether or THF at low temperatures ranging from -78°C to -20°C followed by a hydrolysis reaction at normal temperature for from 1 to 2 hours.

Likewise, [12-c] may be also synthesized according to the known method described in the literature (Watanabe, Masami, et al., J. Chem. Soc. Perkin Trans.1, 21, pp. 3125-3128 (1994)) by subjecting nitrile [12-d] or acid chloride [12-f] to a nucleophilic reaction with a γ-ketal Grignard reagent [12-g] (easily prepared by a treatment of [12-e] with magnesium metal in diethyl ether) in an amount of from 1.0 to 1.1 times by mole in an ether type anhydrous solvent such as diethyl ether or THF at low temperatures ranging from -78°C to -20°C followed by a hydrolysis reaction at normal temperature for from 1 to 2 hours.

The ruthenium/optically active bidentate phosphine/diamine complex to be used in the present invention is represented by the general formula (2). In the formula, m represents an integer of from 0 to 2, with 0 being preferred.

X and Y may be the same or different and may be bonded to the ruthenium via covalent bond or ionic bond. They specifically represents:
(a) hydrogen,
(b) halogen,
(c) an alkoxy group,
(d) a carboxyl group, or
(e) other anion group.
(f) other anion group may be selected from a variety of anion groups and examples thereof include [BH₄]⁻ and [BF₄]⁻. X and Y are more preferably hydrogen, halogen or [BH₄]⁻.

The ruthenium/optically active bidentate phosphine/diamine complex comprises in the molecule an optically active bidentate phosphine ligand and a diamine ligand. The optically active bidentate phosphine ligand is represented by the general formula (13): 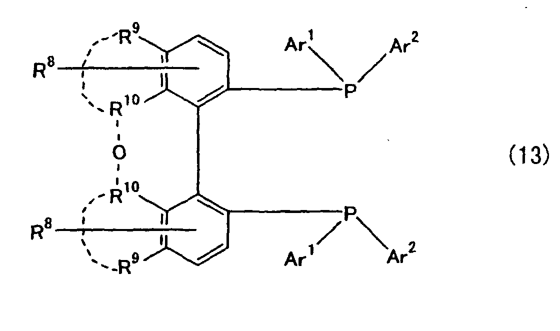 wherein
R⁸ represents:
   (a) hydrogen,
   (b) a lower alkyl group,
   (c) a lower alkoxy group, or
   (d) N(R¹⁴)₂ wherein R¹⁴ represents a lower alkyl group;
R⁹ represents:
   (a) hydrogen,
   (b) a lower alkyl group, or
   (c) a lower alkoxy group;
R¹⁰ represents:
   (a) a lower alkyl group, or
   (b) a lower alkoxy group;
the dashed line linking one R¹⁰ to the other R¹⁰ means that one R¹⁰ may be bonded to the other R¹⁰ via an oxygen atom; one dashed line linking R⁹ to R¹⁰, and the other dashed line linking R⁹ to R¹⁰ independently mean that each pair of R⁹ and R¹⁰ taken together with the benzene ring to which they are attached may form an optionally substituted tetralin ring, an optionally substituted naphthalene ring, or an optionally substituted 1,3-benzodioxole ring;
Ar¹ and Ar² may be the same or different and independently represent a phenyl group substituted with from zero to five substituents selected from straight-chain or branched-chain lower alkyl group, halogen or lower alkoxy group.

In the formula (13), R⁸ represents
(a) hydrogen,
(b) a lower alkyl group,
(c) a lower alkoxy group, or
(d) N(R¹⁴)₂, wherein R¹⁴ represents a lower alkyl group.

As R⁸,
(b) the lower alkyl group may be methyl group, ethyl group, isopropyl group, tert-butyl group, or trifluoromethyl group, with methyl group and trifluoromethyl group being preferred;
(c) the lower alkoxy group may be methoxy group, ethoxy group, isopropoxy group, tert-butoxy group, or trifluoromethoxy group, with methoxy group and trifluoromethoxy group being preferred; and
(d) R¹⁴ in N(R¹⁴)₂ may be methyl group, ethyl group or isopropyl group, with methyl group being preferred.

Among the above, R⁸ is preferably
(a) hydrogen,
(b) a lower alkyl group, or
(c) a lower alkoxy group; and
more preferably
(a) hydrogen, or
(b) a lower alkyl group.

R⁹ represents:
(a)hydrogen,
(b) a lower alkyl group, or
(c) a lower alkoxy group; or alternatively
one dashed line linking R⁹ to R¹⁰, and the other dashed line linking R⁹ to R¹⁰ independently mean that each pair of R⁹ and R¹⁰ taken together with the benzene ring to which they are attached may form:
(a) a tetralin ring optionally substituted with R⁸,
(b) a naphthalene ring optionally substituted with R⁸, or
(c) a 1,3-benzodioxole ring optionally substituted with R⁸.

R¹⁰ represents:
(a) a lower alkyl group, or
(b) a lower alkoxy group; or alternatively,
like the above, one dashed line linking R⁹ to R¹⁰, and the other dashed line linking R⁹ to R¹⁰ independently mean that each pair of R⁹ and R¹⁰ taken together with the benzene ring to which they are attached may form:
(a) a tetralin ring optionally substituted with R⁸,
(b) a naphthalene ring optionally substituted with R⁸, or
(c) a 1,3-benzodioxole ring optionally substituted with R⁸.

As R⁹,
(b) the lower alkyl group may be methyl group, ethyl group, isopropyl group, tert-butyl group, or trifluoromethyl group, with methyl group and trifluoromethyl group being preferred; and
(c) the lower alkoxy group may be methoxy group, ethoxy group, isopropoxy group, tert-butoxy group, or trifluoromethoxy group, with methoxy group and trifluoromethoxy group being preferred.

As R¹⁰,
(a) the lower alkyl group may be methyl group, ethyl group, isopropyl group, tert-butyl group, or trifluoromethyl group, with methyl group and trifluoromethyl group being preferred; and
(b) the lower alkoxy group may be methoxy group, ethoxy group, isopropoxy group, tert-butoxy group, or trifluoromethoxy group, with methoxy group and trifluoromethoxy group being preferred.

Among the above, examples of a preferred embodiment of R⁹ include:
(b) a lower alkyl group,
(c) a lower alkoxy group, and
forming a ring selected from (a) a tetralin ring optionally substituted with R⁸, (b) a naphthalene ring optionally substituted with R⁸ or (c) a 1,3-benzodioxole ring optionally substituted with R⁸, wherein the ring is formed by each pair of R⁹ and R¹⁰ together with the benzene ring to which they are attached. More preferred embodiment of R⁹ may be the forming of a ring selected from (a) a tetralin ring optionally substituted with R⁸, (b) a naphthalene ring optionally substituted with R⁸ and (c) a 1,3-benzodioxole ring optionally substituted with R⁸, wherein the ring is formed by each pair of R⁹ and R¹⁰ together with the benzene ring to which they are attached. Further, the forming of a naphthalene ring optionally substituted with R⁸ may be mentioned as a more preferred embodiment of R⁹.

Among the above, examples of a preferred embodiment of R¹⁰ include
(a) a lower alkyl group,
(b) a lower alkoxy group, and
forming a ring selected from (a) a tetralin ring optionally substituted with R⁸, (b) a naphthalene ring optionally substituted with R⁸ and (c) a 1,3-benzodioxole ring optionally substituted with R⁸, wherein the ring is formed by each pair of R⁹ and R¹⁰ together with the benzene ring to which they are attached. More preferred embodiment of R¹⁰ may be the forming of a ring selected from (a) a tetralin ring optionally substituted with R⁸, (b) a naphthalene ring optionally substituted with R⁸ and (c) a 1,3-benzodioxole ring optionally substituted with R⁸, wherein the ring is formed by each pair of R⁹ and R¹⁰ together with the benzene ring to which they are attached. Further, the forming of a naphthalene ring optionally substituted with R⁸ may be mentioned as a more preferred embodiment of R¹⁰.

The dashed line linking one R¹⁰ to the other R¹⁰ means that one R¹⁰ may be bonded to the other R¹⁰ via an oxygen atom. It is preferred, however, that two R¹⁰ are not bonded to each other.

Art and Ar² independently represent a phenyl group substituted with from zero to five substituents selected from straight-chain or branched-chain lower alkyl group, halogen or lower alkoxy group. As the said substituents
(a) the straight or branched chain lower alkyl group may be methyl group, ethyl group, isopropyl group, or tert-butyl group, with methyl group being preferred;
(b) the halogen substituent may be fluorine, chlorine, bromine, or iodine, with fluorine being preferred; and
(c) the lower alkoxy group may be methoxy group, ethoxy group, isopropoxy group, or tert-butoxy group, with methoxy group being preferred.

Art¹ and Ar² may be the same or different and are preferably a phenyl group substituted with from zero to five substituents set forth above. Further, Ar¹ and Ar² may be the same or different and are also preferably a phenyl group substituted with from two to five substituents set forth above in which at least two substituents are straight or branched chain lower alkyl groups. Further, it may be particularly preferred that Ar¹ and Ar² are the same and are a phenyl group substituted with from two to five substituents set forth above in which at least two substituents are straight or branched chain lower alkyl groups.

Specific examples of Ar¹ and Ar² include phenyl group, m-tolyl group, p-fluorophenyl group, p-chlorophenyl group, 3,5-dimethyl-phenyl group (xylyl group), 3,5-di-tert-butylphenyl group, p-methoxyphenyl group, p-tert-butylphenyl group, p-tolyl group, and 3,5-dimethyl-4-methoxyphenyl group. Preferably, Ar¹ and Ar² each independently may be 3,5-dimethylphenyl group (xylyl group) or 3,5-dimethyl-4-methoxyphenyl group. Further, it may be particularly preferred that Ar¹ and Ar² are the same and represent 3,5-dimethylphenyl group (xylyl group) or 3,5-dimethyl-4-methoxy-phenyl group.

Further, among the optically active bidentate phosphine ligands represented by the general formula (13), the following class of compounds may be preferred. That is, the optically active bidentate phosphine ligands of the formula (13) in which R⁸ represents hydrogen, methyl group, or trifluoromethyl group; R⁹ represents hydrogen, methyl group, trifluoromethyl group, methoxy group, or trifluoromethoxy group, or alternatively R⁹ is bonded to R¹⁰ to form a tetralin ring or a naphthalene ring; R¹⁰ represents methyl group, trifluoromethyl group, methoxy group, or trifluoromethoxy group, or alternatively R¹⁰ is bonded to R⁹ to form a tetralin ring or a naphthalene ring; one R¹⁰ and the other R¹⁰ are not bonded to each other via an oxygen atom; Ar¹ and Ar² are the same and represent phenyl group, m-tolyl group, p-fluorophenyl group, p-chlorophenyl group, 3,5-dimethylphenyl group (xylyl group), 3,5-di-tert-butylphenyl group, p-methoxyphenyl group, p-tert-butylphenyl group, p-tolyl group, or 3,5-dimethyl-4-methoxyphenyl group.

Preferred examples of the optically active bidentate phosphine ligand having the above substituents are mentioned below. That is, such preferred examples include each optical isomer of 2,2'-bis-(diphenylphosphino)-1,1'-binaphthyl (abbreviated name: BINAP); BINAP derivatives in which the naphthalene ring of BINAP is partially reduced, such as each optical isomer of 2,2'-bis(diphenylphosphino)-5,5',6,6',7,7',8,8'-octahydro-1,1'-binaphthyl (abbreviated name: H₈BINAP); BINAP derivatives in which the naphthalene ring of BINAP carries substituent(s), such as each optical isomer of 2,2'-bis-(diphenylphosphino)-6,6'-dimethyl-1,1'-binaphthyl (abbreviated name: 6MeBINAP); BINAP derivatives in which the benzene ring on the phosphorus atom of BINAP is substituted with lower alkyl group(s), such as each optical isomer of 2,2'-bis-(di-p-tolylphosphino)-1,1'-binaphthyl (abbreviated name: Tol-BINAP), each optical isomer of 2,2'-bis[bis(3-methylphenyl)phosphino]-1,1'-binaphthyl, each optical isomer of 2,2'-bis[bis(3,5-di-tert-butylphenyl)phosphino]-1,1'-binaphthyl, each optical isomer of 2,2'-bis[bis(4-tert-butylphenyl)phosphino]-1,1'-binaphthyl, each optical isomer of 2,2'-bis[bis(3,5-dimethylphenyl)phosphino]-1,1'-binaphthyl (abbreviated name: Xyl-BINAP), and each optical isomer of 2,2'-bis[bis(3,5-dimethyl-4-methoxyphenyl)phosphino]-1,1'-binaphthyl (abbreviated name: Dmanyl-BINAP); BINAP derivatives in which the naphthalene ring of BINAP carries substituent(s) and the benzene ring on the phosphorus atom of BINAP is substituted with from 1 to 5 lower alkyl substituents, such as each optical isomer of 2,2'-bis[bis-(3,5-dimethylphenyl)phosphino]-6,6'-dimethyl-1,1'-binaphthyl (abbreviated name: Xyl-6MeBINAP); and, BINAP derivatives in which the naphthalene ring of BINAP is condensed with a saturated hydrocarbon ring, such as each optical isomer of 3,3'-bis-(diphenylphosphanyl)-13,13'-dimethyl-12,13,14,15,16,17,12',13',14',15',16',17'-dodecahydro-11H,11'H-[4,4']bi[cyclopenta[a]phenanthrenyl]. Further, particularly preferred examples include each optical isomer of 2,2'-bis[bis-(3,5-dimethylphenyl)phosphino]-1,1'-binaphthyl (abbreviated name: Xyl-BINAP); each optical isomer of 2,2'-bis[bis(3,5-dimethyl-4-methoxyphenyl)phosphino]-1,1'-binaphthyl (abbreviated name: Dmanyl-BINAP); and BINAP derivatives in which the naphthalene ring of BINAP carries substituent(s) and the benzene ring on the phosphorus atom of BINAP is substituted with from 1 to 5 lower alkyl substituents, such as each optical isomer of 2,2'-bis[bis-(3,5-dimethylphenyl)phosphino]-6,6'-dimethyl-1,1'-binaphthyl (abbreviated name: Xyl-6MeBINAP).

Further, each optical isomer of 2,2'-bis[bis(4-fluorophenyl)phosphino]-1,1'-binaphthyl, and each optical isomer of 2,2'-bis-[bis(4-chlorophenyl)phosphino]-1,1'-binaphthyl may be also mentioned as examples of BINAP derivatives carrying fluorine substituent(s) and/or chlorine substituent(s).

In addition, bidentate tertiary phosphine compounds other than the BINAP derivatives, such as each optical isomer of 2,2'-bis(diphenylphosphino)-6,6'-disubstituted-1,1'-biphenyl derivatives, may also be preferred. Specific examples thereof may be biphenyl derivatives including each optical isomer of 6,6'-bis(diphenylphosphino)-3,3'-dimethoxy-2,2',4,4'-tetramethyl-1,1'-biphenyl, each optical isomer of 6,6'-bis[bis(4-methoxyphenyl)phosphanyl]-3,3'-dimethoxy-2,4,2',4'-tetramethylbiphenyl, each optical isomer of 2,2'-bis(diphenylphosphino)-6,6'-dimethylbiphenyl, each optical isomer of 2,2'-bis(diphenylphosphanyl)-4,6,4',6'-tetramethylbiphenyl, each optical isomer of [4,4'-bis(dimethylamino)-6,6'-dimethylbiphenyl-2,2'-diyl]bis(diphenylphosphine), each optical isomer of 5,7-dihydrodibenzo[c,e]oxepin-1,11-bis(diphenylphosphine), and each optical isomer of [(5,6), (5',6')-bis(methylenedioxy)biphenyl-2,2'-diyl]bis(diphenylphosphine) (abbreviated name: SEGPHOS). Of course, optically active bidentate phosphine ligands which can be used according to the present invention should not be limited to the above.

These optically active BINAP derivatives may be prepared according to the process using a nickel catalyst described in the literature (Dongwei Cai, et al., J. Org. Chem., 59, pp. 7180-7181 (1994); or Scott A. Laneman, et al., Chem. Commun., pp. 2359-2360 (1997)), or the process comprising reducing BINAPO derivatives (obtained by oxidizing trivalent phosphorus atom of BINAP derivatives to pentavalent phosphorus atom) with trichlorosilane described in the literature (Hidemasa Takaya; Susumu Akutagawa; Ryoji Noyori, et al., J. Org. Chem., 51, pp. 629-635 (1986)), or the process described in JP-B-07-68260. Further, the above optically active biphenyl derivatives may be prepared by the process for preparing the BINAP derivatives set forth above. Further, optically active biphenyl derivatives in which the benzene ring of the biphenyl carries alkoxy substituent(s) may be prepared according to the process described in JP-A-11-269185.

On the other hand, the diamine ligand is represented by the general formula (14): 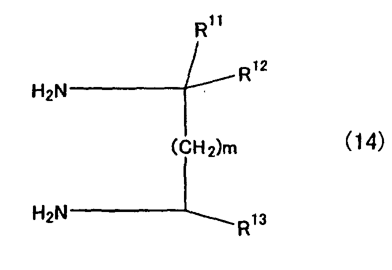 wherein m represents an integer of from 0 to 2,
R¹¹ represents:
   (a) hydrogen,
   (b) a straight-chain lower alkyl group,
   (c) a branched-chain lower alkyl group,
   (d) a cyclic lower alkyl group,
   (e) a phenyl group substituted with from zero to five lower alkyl or lower alkoxy groups,
   (f) a 1-naphthyl group substituted with from zero to seven lower alkyl or lower alkoxy groups, or
   (g) a 2-naphthyl group substituted with from zero to seven lower alkyl or lower alkoxy groups;
provided that (I) when R¹¹ is hydrogen, then R¹² and R¹³ are independently represent:
   (a) hydrogen,
   (b) a straight-chain lower alkyl group,
   (c) a branched-chain lower alkyl group,
   (d) a cyclic lower alkyl group,
   (e) a phenyl group substituted with from zero to five lower alkyl or lower alkoxy groups,
   (f) a 1-naphthyl group substituted with from zero to seven lower alkyl or lower alkoxy groups, or
   (g) a 2-naphthyl group substituted with from zero to seven lower alkyl or lower alkoxy groups, or
   (h) alternatively, R¹² and R¹³ may be bonded to each other to form a ring selected from:
      (h-1) a cycloalkyl ring, or
      (h-2) a heteroatom-containing heterocyclic ring; or
(II) when R¹¹ is other than hydrogen, then R¹² represents:
   (a) a straight-chain lower alkyl group,
   (b) a branched-chain lower alkyl group,
   (c) a cyclic lower alkyl group,
   (d) a phenyl group substituted with from zero to five lower alkyl or lower alkoxy groups,
   (e) a 1-naphthyl group substituted with from zero to seven lower alkyl or lower alkoxy groups, or
   (f) a 2-naphthyl group substituted with from zero to seven lower alkyl or lower alkoxy groups; and
R¹³ represents:
   (a) hydrogen,
   (b) a lower alkyl group, or
   (c) a benzyl group.

In the general formula (14), m represents an integer of from 0 to 2, with 0 being preferred.

R¹¹ represents
(a) hydrogen,
(b) a straight-chain lower alkyl group,
(c) a branched-chain lower alkyl group,
(d) a cyclic lower alkyl group,
(e) a phenyl group substituted with from zero to five lower alkyl or lower alkoxy groups,
(f) a 1-naphthyl group substituted with from zero to seven lower alkyl or lower alkoxy groups, or
(g) a 2-naphthyl group substituted with from zero to seven lower alkyl or lower alkoxy group.

Examples of the group represented by R¹¹ are as follows.

Examples of
(b) a straight-chain lower alkyl group,
(c) a branched-chain lower alkyl group, or
(d) a cyclic lower alkyl group
include, but are not limited to, methyl group, ethyl group, n-propyl group, isopropyl group, n-butyl group, sec-butyl group, isobutyl group, tert-butyl group, cyclopropyl group, cyclobutyl group, cyclopentyl group, cyclohexyl group, and cycloheptyl group, with cyclobutyl group, cyclopentyl group and cyclohexyl group being preferred.

Examples of (e) a phenyl group substituted with from zero to five lower alkyl or lower alkoxy groups include, but are not limited to, phenyl group, 2,3-dimethylphenyl group, 2,4-dimethylphenyl group, 2,5-dimethylphenyl group, 2,6-dimethylphenyl group, 3,4-dimethylphenyl group, 3,5-dimethylphenyl group, 2,3,4-trimethylphenyl group, 2,3,5-trimethylphenyl group, 2,3,6-trimethylphenyl group, 2,4,5-trimethylphenyl group, 2,4,6-trimethylphenyl group, 3,4,5-trimethylphenyl group, 2-methoxyphenyl group, 3-methoxyphenyl group, 4-methoxyphenyl group, 2,3-dimethoxyphenyl group, 2,4-dimethoxyphenyl group, 2,5-dimethoxyphenyl group, 2,6-dimethoxyphenyl group, 3,4-dimethoxyphenyl group, 3,5-dimethoxyphenyl group, 2,3,4-trimethoxyphenyl group, 2,3,5-trimethoxyphenyl group, 2,3,6-trimethoxyphenyl group, 2,4,5-trimethoxyphenyl group, 2,4,6-trimethoxyphenyl group, and 3,4,5-trimethoxyphenyl group. Preferred examples thereof includes 3,4-dimethylphenyl group, 3,5-dimethylphenyl group, 3,4,5-trimethylphenyl group, 3-methoxyphenyl group, 4-methoxyphenyl group, 3,4-dimethoxyphenyl group, 3,5-dimethoxyphenyl group, and 3,4,5-trimethoxyphenyl group, with 3,5-dimethylphenyl group, 4-methoxyphenyl group, 3,4-dimethoxyphenyl group, and 3,5-dimethoxyphenyl group being more preferred.

Preferred examples of (f) a 1-naphthyl group substituted with from zero to seven lower alkyl or lower alkoxy groups include, but are not limited to, 4-methoxy-1-naphthyl group.

Preferred examples of (g) a 2-naphthyl group substituted with from zero to seven lower alkyl or lower alkoxy groups include, but are not limited to, 1-methoxy-2-naphthyl group and 4-methoxy-2-naphthyl group.

Among the above, R¹¹ is preferably
(a) hydrogen, or
(e) a phenyl group substituted with from zero to five lower alkyl or lower alkoxy groups; and
   particularly preferably
(e) a phenyl group substituted with from zero to five lower alkyl or lower alkoxy groups.

(I) When R¹¹ is (a) hydrogen, then R¹² and R¹³ are independently represent:
   (Ia) hydrogen,
   (Ib) a straight-chain lower alkyl group,
   (Ic) a branched-chain lower alkyl group,
   (Id) a cyclic lower alkyl group,
   (Ie) a phenyl group substituted with from zero to five lower alkyl or lower alkoxy groups,
   (If) a 1-naphthyl group substituted with from zero to seven lower alkyl or lower alkoxy groups, or
   (Ig) a 2-naphthyl group substituted with from zero to seven lower alkyl or lower alkoxy groups, or
   (Ih) alternatively, R¹² and R¹³ may be bonded to each other to form a ring selected from:
      (h-1) a cycloalkyl ring, or
      (h-2) a heteroatom-containing heterocyclic ring; or, alternatively,
(II) when R¹¹ is other than hydrogen, then R¹² represents:
   (IIa) a straight-chain lower alkyl group,
   (IIb) a branched-chain lower alkyl group,
   (IIc) a cyclic lower alkyl group,
   (IId) a phenyl group substituted with from zero to five lower alkyl or lower alkoxy groups,
   (IIe) a 1-naphthyl group substituted with from zero to seven lower alkyl or lower alkoxy groups, or
   (IIf) a 2-naphthyl group substituted with from zero to seven lower alkyl or lower alkoxy groups; and
R¹³ represents:
   (IIg) hydrogen,
   (IIh) a lower alkyl group, or
   (IIi) a benzyl group.

(I) When R¹¹ is (a) hydrogen, examples of the groups represented by R¹² and R¹³ are each independently as follows.
   Examples of
   (Ib) a straight-chain lower alkyl group,
   (Ic) a branched-chain lower alkyl group, or
   (Id) a cyclic lower alkyl group,
   include, but are not limited to, methyl group, ethyl group, n-propyl group, isopropyl group, n-butyl group, sec-butyl group, isobutyl group, tert-butyl group, cyclopropyl group, cyclobutyl group, cyclopentyl group, cyclohexyl group, and cycloheptyl group, with cyclobutyl group, cyclopentyl group, and cyclohexyl group being preferred.

Examples of (Ie) a phenyl group substituted with from zero to five lower alkyl or lower alkoxy groups include, but are not limited to, phenyl group, 2,3-dimethylphenyl group, 2,4-dimethyl-phenyl group, 2,5-dimethylphenyl group, 2,6-dimethylphenyl group, 3,4-dimethylphenyl group, 3,5-dimethylphenyl group, 2,3,4-trimethylphenyl group, 2,3,5-trimethylphenyl group, 2,3,6-trimethylphenyl group, 2,4,5-trimethylphenyl group, 2,4,6-trimethylphenyl group, 3,4,5-trimethylphenyl group, 2-methoxyphenyl group, 3-methoxyphenyl group, 4-methoxyphenyl group, 2,3-dimethoxyphenyl group, 2,4-dimethoxyphenyl group, 2,5-dimethoxyphenyl group, 2,6-dimethoxyphenyl group, 3,4-dimethoxyphenyl group, 3,5-dimethoxyphenyl group, 2,3,4-trimethoxyphenyl group, 2,3,5-trimethoxyphenyl group, 2,3,6-trimethoxyphenyl group, 2,4,5-trimethoxyphenyl group, 2,4,6-trimethoxyphenyl group, and 3,4,5-trimethoxyphenyl group. Preferred examples thereof include phenyl group, 3,4-dimethylphenyl group, 3,5-dimethylphenyl group, 3,4,5-trimethylphenyl group, 3-methoxyphenyl group, 4-methoxyphenyl group, 3,4-dimethoxyphenyl group, 3,5-dimethoxyphenyl group, and 3,4,5-trimethoxyphenyl group, with phenyl group, 3,5-dimethylphenyl group, 4-methoxyphenyl group, 3,4-dimethoxyphenyl group, and 3,5-dimethoxyphenyl group being more preferred.

Preferred examples of (If) a 1-naphthyl group substituted with from zero to seven lower alkyl or lower alkoxy groups include, but are not limited to, 1-naphthyl group and 4-methoxy-1-naphthyl group.

Preferred examples of (Ig) a 2-naphthyl group substituted with from zero to seven lower alkyl or lower alkoxy groups include, but are not limited to, 2-naphthyl group, 1-methoxy-2-naphthyl group, and 4-methoxy-2-naphthyl group.

Alternatively, (Ih) R² and R³ may be bonded to each other to form a ring, and examples thereof are as follows.

Examples of (Ih-1) a cycloalkyl ring include, but are not limited to, cyclopropane ring, cyclobutane ring, cyclopentane ring, cyclohexane ring, cycloheptane ring, and cyclooctane ring, with cyclohexane ring being preferred;

Examples of (Ih-2) a heteroatom-containing heterocyclic ring include, but are not limited to, tetrahydrofuran ring, tetrahydropyran ring, and dioxane ring.

Among the above, R¹² and R¹³ may be preferably:
(Ie) a phenyl group substituted with from zero to five lower alkyl or lower alkoxy groups,
(If) a 1-naphthyl group substituted with from zero to seven lower alkyl or lower alkoxy groups, or
(Ig) a 2-naphthyl group substituted with from zero to seven lower alkyl or lower alkoxy groups, or
(Ih) alternatively R¹² and R¹³ may be preferably bonded to each other to form (Ih-1) a cycloalkyl ring.
R¹² and R¹³ may be particularly preferably:
(Ie) a phenyl group substituted with from zero to five lower alkyl or lower alkoxy group, or
(Ih) alternatively R¹² and R¹³ may be particularly preferably bonded to each other to form (Ih-1) a cycloalkyl ring.
However, R¹² and R¹³ are not limited to the above.

Further, (II) when R¹¹ is other than hydrogen, examples of the groups represented by R¹² are as follows.

Examples of
(IIa) a straight-chain lower alkyl group,
(IIb) a branched-chain lower alkyl group, or
(IIc) a cyclic lower alkyl group,
include, but are not limited to, methyl group, ethyl group, n-propyl group, isopropyl group, n-butyl group, sec-butyl group, isobutyl group, tert-butyl group, cyclopropyl group, cyclobutyl group, cyclopentyl group, cyclohexyl group, and cycloheptyl group, with cyclobutyl group, cyclopentyl group, and cyclohexyl group being preferred.

Examples of (IId) a phenyl group substituted with from zero to five lower alkyl or lower alkoxy groups include, but are not limited to, phenyl group, 2,3-dimethylphenyl group, 2,4-dimethylphenyl group, 2,5-dimethylphenyl group, 2,6-dimethylphenyl group, 3,4-dimethylphenyl group, 3,5-dimethylphenyl group, 2,3,4-trimethylphenyl group, 2,3,5-trimethylphenyl group, 2,3,6-trimethylphenyl group, 2,4,5-trimethylphenyl group, 2,4,6-trimethylphenyl group, 3,4,5-trimethylphenyl group, 2-methoxy-phenyl group, 3-methoxyphenyl group, 4-methoxyphenyl group, 2,3-dimethoxyphenyl group, 2,4-dimethoxyphenyl group, 2,5-dimethoxyphenyl group, 2,6-dimethoxyphenyl group, 3,4-dimethoxyphenyl group, 3,5-dimethoxy-phenyl group, 2,3,4-trimethoxyphenyl group, 2,3,5-trimethoxyphenyl group, 2,3,6-trimethoxyphenyl group, 2,4,5-trimethoxyphenyl group, 2,4,6-trimethoxyphenyl group, and 3,4,5-trimethoxyphenyl group. Preferred examples thereof include phenyl group, 3,4-dimethylphenyl group, 3,5-dimethylphenyl group, 3,4,5-trimethylphenyl group, 3-methoxyphenyl group, 4-methoxyphenyl group, 3,4-dimethoxyphenyl group, 3,5-dimethoxyphenyl group, and 3,4,5-trimethoxyphenyl group, with phenyl group, 3,5-dimethylphenyl group, 4-methoxyphenyl group, 3,4-dimethoxyphenyl group, and 3,5-dimethoxyphenyl group being more preferred.

Preferred examples of (IIe) a 1-naphthyl group substituted with from zero to seven lower alkyl or lower alkoxy groups include, but are not limited to, 1-naphthyl group and 4-methoxy-1-naphthyl group.

Preferred examples of (IIf) a 2-naphthyl group substituted with from zero to seven lower alkyl or lower alkoxy groups include, but are not limited to, 2-naphthyl group, 1-methoxy-2-naphthyl group and 4-methoxy-2-naphthyl group.

Then, examples of the groups represented by R¹³ are as follows.

Examples of (IIh) a lower alkyl group include, but are not limited to, methyl group, ethyl group, isopropyl group, and isobutyl group. Preferred examples include methyl group and isopropyl group, with methyl group being particularly preferred.
(II) When R¹¹ is other than hydrogen, R¹² may be preferably
(IId) a phenyl group substituted with from zero to five lower alkyl or lower alkoxy groups,
(IIe) a 1-naphthyl group substituted with from zero to seven lower alkyl or lower alkoxy groups, or
(IIf) a 2-naphthyl group substituted with from zero to seven lower alkyl or lower alkoxy groups; and
   particularly preferably
(IId) a phenyl group substituted with from zero to five lower alkyl or lower alkoxy groups;
more preferably, R¹² may be the same with R¹¹. However, R¹² is not limited to the above.

Then, R¹³ may be preferably
(IIh) a lower alkyl group, or
(IIi) a benzyl group;
   more preferably
(IIh) a lower alkyl group.
However, R¹³ is not limited to the above.

Among the diamine ligands represented by the general formula (14), the following class of compounds may be preferred. That is, the diamine ligands of the formula (14) in which m is 0; R¹¹ represents 3,5-dimethylphenyl group, 4-methoxyphenyl group, 3,4-dimethoxyphenyl group, or 3,5-dimethoxyphenyl group; R¹² is the same with R¹¹ and represents 3,5-dimethylphenyl group, 4-methoxyphenyl group, 3,4-dimethoxyphenyl group, or 3,5-dimethoxyphenyl group; and R¹³ represents methyl group, isopropyl group or benzyl group.

Examples of the diamine ligand are mentioned below. That is, such examples include methylenediamine, ethylenediamine, 1,2-diaminopropane, 1,3-diaminopropane, 1,4-diaminobutane, 2,3-diaminobutane, 1,2-cyclopentanediamine, 1,2-cyclohexanediamine, 1,1-diphenylethylenediamine, 1,1-di(p-methoxyphenyl)ethylene-diamine, 1,1-di(3,5-dimethoxyphenyl)ethylenediamine, and 1,1-dinaphthylethylenediamine. Optically active diamine compounds may be also used. Examples thereof include, for example, optically active 1,2-diphenylethylenediamine (abbreviated name: DPEN), 1,2-di(p-methoxyphenyl)ethylenediamine, 1,2-cyclohexanediamine, 1,2-cycloheptanediamine, 2,3-dimethylbutanediamine, 1-methyl-2,2-diphenylethylenediamine, 1-isobutyl-2,2-diphenylethylenediamine, 1-isopropyl-2,2-diphenylethylenediamine, 1-benzyl-2,2-diphenylethylenediamine, 1-methyl-2,2-di(p-methoxyphenyl)ethylenediamine (abbreviated name: DAMEN), 1-isobutyl-2,2-di(p-methoxyphenyl)-ethylenediamine (abbreviated name: DAIBEN), 1-isopropyl-2,2-di(p-methoxyphenyl)ethylenediamine (abbreviated name: DAIPEN), 1-benzyl-2,2-di(p-methoxyphenyl)ethylenediamine, 1-methyl-2,2-di(3,5-dimethoxyphenyl)ethylenediamine, 1-isopropyl-2,2-di(3,5-dimethoxyphenyl)ethylenediamine, 1-isobutyl-2,2-di(3,5-dimethoxy-phenyl)ethylenediamine, 1-benzyl-2,2-di(3,5-dimethoxyphenyl)ethylenediamine, 1-methyl-2,2-dinaphthyiethylenediamine, 1-isobutyl-2,2-dinaphthylethylenediamine, 1-isopropyl-2,2-dinaphthylethylenediamine, and 1-benzyl-2,2-dinaphthylethylenediamine.

Further, optically active diamine compounds which can be used are not limited to the abovementioned optically active ethylenediamine derivatives. Optically active propanediamine, butanediamine and cyclohexanediamine derivatives may be also used.

In addition, these diamine ligands may be prepared by the process starting from α-amino acid described in the literature (Burrows, C. J., et al., Tetrahedron Letters, 34(12), pp. 1905-1908 (1993)), or by a variety of processes described in the general remark (T. Le Gall, C. Mioskowski, and D. Lucet, Angew. Chem. Int. Ed., 37, pp. 2580-2627 (1998)).

Preferred examples of the optically active diamine ligand are mentioned below. That is, the preferred examples include 1-methyl-2,2-diphenylethylenediamine, 1-isobutyl-2,2-diphenylethylenediamine, 1-isopropyl-2,2-diphenylethylenediamine, 1-benzyl-2,2-diphenylethylenediamine, 1-methyl-2,2-di(p-methoxyphenyl)ethylenediamine (abbreviated name: DAMEN), 1-isobutyl-2,2-di(p-methoxyphenyl)ethylenediamine (abbreviated name: DAIBEN), 1-isopropyl-2,2-di(p-methoxyphenyl)ethylenediamine (abbreviated name: DAIPEN), 1-benzyl-2,2-di(p-methoxyphenyl)ethylenediamine, 1-methyl-2,2-di(3,5-dimethoxyphenyl)ethylenediamine, 1-isopropyl-2,2-di(3,5-dimethoxyphenyl)ethylenediamine, 1-isobutyl-2,2-di(3,5-dimethoxyphenyl)ethylenediamine, and 1-benzyl-2,2-di(3,5-dimethoxyphenyl)ethylenediamine.

Further, more preferred examples of the optically active diamine ligand include 1-methyl-2,2-di(p-methoxyphenyl)ethylenediamine (abbreviated name: DAMEN), 1-isopropyl-2,2-di(p-methoxyphenyl)ethylenediamine (abbreviated name: DAIPEN), 1-benzyl-2,2-di(p-methoxyphenyl)ethylenediamine, 1-methyl-2,2-di(3,5-dimethoxy-phenyl)ethylenediamine, 1-isopropyl-2,2-di(3,5-dimethoxyphenyl)ethylenediamine, and 1-benzyl-2,2-di(3,5-dimethoxyphenyl)ethylenediamine.

The ruthenium/bidentate phosphine/diamine complex to be used by the present invention may be prepared by the known process described in JP-A-11-189600. More specifically, the complex may be synthesized by reacting the ruthenium compound as a starting material with the bidentate phosphine ligand and the diamine ligand sequentially or inversely or simultaneously. As a ruthenium compound which is a starting material for synthesizing the complex, zerovalent, monovalent, divalent, trivalent or highvalent ruthenium compounds may be used. When a zerovalent or monovalent ruthenium compound is used, it is needed to oxidize the ruthenium atom before the final reaction step. When a divalent ruthenium compound is used, the complex may be synthesized by reacting the ruthenium compound with the phosphine ligand and the diamine ligand sequentially or inversely or simultaneously. When a trivalent or polyvalent ruthenium compound is used as a starting material, it is needed to reduce the ruthenium atom before the final reaction step.

Examples of a starting ruthenium compound to be used include inorganic ruthenium compounds, such as ruthenium(III) chloride hydrate, ruthenium(III) bromide hydrate, and ruthenium(III) iodide hydrate; ruthenium compounds coordinated with diene, such as [ruthenium(norbornadiene) dichloride] polymer complex, [ruthenium(cyclooctadiene) dichloride] polymer complex, and bis[(methylallyl)ruthenium(cyclooctadiene)]; ruthenium complex compounds coordinated with aromatic compound, such as [ruthenium(benzene) dichloride] dimer complex, [ruthenium(p-cymene) dichloride] dimer complex, [ruthenium(trimethylbenzene) dichloride] dimer complex, and [ruthenium(hexamethylbenzene) dichloride] dimer complex; and ruthenium complex compounds coordinated with phosphine, such as dichlorotris(triphenylphosphine)ruthenium. In addition to the abovementioned neutral ruthenium complexes, cationic ruthenium complexes, such as [chlororuthenium(binap)(benzene)] chloride, and [chlororuthenium(binap)(p-cymene)] chloride (described in J. Org. Chem., 59, p 3064 (1994)), and anionic complexes may be used. Further, ruthenium compounds to be used are not limited to the compounds mentioned above as long as they have ligands which can be substituted for bidentate phosphine ligand and diamine ligand. As the starting material, a variety of ruthenium compounds described in, for example, COMPREHENSIVE ORGANOMETALLIC CHEMISTRY II, Vol. 7, pp. 294-296 (PERGAMON) may be used.

A preferred example of the process uses a divalent ruthenium compound as the starting ruthenium compound and comprises reacting the ruthenium compound with the bidentate phosphine compound and the diamine compound sequentially or inversely or simultaneously. For example, a ruthenium compound coordinated with diene, such as [ruthenium(norbornadiene) dichloride] polymer complex, [ruthenium(cyclooctadiene) dichloride] polymer complex or bis[(methylallyl)ruthenium(cyclooctadiene)], a ruthenium complex compound coordinated with aromatic compound, such as [ruthenium(benzene) dichloride] dimer complex, [ruthenium(p-cymene) dichloride] dimer complex, [ruthenium(trimethylbenzene) dichloride] dimer complex or [ruthenium(hexamethylbenzene) dichloride] dimer complex, or a ruthenium complex compound coordinated with phosphine, such as dichlorotris(triphenylphosphine)ruthenium, may be reacted with a bidentate phosphine compound in aromatic hydrocarbon solvent (such as toluene or xylene), aliphatic hydrocarbon solvent (such as pentane or hexane), halogen-containing hydrocarbon solvent (such as dichloromethane), ether type solvent (such as diethyl ether or tetrahydrofuran), alcohol type solvent (such as methanol, ethanol, 2-propanol, butanol or benzyl alcohol), or heteroatom-containing organic solvent (such as acetonitrile, N,N-dimethylformamide (DMF), N,N-dimethylacetamide(DMA), N-methylpyrrolidone (NMP), or dimethylsulfoxide (DMSO)) at reaction temperatures ranging from -100°C to 200°C to obtain a bidentate phosphine/ruthenium halide complex. More preferably, a bidentate phosphine/ruthenium halide complex may be obtained by the process described in Masato Kitamura; Makoto Tokunaga; Takeshi Ohkuma; and Ryoji Noyori, Organic Synthesis, 71, pp. 1-13 (1993), which process comprises heating a commercially available compound of [RuCl₂(C₆H₆)]₂ and a bidentate phosphine compound in an amount of from 1.0 to 1.05 mol for 1 mol of the Ru metal in a solvent selected from DMF and DMA (the amount of the solvent to be used is from 10 to 20 mL per mmol of the bidentate phosphine compound) at 100°C for from 10 to 15 minutes; and distilling off the solvent.

The thus obtained bidentate phosphine/ruthenium halide complex may be reacted with a diamine compound in aromatic hydrocarbon solvent (such as toluene or xylene), aliphatic hydrocarbon solvent (such as pentane or hexane), halogen-containing hydrocarbon solvent (such as dichloromethane), ether type solvent (such as diethyl ether or tetrahydrofuran), alcohol type solvent (such as methanol, ethanol, 2-propanol, butanol, or benzyl alcohol), or heteroatom-containing organic solvent (such as acetonitrile, DMF, DMA, NMP, or DMSO) at reaction temperatures ranging from -100°C to 200°C to yield a diamine/bidentate phosphine/ruthenium halide complex. The reaction is preferably carried out in a halogen-containing hydrocarbon solvent such as dichloromethane at temperatures ranging from 10°C to 30°C for from 0.5 to 1.5 hours to yield a diamine/bidentate phosphine/ruthenium halide complex. Further, under the same reaction conditions, a diamine/bidentate phosphine/ruthenium halide complex may be also obtained by reacting a cationic ruthenium complex such as [chlororuthenium-(binap) (benzene)] chloride with a diamine compound.

Further, according to one of the most preferred characteristic embodiments of the present invention, the process uses a diamine/bidentate phosphine/ruthenium halide complex obtained as set forth above as a catalyst precursor and comprises a step of previously activating the complex precursor by a hydrogenation reaction in the presence of a base or by a hydride-forming reaction under a hydrogen transfer reducing reaction condition; and then a step of reacting a ketone compound containing a nitrogenous or oxygenic functional group. Specifically, a diamine/bidentate phosphine/ruthenium hydride complex may be obtained by reacting a diamine/bidentate phosphine/ruthenium halide complex with hydrogen, a metal hydride (such as sodium borohydride or lithium aluminium hydride), an organometallic compound (such as methylmagnesium bromide, ethylmagnesium bromide, propylmagnesium bromide, methyllithium, ethyllithium, or propyllithium), or an alkali or alkaline earth metal salt (such as KOH, K₂CO₃, KOCH₃, KOCH(CH₃)₂, KC₁₀H₈, tert-BuOK, NaOH, Na₂CO₃, NaOCH₃, NaOCH(CH₃)₂, LiOH, LiOCH₃, or LiOCH(CH₃)₂) in aromatic hydrocarbon solvent (such as toluene or xylene), aliphatic hydrocarbon solvent (such as pentane or hexane), halogen-containing hydrocarbon solvent (such as dichloromethane), ether type solvent (such as diethyl ether or tetrahydrofuran), alcohol type solvent (such as methanol, ethanol, 2-propanol, butanol, or benzyl alcohol), or heteroatom-containing organic solvent (such as acetonitrile, DMF, DMA, NMP, or DMSO), preferably, in 2-propanol, at temperatures ranging from -100°C to 200°C.

Most preferably, the process comprises reacting a diamine/bidentate phosphine/ruthenium halide complex in the presence of a base such as potassium tert-butoxide in an amount of from 2 to 10 mol of for 1 mol of the halide complex, in a degassed alcohol type solvent such as 2-propanol at reaction temperatures ranging from 55°C to 65°C for 0.5 hour or more to activate the catalyst; then adding a solution of a ketone compound having a nitrogenous or oxygenic functional group in a degassed alcohol solvent such as 2-propanol to the activated catalyst, or alternatively adding a solution of the catalyst active species to a solution of the ketone compound; then introducing hydrogen into the reactor until a required pressure is obtained; and vigorously stirring the reaction liquid to yield a diamine/bidentate phosphine/ruthenium hydride complex and, at the same time, to allow asymmetric hydrogenation of the ketone compound to directly proceed.

Further, a diamine/bidentate phosphine/ruthenium hydride complex may be also obtained by first converting a bidentate phosphine/ruthenium halide complex into the corresponding bidentate phosphine/ruthenium hydride complex, followed by a reaction with a diamine compound. For example, RuH₂(tolbinap)(dpen) may be obtained by converting RuCl₂(tolbinap) (dmf)n into RuH₂(tolbinap)(dmf)n (wherein dmf represents dimethylformamide), followed by a reaction with DPEN. Further, a bidentate phosphine/ruthenium hydride complex may be obtained by reacting a bidentate phosphine/ruthenium halide complex with hydrogen, a metal hydride (such as sodium borohydride or lithium aluminium hydride), an organometallic compound (such as methylmagnesium bromide, ethylmagnesium bromide, propylmagnesium bromide, methyllithium, ethyllithium, or propyllithium), or an alkali or alkaline earth metal salt (such as KOH, K₂CO₃, KOCH₃, KOCH(CH₃)₂, KC₁₀H₈, tert-BuOK, NaOH, Na₂CO₃, NaOCH₃, NaOCH(CH₃)₂, LiOH, LiOCH₃, or LiOCH(CH₃)₂) in aromatic hydrocarbon solvent (such as toluene or xylene), aliphatic hydrocarbon solvent (such as pentane or hexane), halogen-containing hydrocarbon solvent (such as dichloromethane), ether type solvent (such as diethyl ether or tetrahydrofuran), alcohol type solvent (such as methanol, ethanol, 2-propanol, butanol, or benzyl alcohol), or heteroatom-containing organic solvent (such as acetonitrile, DMF, DMA, NMP, or DMSO) at temperatures ranging from -100°C to 200°C.

The thus obtained bidentate phosphine/ruthenium hydride complex can be converted into the corresponding diamine/bidentate phosphine/ruthenium hydride complex by a reaction with a diamine compound which may be carried out in aromatic hydrocarbon solvent (such as toluene or xylene), aliphatic hydrocarbon solvent (such as pentane or hexane), halogen-containing hydrocarbon solvent (such as dichloromethane), ether type solvent (such as diethyl ether or tetrahydrofuran), alcohol type solvent (such as methanol, ethanol, 2-propanol, butanol, or benzyl alcohol), or heteroatom-containing organic solvent (such as acetonitrile, DMF, DMA, NMP, or DMSO) at temperatures ranging from -100°C to 200°C.

Examples of ruthenium/bidentate phosphine/diamine complex which may be synthesized as set forth above, include trans-RuCl₂[(S)-xylbinap][(S)-damen], trans-RuCl₂[(R)-xylbinap][(R)-damen], trans-RuCl₂[(S)-xylbinap][(R)-damen], trans-RuCl₂[(R)-xylbinap][(S)-damen], trans-RuCl₂[(S)-xylbinap][(S)-daipen], trans-RuCl₂[(R)-xylbinap][(R)-daipen], trans-RuCl₂[(S)-xylbinap][(R)-daipen], trans-RuCl₂[(R)-xylbinap][(S)-daipen], trans-RuCl₂[(S)-tolbinap][(S)-daipen], trans-RuCl₂[(R)-tolbinap][(R)-daipen], trans-RuCl₂[(R)-tolbinap][(S)-daipen], trans-RuCl₂[(S)-tolbinap]-[(R)-daipen], trans-RuCl₂[(S)-xylbinap][(S,S)-dpen], trans-RuCl₂-[(R)-xylbinap][(R,R)-dpen], trans-RuCl₂[(S)-xylbinap][(R,R)-dpen], trans-RuCl₂[(R)-xylbinap][(S,S)-dpen], trans-RuCl₂[(S)-dmanylbinap][(S)-damen], trans-RuCl₂[(R)-dmanylbinap] [(R)-damen], trans-RuCl₂[(S)-dmanylbinap][(R)-damen], trans-RuCl₂[(R)-dmanylbinap][(S)-damen], trans-RuCl₂[(S)-6mebinap][(S)-damen], trans-RuCl₂[(R)-6mebinap][(R)-damen], trans-RuCl₂[(S)-6mebinap]-[(R)-damen], trans-RuCl₂[(R)-6mebinap][(S)-damen], trans-RuCl₂[(S)-xyl-6mebinap][(S)-damen], trans-RuCl₂[(R)-xyl-6mebinap] [(R)-damen], trans-RuCl₂[(S)-xyl-6mebinap][(R)-damen], and trans-RuCl₂[(R)-xyl-6mebinap][(S)-damen].

In addition, preferred examples of ruthenium/bidentate phosphine/diamine complex, include trans-PuCl₂[(S)-xylbinap] [(S)-damen], trans-RuCl₂[(R)-xylbinap][(R)-damen], trans-RuCl₂[(S)-xylbinap] [(R)-damen], trans-RuCl₂[(R)-xylbinap][(S)-damen], trans-RuCl₂[(S)-xylbinap][(S)-daipen], trans-RuCl₂[(R)-xylbinap][(R)-daipen], trans-RuCl₂[(S)-xylbinap][(R)-daipen], trans-RuCl₂[(R)-xylbinap][(S)-daipen], trans-RuCl₂[(S)-dmanylbinap][(S)-damen], trans-RuCl₂[(R)-dmanylbinap][(R)-damen], trans-RuCl₂[(S)-dmanylbinap][(R)-damen], trans-RuCl₂[(R)-dmanylbinap][(S)-damen], trans-RuCl₂[(S)-xyl-6mebinap][(S)-damen], trans-RuCl₂[(R)-xyl-6mebinap][(R)-damen], trans-RuCl₂[(S)-xyl-6mebinap][(R)-damen], and trans-RuCl₂[(R)-xyl-6mebinap][(S)-damen].

Further, in order to achieve a higher asymmetric yield of the reaction using the ruthenium/bidentate phosphine/diamine complex as a hydrogenation catalyst, it is preferred that the symbols of absolute configurations of an optically active bidentate phosphine ligand and an optically active diamine ligand are the same [for example, (R,R), [R,(R,R)], or the like]. However, a diamine ligand to be used does not necessarily contain any asymmetric center.

Among ketone compounds represented by the general formula (1), the following class of compounds may be mentioned as important intermediates for synthesizing medicaments which are described in WO 97/25311 and WO 99/01431 as being very useful for treating and preventing diabetes, obesity, hyperlipidemia and the like. That is, aminoketone compounds of the formula (1) wherein n is 0 and A is CH₂NR²R³; and wherein R¹ represents 3-aminophenyl group, 3-nitrophenyl group, 3-[(benzyl)(methylsulfonyl)amino]phenyl group, 4-chloro-3-aminophenyl group, 4-chloro-3-[(benzyl)(methylsulfonyl)amino]phenyl group, 4-chloro-3-nitrophenyl group, 4-bromo-3-aminophenyl group, 4-bromo-3-[(benzyl)(methylsulfonyl)amino]phenyl group, 4-bromo-3-nitrophenyl group, 4-benzyloxy-3-nitrophenyl group, 4-benzyloxy-3-aminophenyl group, 4-benzyloxy-3-[(benzyl)-(methylsulfonyl)amino]phenyl group, 4-benzyloxy-3-[(benzyl)(benzylsulfonyl)amino]phenyl group, or 4-benzyloxy-3-[(hydroxy)methyl]phenyl group, preferably substituted phenyl group such as 4-chloro-3-[(benzyl)(methylsulfonyl)amino]phenyl group, 4-bromo-3-[(benzyl)(methylsulfonyl)amino]phenyl group, 3-[(benzyl)(methylsulfonyl)amino]phenyl group or 4-benzyloxy-3-[(benzyl)(methylsulfonyl)amino]phenyl group; R² is an amino-protecting group which can be removed under a mild condition and specifically represents an acyl group, such as acetyl group, propionyl group, butyryl group, pentanoyl group, hexanoyl group, trifluoroacetyl group, pivaloyl group, benzoyl group, 4-methoxybenzoyl group, 2,4,6-trimethylbenzoyl group or naphthoyl group, a carbamate type protecting group, such as tert-butoxycarbonyl group, benzyloxycarbonyl group, p-methoxybenzyloxycarbonyl group or 2,2,2-trichloroethoxycarbonyl group, or an aralkyl group such as benzyl group, p-methoxybenzyl group or p-nitrobenzyl group, preferably benzoyl group or benzyl group; and R³ is an ethyl group which is bonded at its end to the tricyclic fused ring group via an oxygen atom and specifically represents 2-(dibenzofuran-3-yloxy)ethyl group, 2-(dibenzothiophen-3-yloxy)ethyl group, 2-(9H-carbazol-2-yloxy)ethyl group, 2-(1,2,3,4-tetrahydrodibenzofuran-6-yloxy)ethyl group, 2-(1,2,3,4-tetrahydrodibenzothiophen-6-yloxy)ethyl group or 2-(1,2,3,4-tetrahydro-9H-carbazol-7-yloxy)ethyl group, preferably 2-(9H-carbazol-2-yloxy)ethyl group. With respect to the abovementioned aminoketone compounds, the following ruthenium/bidentate phosphine/diamine complexes are preferably used as a hydrogenation catalyst. Examples of such a hydrogenation catalyst include trans-RuCl₂[(S)-xylbinap][(S)-damen], trans-RuCl₂[(R)-xylbinap][(R)-damen], trans-RuCl₂[(S)-xylbinap][(R)-damen], trans-RuCl₂[(R)-xylbinap][(S]-damen], trans-RuCl₂[(S)-xylbinap)[(S)-daipen], trans-RuCl₂[(R)-xylbinap][(R)-daipen], trans-RuCl₂[(S)-xylbinap][(R)-daipen], trans-RuCl₂[(R)-xylbinap][(S)-daipen], trans-RuCl₂[(S)-dmanylbinap][(S)-damen], trans-RuCl₂[(R)-dmanylbinap][(R)-damen], trans-RuCl₂[(S)-dmanylbinap][(R)-damen], trans-RuCl₂[(R)-dmanylbinap][(S)-damen], trans-RuCl₂[(S)-xyl-6mebinap][(S)-damen], trans-RuCl₂[(R)-xyl-6mebinap][(R)-damen], trans-RuCl₂[(S)-xyl-6mebinap][(R)-damen], and trans-RuCl₂[(R)-xyl-6mebinap][(S)-damen].

Particularly preferred aminoketone compounds which may be hydrogenated with the abovementioned ruthenium/bidentate phosphine/diamine complexes as a hydrogenation catalyst may be those in which R² is benzyl group. Example thereof include:
1- [4-benzyloxy-3-[(benzyl)(methylsulfonyl)amino]phenyl]-2-[[2-(dibenzothiophen-3-yloxy)ethyl]benzylamino]ethanone;
1-[4-benzyloxy-3-[(benzyl)(methylsulfonyl)amino]phenyl]-2-[[2-(dibenzofuran-3-yloxy)ethyl]benzylamino]ethanone;
1-[4-benzyloxy-3-[(benzyl)(methylsulfonyl)amino]phenyl]-2-[[2-(9H-carbazol-2-yloxy)ethyl]benzylamino]ethanone;
1-[4-benzyloxy-3-[(benzyl)(methylsulfonyl)amino]phenyl]-2-[[2-(1,2,3,4-tetrahydrodibenzothiophen-6-yloxy)ethyl] benzylamino] ethanone;
1-[4-benzyloxy-3-[(benzyl)(methylsulfonyl)amino]phenyl]-2-[[2-(1,2,3,4-tetrahydrodibenzofuran-6-yloxy)ethyl]benzylamino]-ethanone;
1-[4-benzyloxy-3-[(benzyl)(methylsulfonyl)amino]phenyl]-2-[[2-(1,2,3,4-tetrahydro-9H-carbazol-7-yloxy)ethyl]benzylamino]-ethanone;
1-[4-benzyloxy-3-[(benzyl)(benzylsulfonyl)amino]phenyl]-2-[[2-(dibenzothiophen-3-yloxy)ethyl]benzylamino]ethanone;
1-[4-benzyloxy-3-[(benzyl)(benzylsulfonyl)amino]phenyl]-2-[[2-(dibenzofuran-3-yloxy)ethyl]benzylamino]ethanone;
1-[4-benzyloxy-3-[(benzyl)(benzylsulfonyl)amino]phenyl]-2-[[2-(9H-carbazol-2-yloxy)ethyl]benzylamino]ethanone;
1-[4-benzyloxy-3-[(benzyl)(benzylsulfonyl)amino]phenyl]-2-[[2-(1,2,3,4-tetrahydrodibenzothiophen-6-yloxy)ethyl]benzyl-amino]ethanone;
1-[4-benzyloxy-3-[(benzyl)(benzylsulfonyl)amino]phenyl]-2-[[2-(1,2,3,4-tetrahydrodibenzofuran-6-yloxy)ethyl]benzylamino]-ethanone;
1-[4-benzyloxy-3-[(benzyl)(benzylsulfonyl)amino]phenyl]-2-[[2-(1,2,3,4-tetrahydro-9H-carbazol-7-yloxy)ethyl]benzylamino]-ethanone;
1-[4-chloro-3-[(benzyl)(methylsulfonyl)amino]phenyl]-2-[[2-(dibenzothiophen-3-yloxy)ethyl]benzylamino]ethanone;
1-[4-chloro-3-[(benzyl)(methylsulfonyl)amino]phenyl]-2-[[2-(dibenzofuran-3-yloxy)ethyl]benzylamino]ethanone;
1-[4-chloro-3-[(benzyl)(methylsulfonyl)amino]phenyl]-2-[[2-(9H-carbazol-2-yloxy)ethyl]benzylamino]ethanone;
1-[4-chloro-3-[(benzyl)(methylsulfonyl)amino]phenyl]-2-[[2-(1,2,3,4-tetrahydrodibenzothiophen-6-yloxy)ethyl]benzyl-amino]ethanone;
1-[4-chloro-3-[(benzyl)(methylsulfonyl)amino]phenyl]-2-[[2-(1,2,3,4-tetrahydrodibenzofuran-6-yloxy)ethyl]benzylamino]-ethanone;
1-[4-chloro-3-[(benzyl)(methylsulfonyl)amino]phenyl]-2-[[2-(1,2,3,4-tetrahydro-9H-carbazol-7-yloxy)ethyl]benzylamino]-ethanone;
1-[4-bromo-3-[(benzyl)(methylsulfonyl)amino]phenyl]-2-[[2-(dibenzothiophen-3-yloxy)ethyl]benzylamino]ethanone;
1-[4-bromo-3-[(benzyl)[methylsulfonyl)amino]phenyl]-2-[[2-(dibenzofuran-3-yloxy)ethyl]benzylamino]ethanone;
1-[4-bromo-3-[(benzyl)(methylsulfonyl)amino]phenyl]-2-[[2-(9H-carbazol-2-yloxy)ethyl]benzylamino]ethanone;
1-[4-bromo-3-[(benzyl)(methylsulfonyl)amino]phenyl]-2-[[2-(1,2,3,4-tetrahydrodibenzothiophen-6-yloxy)ethyl]benzylamino]-ethanone;
1-[4-bromo-3-[(benzyl)(methylsulfonyl)amino]phenyl]-2-[[2-(1,2,3,4-tetrahydrodibenzofuran-6-yloxy) ethyl]benzylamino]-ethanone;
1-[4-bromo-3-[(benzyl)(methylsulfonyl)amino]phenyl]-2-[[2-(1,2,3,4-tetrahydro-9H-carbazol-7-yloxy)ethyl]benzylamino]-ethanone;
1-[3-[(benzyl)(methylsulfonyl)amino]phenyl]-2-[[2-(dibenzothiophen-3-yloxy)ethyl]benzylamino]ethanone;
1-[3-[(benzyl)(methylsulfonyl)amino]phenyl]-2-[[2-(dibenzofuran-3-yloxy)ethyl]benzylamino]ethanone;
1-[3-[(benzyl)(methylsulfonyl)amino]phenyl]-2-[[2-(9H-carbazol-2-yloxy)ethyl]benzylamino]ethanone;
1-[3-[(benzyl](methylsulfonyl)amino]phenyl]-2-[[2-(1,2,3,4-tetrahydrodibenzothiophen-6-yloxy)ethyl]benzylamino]-ethanone;
1-[3-[(benzyl)(methylsulfonyl)amino]phenyl]-2-[[2-(1,2,3,4-tetrahydrodibenzofuran-6-yloxy)ethyl]benzylamino]-ethanone;
1-[3-[(benzyl)(methylsulfonyl)amino]phenyl]-2-[[2-(1,2,3,4-tetrahydro-9H-carbazol-7-yloxy)ethyl)]benzylamino]-ethanone;
1-(4-benzyloxy-3-aminophenyl)-2-[[2-(dibenzothiophen-3-yloxy)ethyl]benzylamino]ethanone;
1-(4-benzyloxy-3-aminophenyl)-2-[[2-(dibenzofuran-3-yloxy)ethyl]benzylamino]ethanone;
1-(4-benzyloxy-3-aminophenyl)-2-[[2-(9H-carbazol-2-yloxy)-ethyl]benzylamino]ethanone;
1-(4-benzyloxy-3-aminophenyl)-2-[[2-(1,2,3,4-tetrahydrodibenzothiophen-6-yloxy)ethyl]benzylamino]ethanone;
1-(4-benzyloxy-3-aminophenyl)-2-[[2-(1,2,3,4-tetrahydrodibenzofuran-6-yloxy)ethyl]benzylamino]ethanone;
1-(4-benzyloxy-3-aminophenyl)-2-[[2-(1,2,3,4-tetrahydro-9H-carbazol-7-yloxy]ethyl]benzylamino]ethanone;
1-(4-chloro-3-aminophenyl)-2-[[2-(dibenzothiophen-3-yloxy)ethyl]benzylamino]ethanone;
1-(4-chloro-3-aminophenyl)-2-[[2-(dibenzofuran-3-yloxy)-ethyl]benzylamino]ethanone;
1-(4-chloro-3-aminophenyl)-2-[[2-(9H-carbazol-2-yloxy)-ethyl]benzylamino]ethanone;
1-(4-chloro-3-aminophenyl)-2-[[2-(1,2,3,4-tetrahydrodibenzothiophen-6-yloxy)ethyl]benzylamino]ethanone;
1-(4-chloro-3-aminophenyl)-2-[[2-(1,2,3,4-tetrahydrodibenzofuran-6-yloxy)ethyl]benzylamino]ethanone;
1-(4-chloro-3-aminophenyl)-2-[[2-(1,2,3,4-tetrahydro-9H-carbazol-7-yloxy)ethyl]benzylamino]ethanone;
1-(4-bromo-3-aminophenyl)-2-[[2-(dibenzothiophen-3-yloxy)-ethyl]benzylamino]ethanone;
1-(4-bromo-3-aminophenyl)-2-[[2-(dibenzofuran-3-yloxy)-ethyl]benzylamino]ethanone;
1-(4-bromo-3-aminophenyl)-2-[[2-(9H-carbazol-2-yloxy)-ethyl]benzylamino]ethanone;
1-(4-bromo-3-aminophenyl)-2-[[2-(1,2,3,4-tetrahydrodibenzothiophen-6-yloxy)ethyl]benzylamino]ethanone;
1-(4-bromo-3-aminophenyl)-2-[[2-(1,2,3,4-tetrahydrodibenzofuran-6-yloxy)ethyl]benzylamino]ethanone;
1-(4-bromo-3-aminophenyl)-2-[[2-(1,2,3,4-tetrahydro-9H-carbazol-7-yloxy)ethyl]benzylamino]ethanone;
1-(3-aminophenyl)-2-[[2-(dibenzothiophen-3-yloxy)ethyl]-benzylamino]ethanone;
1-(3-aminophenyl)-2-[[2-(dibenzofuran-3-yloxy)ethyl]-benzylamino]ethanone;
1-(3-aminophenyl)-2-[[2-(9H-carbazol-2-yloxy)ethyl]-benzylamino]ethanone;
1-(3-aminophenyl)-2-[[2-(1,2,3,4-tetrahydrodibenzothiophen-6-yloxy)ethyl]benzylamino]ethanone;
1-(3-aminophenyl)-2-[[2-(1,2,3,4-tetrahydrodibenzofuran-6-yloxy)ethyl]benzylamino]ethanone;
1-(3-aminophenyl)-2-[[2-(1,2,3,4-tetrahydro-9H-carbazol-7-yloxy)ethyl]benzylamino]ethanone;
1-(4-benzyloxy-3-nitrophenyl)-2-[[2-(dibenzothiophen-3-yloxy)ethyl]benzylamino]ethanone;
1-(4-benzyloxy-3-nitrophenyl)-2-[[2-(dibenzofuran-3-yloxy)ethyl]benzylamino]ethanone;
1-(4-benzyloxy-3-nitrophenyl)-2-[[2-(9H-carbazol-2-yloxy)ethyl]benzylamino]ethanone;
1-(4-benzyloxy-3-nitrophenyl)-2-[[2-(1,2,3,4-tetrahydro-dibenzothiophen-6-yloxy)ethyl]benzylamino]ethanone;
1-(4-benzyloxy-3-nitrophenyl)-2-[[2-(1,2,3,4-tetrahydro-dibenzofuran-6-yloxy)ethyl]benzylamino]ethanone;
1-(4-benzyloxy-3-nitrophenyl)-2-[[2-(1,2,3,4-tetrahydro-9H-carbazol-7-yloxy)ethyl]benzylamino]ethanone;
1-(4-chloro-3-nitrophenyl)-2-[[2-(dibenzothiophen-3-yloxy)ethyl]benzylamino]ethanone;
1-(4-chloro-3-nitrophenyl)-2-[[2-(dibenzofuran-3-yloxy)ethyl]benzylamino]ethanone;
1-(4-chloro-3-nitrophenyl)-2-[[2-(9H-carbazol-2-yloxy)ethyl]-benzylamino]ethanone;
1-(4-chloro-3-nitrophenyl)-2-[[2-(1,2,3,4-tetrahydrodibenzothiophen-6-yloxy)ethyl]benzylamino]ethanone;
1-(4-chloro-3-nitrophenyl)-2-[[2-(1,2,3,4-tetrahydrodibenzofuran-6-yloxy)ethyl]benzylamino]ethanone;
1-(4-chloro-3-nitrophenyl)-2-[[2-(1,2,3,4-tetrahydro-9H-carbazol-7-yloxy)ethyl]benzylamino]ethanone;
1-(4-bromo-3-nitrophenyl)-2-[[2-(dibenzothiophen-3-yloxy)-ethyl]benzylamino]ethanone;
1-(4-bromo-3-nitrophenyl)-2-[[2-(dibenzofuran-3-yloxy)-ethyl]benzylamino]ethanone;
1-(4-bromo-3-nitrophenyl)-2-[[2-(9H-carbazol-2-yloxy)-ethyl]benzylamino]ethanone;
1-(4-bromo-3-nitrophenyl)-2-[[2-(1,2,3,4-tetrahydrodibenzothiophen-6-yloxy)ethyl]benzylamino]ethanone;
1-(4-bromo-3-nitrophenyl)-2-[[2-(1,2,3,4-tetrahydrodibenzofuran-6-yloxy)ethyl]benzylamino]ethanone;
1-(4-bromo-3-nitrophenyl)-2-[[2-(1,2,3,4-tetrahydro-9H-carbazol-7-yloxy)ethyl]benzylamino]ethanone;
1-(3-nitrophenyl)-2-[[2-(dibenzothiophen-3-yloxy]ethyl]-benzylamino]ethanone;
1-(3-nitrophenyl)-2-[[2-(dibenzofuran-3-yloxy)ethyl]-benzylamino]ethanone;
1-(3-nitrophenyl)-2-[[2-(9H-carbazol-2-yloxy)ethyl]-benzylamino]ethanone;
1-(3-nitrophenyl)-2-[[2-(1,2,3,4-tetrahydrodibenzothiophen-6-yloxy)ethyl]benzylamino]ethanone;
1-(3-nitrophenyl)-2-[[2-(1,2,3,4-tetrahydrodibenzofuran-6-yloxy)ethyl]benzylamino]ethanone; and
1-(3-nitrophenyl)-2-[[2-(1,2,3,4-tetrahydro-9H-carbazol-7-yloxy)ethyl]benzylamino]ethanone;
and their salts with acceptable acids, such as hydrochloride, acetate, hydrobromide and trifluoroacetate.

Specific examples of the compounds having a benzoyl group as R² include:
1-[4-benzyloxy-3-[(benzyl)(methylsulfonyl)amino]phenyl]-2-[[2-(dibenzothiophen-3-yloxy)ethyl]benzoylamino]ethanone;
1-[4-benzyloxy-3-[(benzyl)(methylsulfonyl)amino]phenyl]-2-[[2-(dibenzofuran-3-yloxy)ethyl]benzoylamino]ethanone;
1-[4-benzyloxy-3-[(benzyl)(methylsulfonyl)amino]phenyl]-2-[[2-(9H-carbazol-2-yloxy)ethyl]benzoylamino]ethanone;
1-[4-benzyloxy-3-[(benzyl)(methylsulfonyl)amino]phenyl]-2-[[2-(1,2,3,4-tetrahydrodibenzothiophen-6-yloxy)ethyl]benzoylamino]ethanone;
1-[4-benzyloxy-3-[(benzyl)(methylsulfonyl)amino]phenyl]-2-[[2-(1,2,3,4-tetrahydrodibenzofuran-6-yloxy)ethyl]benzoylamino]ethanone;
1-[4-benzyloxy-3-[(benzyl)(methylsulfonyl)amino]phenyl]-2-[[2-(1,2,3,4-tetrahydro-9H-carbazol-7-yloxy)ethyl]benzoylamino]ethanone;
1-[4-benzyloxy-3-[(benzyl)(benzylsulfonyl)amino]phenyl]-2-[[2-(dibenzothiophen-3-yloxy)ethyl]benzoylamino]ethanone;
1-[4-benzyloxy-3-[(benzyl)(benzylsulfonyl)amino]phenyl]-2-[[2-(dibenzofuran-3-yloxy)ethyl]benzoylamino]ethanone;
1-[4-benzyloxy-3-[(benzyl)(benzylsulfonyl)amino]phenyl]-2-[[2-(9H-carbazol-2-yloxy)ethyl]benzoylamino]ethanone;
1-[4-benzyloxy-3-[(benzyl)(benzylsulfonyl)amino]phenyl]-2-[[2-(1,2,3,4-tetrahydrodibenzothiophen-6-yloxy)ethyl]benzoylamino]ethanone;
1-[4-benzyloxy-3-[(benzyl)(benzylsulfonyl)amino]phenyl]-2-[[2-(1,2,3,4-tetrahydrodibenzofuran-6-yloxy)ethyl]benzoyl-amino]ethanone;
1-[4-benzyloxy-3-[(benzyl)(benzylsulfonyl)amino]phenyl]-2-[[2-(1,2,3,4-tetrahydro-9H-carbazol-7-yloxy)ethyl]benzoylamino]ethanone;
1-[4-chloro-3-[(benzyl)(methylsulfonyl)amino]phenyl]-2-[[2-(dibenzothiophen-3-yloxy)ethyl]benzoylamino]ethanone;
1-[4-chloro-3-[(benzyl)(methylsulfonyl)amino]phenyl]-2-[[2-(dibenzofuran-3-yloxy)ethyl]benzoylamino]ethanone;
1-[4-chloro-3-[(benzyl)(methylsulfonyl)amino]phenyl]-2-[[2-(9H-carbazol-2-yloxy)ethyl]benzoylamino]ethanone;
1-[4-chloro-3-[(benzyl)(methylsulfonyl)amino]phenyl]-2-[[2-(1,2,3,4-tetrahydrodibenzothiophen-6-yloxy)ethyl]benzoylamino]ethanone;
1-[4-chloro-3-[(benzyl)(methylsulfonyl)amino]phenyl]-2-[[2-(1,2,3,4-tetrahydrodibenzofuran-6-yloxy)ethyl]benzoylamino]ethanone;
1-[4-chloro-3-[(benzyl)(methylsulfonyl)amino]phenyl]-2-[[2-(1,2,3,4-tetrahydro-9H-carbazol-7-yloxy)ethyl]benzoylamino]ethanone;
1-[4-bromo-3-[(benzyl)(methylsulfonyl)amino]phenyl]-2-[[2-(dibenzothiophen-3-yloxy)ethyl]benzoylamino]ethanone;
1-[4-bromo-3-[(benzyl)(methylsulfonyl)amino]phenyl]-2-[[2-(dibenzofuran-3-yloxy)ethyl]benzoylamino]ethanone;
1-[4-bromo-3-[(benzyl)(methylsulfonyl)amino]phenyl]-2-[[2-(9H-carbazol-2-yloxy)ethyl]benzoylamino]ethanone;
1-[4-bromo-3-[(benzyl)(methylsulfonyl)amino]phenyl]-2-[[2-(1,2,3,4-tetrahydrodibenzothiophen-6-yloxy)ethyl]benzoylamino]ethanone;
1-[4-bromo-3-[(benzyl)(methylsulfonyl)amino]phenyl]-2-[[2-(1,2,3,4-tetrahydrodibenzofuran-6-yloxy)ethyl]benzoylamino]ethanone;
1-[4-bromo-3-[(benzyl)(methylsulfonyl)amino]phenyl]-2-[[2-(1,2,3,4-tetrahydro-9H-carbazol-7-yloxy)ethyl]benzoylamino]ethanone;
1-[3-[(benzyl)(methylsulfonyl)amino]phenyl]-2-[[2-(dibenzothiophen-3-yloxy)ethyl]benzoylamino]ethanone;
1-[3-[(benzyl)(methylsulfonyl)amino]phenyl]-2-[[2-(dibenzofuran-3-yloxy)ethyl]benzoylamino]ethanone;
1-[3-[(benzyl)(methylsulfonyl)amino]phenyl]-2-[[2-(9H-carbazol-2-yloxy)ethyl]benzoylamino]ethanone;
1-[3-[(benzyl)(methylsulfonyl)amino]phenyl]-2-[[2-(1,2,3,4-tetrahydrodibenzothiophen-6-yloxy)ethyl]benzoylamino]-ethanone;
1-[3-[(benzyl)(methylsulfonyl)amino]phenyl]-2-[[2-(1,2,3,4-tetrahydrodibenzofuran-6-yloxy)ethyl]benzoylamino]ethanone;
1-[3-[(benzyl)(methylsulfonyl)amino]phenyl]-2-[[2-(1,2,3,4-tetrahydro-9H-carbazol-7-yloxy)ethyl]benzoylamino]ethanone;
1- (4-benzyloxy-3-aminophenyl)-2-[[2-(dibenzothiophen-3-yloxy)ethyl]benzoylamino]ethanone;
1-(4-benzyloxy-3-aminophenyl)-2-[[2-(dibenzofuran-3-yloxy)ethyl]benzoylamino]ethanone;
1-(4-benzyloxy-3-aminophenyl)-2-[[2-(9H-carbazol-2-yloxy)-ethyl]benzoylamino]ethanone;
1-(4-benzyloxy-3-aminophenyl)-2-[[2-(1,2,3,4-tetrahydrodibenzothiophen-6-yloxy)ethyl]benzoylamino]ethanone;
1-(4-benzyloxy-3-aminophenyl)-2-[[2-(1,2,3,4-tetrahydrodibenzofuran-6-yloxy)ethyl]benzoylamino]ethanone;
1-(4-benzyloxy-3-aminophenyl)-2-[[2-(1,2,3,4-tetrahydro-9H-carbazol-7-yloxy)ethyl]benzoylamino]ethanone;
1-(4-chloro-3-aminophenyl)-2-[[2-(dibenzothiophen-3-yloxy)ethyl]benzoylamino]ethanone;
1-(4-chloro-3-aminophenyl)-2-[[2-(dibenzofuran-3-yloxy)-ethyl]benzoylamino]ethanone;
1-(4-chloro-3-aminophenyl)-2-[[2-(9H-carbazol-2-yloxy)-ethyl]benzoylamino]ethanone;
1-(4-chloro-3-aminophenyl)-2-[[2-(1,2,3,4-tetrahydrodibenzothiophen-6-yloxy)ethyl]benzoylamino]ethanone;
1-(4-chloro-3-aminophenyl)-2-[[2-(1,2,3,4-tetrahydrodibenzofuran-6-yloxy)ethyl]benzoylamino]ethanone;
1-(4-chloro-3-aminophenyl)-2-[[2-(1,2,3,4-tetrahydro-9H-carbazol-7-yloxy)ethyl]benzoylamino]ethanone;
1-(4-bromo-3-aminophenyl)-2-[[2-(dibenzothiophen-3-yloxy)-ethyl]benzoylamino]ethanone,
1-(4-bromo-3-aminophenyl)-2-[[2-(dibenzofuran-3-yloxy)-ethyl]benzoylamino]ethanone;
1-(4-bromo-3-aminophenyl)-2-[[2-(9H-carbazol-2-yloxy)-ethyl]benzoylamino]ethanone;
1-(4-bromo-3-aminophenyl)-2-[[2-(1,2,3,4-tetrahydrodibenzothiophen-6-yloxy)ethyl]benzoylamino]ethanone;
1-(4-bromo-3-aminophenyl)-2-[[2-(1,2,3,4-tetrahydrodibenzofuran-6-yloxy)ethyl]benzoylamino]ethanone;
1-(4-bromo-3-aminophenyl)-2-[[2-(1,2,3,4-tetrahydro-9H-carbazol-7-yloxy)ethyl3benzoylamino]ethanone;
1-(3-aminophenyl)-2-[[2-(dibenzothiophen-3-yloxy]ethyl]-benzoylamino]ethanone;
1-(3-aminophenyl)-2-[[2-(dibenzofuran-3-yloxy)ethyl]-benzoylamino]ethanone;
1-(3-aminophenyl)-2-[[2-(9H-carbazol-2-yloxy)ethyl]-benzoylamino]ethanone;
1-(3-aminophenyl)-2-[[2-(1,2,3,4-tetrahydrodibenzothiophen-6-yloxy)ethyl]benzoylamino] ethanone;
1-(3-aminophenyl)-2-[[2-(1,2,3,4-tetrahydrodibenzofuran-6-yloxy)ethyl]benzoylamino]ethanone;
1-(3-aminophenyl)-2-[[2-(1,2,3,4-tetrahydro-9H-carbazol-7-yloxy)ethyl]benzoylamino]ethanone;
1-(4-benzyloxy-3-nitrophenyl)-2-[[2-(dibenzothiophen-3-yloxy) ethyl]benzoylamino]ethanone;
1-(4-benzyloxy-3-nitrophenyl)-2-[[2-(dibenzofuran-3-yloxy)ethyl]benzoylamino]ethanone;
1-(4-benzyloxy-3-nitrophenyl)-2-[[2-(9H-carbazol-2-yloxy)-ethyl]benzoylamino]ethanone;
1-(4-benzyloxy-3-nitrophenyl)-2-[[2-(1,2,3,4-tetrahydrodibenzothiophen-6-yloxy)ethyl]benzoylamino]ethanone;
1-(4-benzyloxy-3-nitrophenyl)-2-[[2-(1,2,3,4-tetrahydrodibenzofuran-6-yloxy)ethyl]benzoylamino]ethanone;
1-4-benzyloxy-3-nitrophenyl)-2-[[2-(1,2,3,4-tetrahydro-9H-carbazol-7-yloxy)ethyl]benzoylamino]ethanone;
1-(4-chloro-3-nitrophenyl)-2-[[2-(dibenzothiophen-3-yloxy)ethyl]benzoylamino]ethanone;
1-(4-chloro-3-nitrophenyl)-2-[[2-(dibenzofuran-3-yloxy)ethyl]benzoylamino]ethanone;
1-(4-chloro-3-nitrophenyl)-2-[[2-(9H-carbazol-2-yloxy)ethyl]benzoylamino]ethanone;
1-(4-chloro-3-nitrophenyl)-2-[[2-(1,2,3,4-tetrahydrodibenzothiophen-6-yloxy)ethyl]benzoylamino]ethanone;
1-(4-chloro-3-nitrophenyl)-2-[[2-(1,2,3,4-tetrahydrodibenzofuran-6-yloxy)ethyl]benzoylamino]ethanone;
1-(4-chloro-3-nitrophenyl)-2-[[2-(1,2,3,4-tetrahydro-9H-carbazol-7-yloxy)ethyl]benzoylamino]ethanone;
1-(4-bromo-3-nitrophenyl)-2-[[2-(dibenzothiophen-3-yloxy)-ethyl]benzoylamino]ethanone;
1-(4-bromo-3-nitrophenyl)-2-[[2-(dibenzofuran-3-yloxy)-ethyl]benzoylamino]ethanone;
1-(4-bromo-3-nitrophenyl)-2-[[2-(9H-carbazol-2-yloxy)-ethyl]benzoylamino]ethanone;
1-(4-bromo-3-nitrophenyl)-2-[[2-(1,2,3,4-tetrahydrodibenzothiophen-6-yloxy)ethyl]benzoylamino]ethanone;
1-(4-bromo-3-nitrophenyl)-2-[[2-(1,2,3,4-tetrahydrodibenzofuran-6-yloxy)ethyl]benzoylamino]ethanone;
1-(4-bromo-3-nitrophenyl)-2-[[2-(1,2,3,4-tetrahydro-9H-carbazol-7-yloxy)ethyl]benzoylamino]ethanone;
1-(3-nitrophenyl)-2-[[2-(dibenzothiophen-3-yloxy)ethyl]-benzoylamino]ethanone;
1-(3-nitrophenyl)-2-[[2-(dibenzofuran-3-yloxy)ethyl]-benzoylamino]ethanone;
1-(3-nitrophenyl)-2-[[2-(9H-carbazol-2-yloxy)ethyl]-benzoylamino]ethanone;
1-(3-nitrophenyl)-2-[[2-(1,2,3,4-tetrahydrodibenzothiophen-6-yloxy)ethyl]benzoylamino]ethanone;
1-(3-nitrophenyl)-2-[[2-(1,2,3,4-tetrahydrodibenzofuran-6-yloxy)ethyl]benzoylamino]ethanone; and
1-(3-nitrophenyl)-2-[[2-(1,2,3,4-tetrahydro-9H-carbazol-7-yloxy)ethyl]benzoylamino]ethanone.

The amount of a ruthenium/bidentate phosphine/diamine complex represented by the general formula (2) to be used as an asymmetric hydrogenation catalyst or an asymmetric hydrogenation catalyst precursor can vary depending on the type of a reactor used and/or the cost, and may range from 1/1,000 to 1/3,000,000 mol for 1 mol of the ketone compound which is a reaction substrate. More preferred practical amount may range from 1/10,000 to 1/3,000,000 mol for 1 mol of the ketone compound.

When X and Y of the ruthenium complex each are hydrogen, the ketone compound may be hydrogenated by mixing the ketone compound with the ruthenium complex without adding a base; and stirring the resulting mixture under a pressurized hydrogen atmosphere. When the ketone compound is used in a large excess amount with respect to the catalyst, adding a base may be preferred. On the other hand, when X and Y each are a group other than hydrogen, the ketone compound may be more effectively hydrogenated by previously converting the ruthenium complex into its activated hydride form by stirring the ruthenium complex in 2-propanol in the presence of a base such as potassium tert-butoxide at 60°C for 30 minutes; and then mixing the activated hydride form with the ketone compound followed by pressurizing with hydrogen. The amount of the base to be added ranges from 0.5 to 100 equivalents, preferably from 2 to 40 equivalents for the diamine/bidentate phosphine/ruthenium complex, when the substrate, ketone compound is a neutral or basic compound. Further, when the substrate, ketone compound is in the form of a salt with an acidic material such as mineral or organic acid, or has a very weak acidic functional group in the molecule, the base is preferably added in a total amount of a sufficient amount for neutralizing the salt or acidic group and additionally an amount of from 0.5 to 100 equivalents, preferably from 2 to 40 equivalents with respect to the diamine/bidentate phosphine/ruthenium complex. In particular, when an α-aminoketone compound is selected as the reaction substrate, it is preferably subjected to the process in the form of hydrochloride salt in view of the stability of the substrate per se. Using the base in an amount of from 1.01 to 1.2 mol for 1 mol of the α-aminoketone derivative monohydrochloride may be mentioned as the most preferred example.

Examples of the base include alkali metal salts and alkaline earth metal salts, such as KOH, K₂CO₃, KOCH₃, KOCH(CH₃)₂, KOC(CH₃)₃, KC₁₀H₈, NaOH, Na₂CO₃, NaOCH₃, NaOCH(CH₃)₂, NaOC(CH₃)₃, LiOH, LiOCH₃, LiOCH(CH₃)₂ and LiO(CH₃)₃, with K₂CO₃, KOCH(CH₃)₂, KOC(CH₃)₃, Na₂CO₃, NaOCH(CH₃)₂ and NaOC(CH₃)₃ being preferred. Any material other than the base may be used as long as the material can afford the diamine/bidentate phosphine ruthenium hydride form. Examples of the material include hydrogen, a metal hydride (such as sodium borohydride or lithium aluminium hydride) and an organometallic compound (such as methylmagnesium bromide, ethylmagnesium bromide, propylmagnesium bromide, methyllithium, ethyllithium or propyllithium).

Any solvent may be used as long as it can solubilize the raw materials of the reaction and the catalyst system. However, the solvent is not necessarily required to completely dissolve the raw materials. For example, aromatic hydrocarbon solvent (such as toluene or xylene), aliphatic hydrocarbon solvent (such as pentane or hexane), halogen-containing hydrocarbon solvent (such as dichloromethane), ether type solvent (such as diethyl ether or tetrahydrofuran), alcohol type solvent (such as methanol, ethanol, 2-propanol, butanol, or tert-butanol), or heteroatom-containing organic solvent (such as acetonitrile, DMF, DMA, NMP, or DMSO) may be used. Alcohol type solvents may be preferably used, since the reaction products to be obtained are alcohol compounds. Most preferably, 2-propanol or tert-butanol may be used. When the reaction substrate is difficult to be dissolved in the solvent, a mixed solvent comprising solvents suitably selected from a variety of solvents mentioned above may be used. Preferred examples of the mixed solvent system include 2-propanol/DMF (from 4/1 to 1/4), 2-propanol/DMA(from 4/1 to 1/4), tert-butanol/DMF(from 4/1 to 1/4), and tert-butanol/DMA (from 4/1 to 1/4). The amount of the solvent is selected depending on the solubility of the reaction substrate and the cost. For example, when 2-propanol is used, a certain substrate may be subjected to the reaction at from a low concentration of 1% or less to a high concentration which is close to a solvent-free reaction condition. The concentration of the substrate ranges preferably from 0.1 to 1.0 mol/L. When α-aminoketone compound is used as the substrate, the concentration thereof is more preferably 0.1 mol/L.

The present catalyst system has a very high activity. Therefore, the process of the present invention can be carried out under a hydrogen atmosphere at 1 atm. However, the process may be carried out at a pressure of from 1 to 200 atm, preferably from 1 to 100 atm. More preferably, the process may be carried out at a lower pressure of from 3 to 10 atm from the viewpoint of a pressure-proof design of the reactor. However, pressures ranging from 3 to 50 atm are also preferred in view of the cost of the whole process. Further, when the process is carried out on a small scale, it can be well carried out at a pressure below 10 atm. The process is preferably carried out at temperatures ranging from 15°C to 100°C in view of the cost. In view of the stability of the catalyst and substrate, however, the process is more preferably carried out at temperatures ranging from 15°C to 80°C, particularly at temperatures ranging from 25°C to 40°C close to room temperature. The present process may be characterized by the fact that the process can proceed at temperatures as low as from -30°C to 0°C and also characterized by the fact that the yield and selectivity are hard to be affected by the reaction temperature. Although the reaction time may be vary depending on the reaction conditions including amount of the catalyst to be used, concentration of the reaction substrate, temperature and pressure, the reaction can be completed for from several minutes to several days. For example, the present process may be also characterized by the fact that when the catalyst is used in an amount of 1/2,000 mol for 1 mol of the substrate, the reaction can be generally completed for from several minutes to 1 hour. These features will be specifically illustrated in Examples. Further, the ketone compound-hydrogenating reaction of the present invention may be carried out by batch system or continuous system.

The nitrogenous or oxygenic functional group-containing optically active secondary alcohols which can be obtained according to the present invention have a high optical purity. Conventional reactions for removing the protecting groups such as acyl group, benzyl group and ketal group and for transforming the functional groups can convert the abovementioned secondary alcohols into the corresponding amino group-containing optically active secondary alcohols which may be important as a medicament or pesticide per se or as an intermediate for preparing a medicament or pesticide. The conversion may be carried out by a combined process comprising conventional known reactions set forth below.

That is, when A of the general formula (3) is CH₂NR²R³, as indicated in the reaction scheme (15): 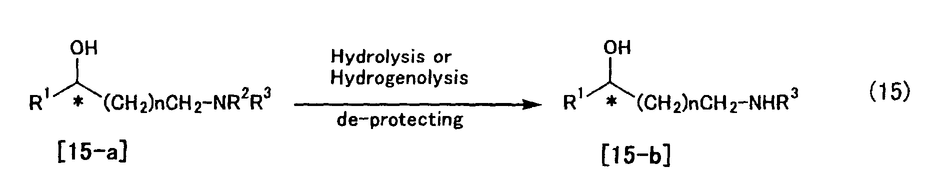 wherein R¹, n, R² and R³ are as defined above, provided that R² and R³ are not bonded to each other; and * represents an asymmetric carbon atom, an optically active secondary alcohol [15-b] may be synthesized by subjecting an optically active secondary alcohol having a nitrogenous or oxygenic functional group [15-a] to a deprotecting reaction to remove a protecting group R². Examples of the protecting group R² include acyl group, alkyloxycarbonyl group and optionally substituted benzyl group. For example, the conventional deprotecting method described in the literature accepted in the art (Greene, T. W., et al., Protective Groups in Organic Synthesis, (Wiley-Interscience Publication)) maybe used. In particular, benzyl group and benzoyl group are specifically mentioned as the protecting group R² of the α-aminoketone compounds included in [15-a] which are described in the above as important intermediates for synthesizing α-aminoalcohols disclosed in WO 97/25311 and WO 99/01431 as being very useful for treating and preventing diabetes, obesity, hyperlipidemia and the like. The benzoyl group may be removed, for example, by a hydrolysis reaction with a base, such as sodium carbonate, potassium carbonate, sodium hydroxide or potassium hydroxide.

The amount of the base to be added may be generally from about 0.1 to about 10 mol for 1 mol of the benzoylamide. Generally, the reaction is preferably carried out in methanol, ethanol, tetrahydrofuran or 1,4-dioxane, or a water-mixed solvent thereof. The amount of such solvent to be used may be generally from about 1 to about 5 mL for 1 g of the benzoylamide. Generally, the reaction is preferably carried out at temperatures ranging from 20°C to about 100°C, particularly, from about 50°C to about 100°C, for example, for from 1 to 24 hours. The benzyl group may be removed by a hydrogenolysis reaction with a catalyst such as palladium/carbon or palladium hydroxide/carbon. The amount of the catalyst to be used may be generally from about 5% to about 20% by weight with respect to the protected amine. Generally, the reaction is preferably carried out in a solvent such as methanol, ethanol, tetrahydrofuran or acetic acid. The amount of the solvent to be used may be from about 1 to about 50 mL for 1 g of the protected amine. Generally, the reaction may be preferably carried out at temperatures ranging from -10°C to 50°C, particularly at room temperature, for example, for from 3 to 10 hours. When the group R¹ contains a halogen, the deprotecting process is carried out according to the process described in the literatures (M. Koreeda, et al., J. O. C., 49, p2081 (1984) and S. Gubert, et al., Synthesis, 4, p318 (1991)).

The tert-butoxycarbonyl(Boc) group may be removed by reacting the corresponding protected amine compound with a known mineral acid or Lewis acid. Preferred examples of the known mineral acid and Lewis acid include hydrochloric acid, hydrobromic acid, sulfuric acid, acetic acid, trifluoroacetic acid, aluminium chloride, bromotrimethylsilane and iodotrimethylsilane, with hydrochloric acid being preferred. The amount of the mineral acid or Lewis acid to be added can generally vary from about the same molar amount to a solvent amount with respect to the protected amine. This reaction can be carried out in a solvent. However, this reaction may also be preferably carried out using the above acid as a solvent. Examples of the solvent include a lower alcohol such as methanol, ethanol or n-propanol, 1,4-dioxane, tetrahydrofuran, acetonitrile and dichloromethane, with methanol and ethanol being preferred. Generally, this reaction is preferably carried out at temperatures ranging from about -30°C to about 100°C, particularly from about 0°C to about 30°C, for example, for 1 to 10 hours. The benzyloxycarbonyl group (Cbz group) may be removed by the same method with that for removing the benzyl group.

The amino-protecting groups may be sequentially or simultaneously removed. For example, when R² is a benzyloxycarbonyl or benzyl group and R¹ is a benzyl-containing group, they can be removed under the same reaction condition and are preferably simultaneously removed. When R² is a tert-butoxycarbonyl or benzoyl group and R¹ is a benzyl-containing group, they are removed by sequential steps comprising, for example, the first removal of the tert-butoxycarbonyl or benzoyl group as R² followed by the removal of the benzyl group in R¹. The order of the removals is not limited to the above and is preferably selected depending on the physical properties and the like of the compound to be deprotected. The condition for removing each protecting group is as set forth above. In addition, these deprotections can be carried out with reference to the teachings of WO 97/25311.

Further, when A of the general formula (3) is CH₂OSiR⁵R⁶R⁷, as indicated in the reaction scheme (16): 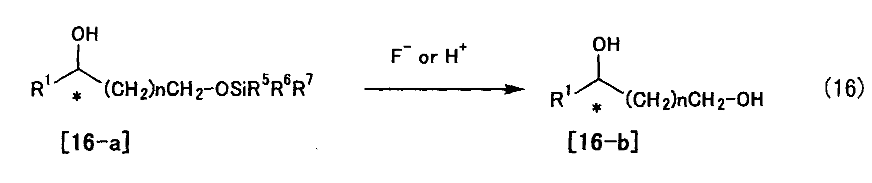 wherein R¹, R⁵, R⁶, R⁷ and n are as defined above, and * represents an asymmetric carbon atom, an optically active diol [16-b] may be synthesized by subjecting an optically active secondary alcohol having an oxygenic functional group [16-a] to a deprotecting reaction to remove the silyl group. For example, the silyl group may be removed by the conventional process, for example, described in the literature accepted in the art (Greene, T. W., et al., Protective Groups in Organic Synthesis (Wiley-Interscience Publication)). Specifically, the process may be a process comprising selectively removing the silyl group using a fluoride anion-containing reagent, such as tetrabutylammonium fluoride.

Further, when A of the general formula (3) is CH(OP¹⁵)₂**,** as indicated in the reaction scheme (17): 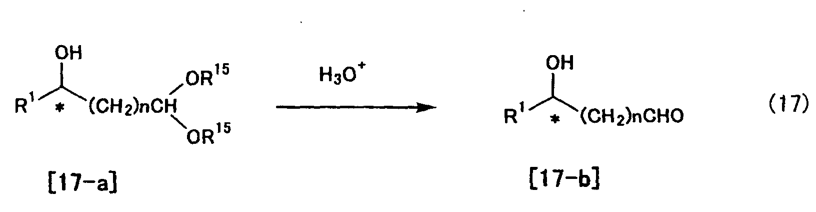 wherein R¹, n and R¹⁵ are as defined above, and * represents an asymmetric carbon atom, an aldehyde group-containing optically active secondary alcohol [17-b] may be synthesized by subjecting a ketal group-containing optically active secondary alcohol [17-a] to a process for deprotecting such a ketal group. The ketal group may be removed by the conventional method, for example, described in the literature accepted in the art (Greene, T. W., et al., Protective Groups in Organic Synthesis (Wiley-Interscience Publication)).

Further, as indicated in the reaction scheme (18): 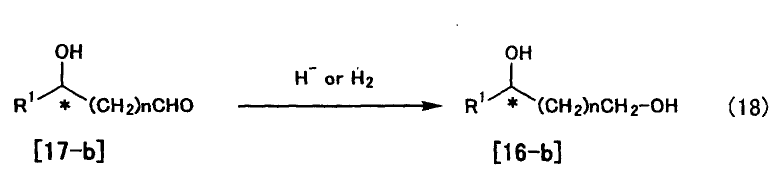 wherein R¹ and n are as defined above, and * represents an asymmetric carbon atom, an aldehyde form [17-b] may be converted into an optically active diol [16-b] by a conventional aldehyde group-reducing reaction. The aldehyde group-reducing reaction is not limited as long as it does not affect the stereochemistry of the optically active secondary hydroxyl group. Examples thereof include a process using a hydride-reducing agent, such as sodium borohydride or lithium aluminium hydride; a catalytic hydrogenation reduction process using Pd/carbon as a catalyst; and a process using the catalyst of the present invention.

Further, as indicated in the reaction scheme (19): 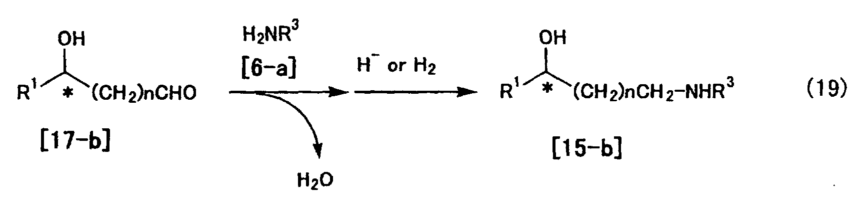 wherein R¹ and n are as defined above, and * represents an asymmetric carbon atom, an amino group-containing optically active secondary alcohol [15-b] may be synthesized by subjecting an aldehyde form [17-b] to an more abbreviated process which comprises preparing an imine compound and a primary amine compound [6-a] represented by H₂NR³ wherein R³ is as defined above, by a thermal dehydration treatment in a solvent such as methanol, ethanol or toluene, or by using various dehydrating agents, followed by a conventional imine-reducing reaction.

Further, as indicated in the reaction scheme (20): 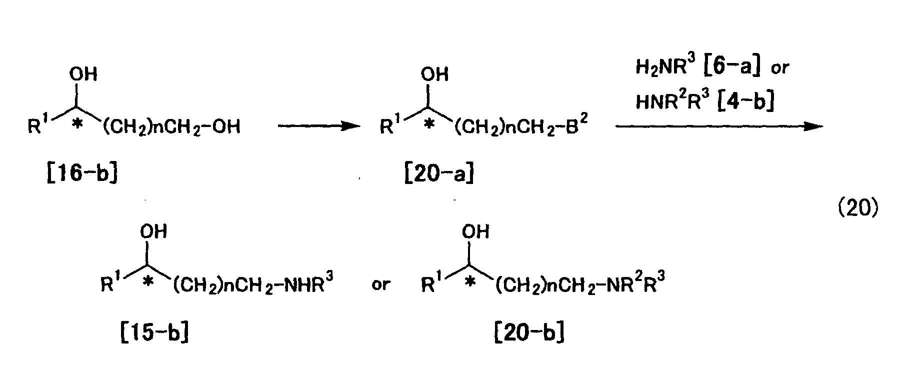 wherein R¹ and n are as defined above, B² represents a leaving group, and * represents an asymmetric carbon atom, an amino group-containing optically active secondary alcohols [15-b] or [20-b] (wherein R¹, R², R³ and n are as defined above, and * represents an asymmetric carbon atom) may be prepared from an optically active diol [16-b] by subjecting the terminal primary hydroxyl group to a conventional reaction for converting such a primary hydroxyl group into a leaving group B², and subjecting the leaving group B² to a conventional S_{N}2 type substitution reaction with a primary amine compound [6-a] or with a secondary amine compound [4-b] (wherein R² and R³ are as defined above, provided that R² is other than acyl group and alkyloxycarbonyl group). The reaction for converting the primary hydroxyl group into the leaving group B² may be a reaction comprising converting the primary hydroxyl group into the corresponding sulfonate by a reaction with methanesulfonyl chloride or p-toluenesulfonyl chloride in an amount of from 1.0 to 1.1 mol for 1 mol of the hydroxyl group in the presence of a base catalyst such as pyridine, NaHCO₃ or K₂CO₃ catalyst. Further, this sulfonate may be replaced with halogen by a reaction with NaBr, NaI or the like. Further, the primary hydroxyl group may be directly converted into halogen by a reaction with from 1.0 to 1.1 mol of carbon tetrabromide, N-bromosuccinimide or the like in the presence of 1.5 mol of triphenylphosphine for 1 mol of the primary hydroxyl group under a mild neutral condition.

Further, as indicated in the reaction scheme (21): 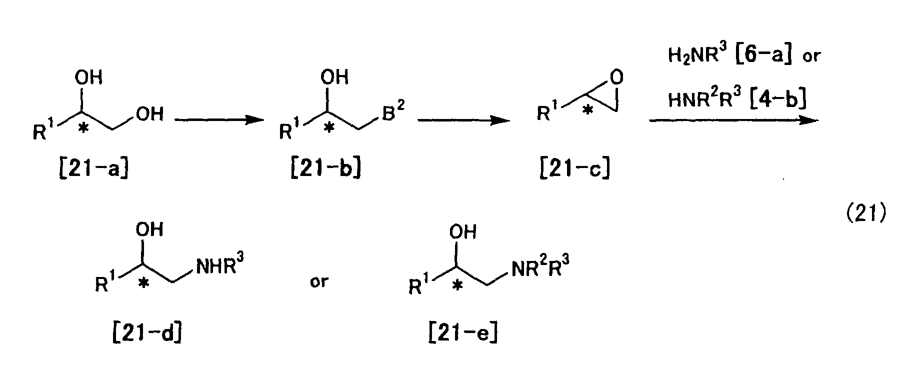 wherein R¹, R² and R³ are as defined above; provided that B² represents a leaving group, and R² is other than acyl group and alkyloxycarbonyl group; and * represents an asymmetric carbon atom, an α-amino group-containing optically active secondary alcohol [21-d] or [21-e] may be obtained from an optically active diol [16-b] wherein n is 0 while maintaining the stereochemistry of *. That is, [21-d] or [21-e] may be obtained by subjecting the primary hydroxyl group to a conventional reaction for converting such a primary hydroxyl group into the leaving group B² to yield the corresponding optically active secondary alcohol having the leaving group B² [21-b]; converting the obtained alcohol [21-b] into the corresponding optically active epoxy compound [21-c] by a cyclization reaction in the presence of a conventional acid scavenger while maintaining the stereochemistry of *; and subjecting the optically active epoxy compound [21-c] to an epoxy ring-opening reaction with a primary amine compound [6-a] or with a secondary amine compound [4-b] to yield the α-amino group-containing optically active secondary alcohol [21-d] or [21-e] in which the stereochemistry of * is maintained.

### Examples

The following examples further illustrate the processes of the present invention but are not intended to limit it in any way. In the examples set forth below, all the reactions were carried out under an atmosphere of inert gas, such as argon gas or nitrogen gas unless otherwise specifically mentioned. In the reactions, dehydrated and degassed solvents were used. The ketone compound-hydrogenating reactions were carried out under a pressurized hydrogen atmosphere in an autoclave. The determination of nuclear magnetic resonance spectrum (NMR) was carried out using GSX-270 (mfd. by NIHON DENSHI; ¹H-NMR, 270 MHz; ¹³C-NMR, 67.5 MHz), JMN-GX 500 (mfd. by NIHON DENSHI; ¹H-NMR, 500 MHz; ¹³C-NMR, 125 MHz; ³¹P-NMR, 202 MHz), α-400 (mfd. by NIHON BUNKO; ¹H-NMR, 400 MHz; ¹³C-NMR, 100 MHz; ³¹P-NMR, 161.6 MHz), or Gemini-300 (mfd. by Varian; ¹H-NMP, 300 MHz). Chemical shift, which is indicated herein in δ(ppm), was determined using tetramethylsilane (TMS) as the internal standard for ¹H-NMR or ¹³C-NMR where the signal from the proton or carbon was defined as δ=0. ³¹P-NMR was measured using a 10% solution of phosphoric acid in heavy water where the signal therefrom was defined as δ=0. Coupling constant is indicated herein in J(Hz). The splitting patterns are indicated using the following abbreviations: s (singlet), d (doublet), t (triplet), q (quartet), m (multiplet), and br (broad). Mass spectrum (MS) was determined by the fast atom bombardment mass spectrometry (FAB-MS) with JEOL-JMS-SX102. Specific rotation [α]_{D} was measured using P-1010-GT (mfd. by NIHON BUNKO) with the solvent specifically mentioned herein and a cell (5 mmf × 5 cm). Gas chromatography analysis was carried out using Type 6890 (mfd. by HEWLETT PACKARD) with capillary column and helium pressure specifically mentioned herein and detection was carried out using FID. High performance liquid chromatography measurement was carried out using Type PU-980 pump (mfd. by NIHON BUNKO) and Type UV-970 UV detector (mfd. by NIHON BUNKO) with column, solvent, flow rate and UV detecting wave length each specifically mentioned herein. Silica gel thin layer chromatography (TLC) and silica gel preparative thin layer chromatography (PTLC) used were Kieselgel 60 F254 Art.1.05719 (mfd. by Merck; thickness 0.25 mm) and Art.1.05715 (mfd. by Merck; thickness 0.25 mm) respectively. Preparative column chromatography used was Kieselgel 60 (mfd. by Merck; 230-400 mesh). Argon gas used was a purified argon gas obtained by passing a standard argon gas (purity 99%) through a column which had been load with BASF catalyst R3-11 and heated to 80°C. Hydrogen gas used was a hydrogen gas (purity 99.99999%) manufactured by NIPPON SANSO. Toluene used was a toluene preserved in a Schlenk tube which had been obtained by distillation from sodium-benzophenone ketyl under an argon atmosphere. N,N-Dimethylformamide (DMF) and N,N-dimethylacetamide (DMA) used were those preserved in Schlenk tubes respectively which had been obtained by distillation from calcium hydride under an argon atmosphere, unless otherwise specifically mentioned. Tetrahydrofuran (THF) used was a tetrahydrofuran freshly obtained by distillation from sodium-benzophenone ketyl under an argon atmosphere with a distillation column. 2-Propanol and dichloromethane used were those freshly obtained respectively by distillation from calcium hydride under a nitrogen atmosphere with a distillation column.

### Example 1

### Synthesis of trans-RuCl₂[(S)-xylbinap][(S)-daipen] (Complex (S,S)-1)

[RuCl₂(benzene)]₂ (261 mg, 0.522 mmol; mfd. by Aldrich) and (S)-Xyl-BINAP (805 mg, 1.10 mmol; synthesized by the process disclosed in JP-B-07-682609) were measured into a 75 mL Schlenk type reaction tube equipped with a teflon-coated stirrer. After air in the tube was reduced evacuated, argon was then introduced. DMF (2 mL) was added with a syringe and the resulting mixture was heated on an oil bath at 100°C under an argon atmosphere for 10 minutes. The reaction solution was allowed to cool down to room temperature and DMF was then distilled off under reduced pressure (1 mmHg). To the thus obtained dark reddish-brown RuCl₂[(S]-xylbinap](dmf)n, (S)-DAIPEN (414 mg, 1.32 mmol; mfd. by KANTO KAGAKU) and dichloromethane (2 mL) were added under an argon stream, followed by stirring at 25°C for 30 minutes. To a green crude product obtained by distilling dichloromethane off under reduced pressure (1 mmHg), hexane (5 mL) was added. The resulting yellow product was dissolved as much as possible and the green impurity was removed by suction filtration. The yellow solution obtained by the suction filtration was concentrated until the complex was deposited. As a result, a solid was obtained.

This procedure was repeated twice. The thus obtained solids were collected by filtration and dried under reduced pressure (1 mmHg) to give Complex (S,S)-1 (803 mg, 0.658 mmol; yield 63%) as a yellow crystal.
¹H-NMR (400 MHz, C₆D₆) δ 0.06 (d, 3H, J=6.6), 0.61 (d, 3H, J=6.8), 1.74 (s, 6H), 1.75 (included in the peaks at δ 1.74 and 1.77, 1H), 1.77 (s, 6H), 2.80 (m, 1H), 3.00 (m, 1H), 3.19 (s, 3H), 3.38 (s, 3H), 3.80 (m, 1H), 4.40 (m, 1H), 5.09 (d, 1H, J=13.4), 5.90-9.00 (m, 32H);
³¹P-NMR (202 MHz, CDCl₃) δ 44.1 (d, J=36.4), 46.7 (d, J=36.4).

### Example 2

### Synthesis of trans-RuCl₂[(S)xylbinap][(S,S)-dpen] (Complex (S,SS)-2)

The title compound was synthesized using [RuCl₂(benzene)]₂ (261 mg, 0.522 mmol), (S)-Xyl-BINAP(805 mg, 1.10 mmol) and (S,S)-DPEN (279 mg, 1.32 mmol; mfd. by KANKYO KAGAKU CENTER) according to the procedure of Example 1, with the exception that the crude product was dissolved in a dichloromethane/diethyl ether solution (3 mL; volume ratio 1/2) and the resulting solution was passed through a column charged with silica gel (10 g) to remove impurities. The yellow solution obtained as a former fraction was concentrated until the complex was deposited. The resulting solid was collected by filtration and dried under reduced pressure (1 mmHg) to give Complex (S,SS)-2 (782 mg, 0.699 mmol; yield 67%) as a yellow crystal.
¹H-NMP (400 MHz, C₆D₆) δ 1.80 (s, 12H), 2.08 (s, 12H), 3.45 (m, 2H), 3.53 (m, 2H), 4.60 (m, 2H), 5.90-9.00 (m, 34H);
³¹P-NMR (202 MHz, CDCI₃) δ 45.0.

### Example 3

### Synthesis of trans-RuCl₂[(S)-xylbinap][(S,S)-cyclohexanediamine] (Complex (S,SS)-3)

The title compound was synthesized using [RuCl₂(benzene)]₂ (130 mg, 0.261 mmol), (S)-Xyl-BINAP (403 mg, 0.548 mmol) and (S,S)-1,2-cyclohexanediamine (75 mg, 0.657 mmol; mfd. by Wako Pure Chemical Industries) according to the procedure of Example 1, with exception that the crude product was dissolved in a dichloromethane/diethyl ether solution (5 mL; volume ratio 1/2) and the resulting solution was passed through a column charged with silica gel (5 g) to remove impurities. The brown solution obtained as a former fraction was concentrated until the complex was deposited. The resulting solid was collected filtration and dried under reduced pressure (1 mmHg) to give Complex (S,SS)-3 (480 mg, 0.470 mmol; yield 90%) as a yellow crystal.
¹H-NMP (400 MHz, C₆D₆) δ 0.50 (m, 4H), 1.00 (m, 2H), 1.10 (m, 2H), 1.80 (s, 12H), 2.10 (m, 2H), 2.18 (s, 12H), 2.70 (m, 2H), 3.00 (br-d, 2H, J=8.6), 5.90-9.0 (m, 24H);
³¹P-NMR (202 MHz, CDCl₃) δ 44.7.

### Example 4

### Synthesis of trans-RuCl₂[(S)-xylbinap][(S)-damen] (Complex (S,S)-4)

The title compound was synthesized from [RuCl₂(benzene)]₂ (25.5 mg, 0.05 mmol), (S)-Xyl-BINAP (73.49 mg, 0.10 mmol) and (S)-DAMEN (31.5 mg, 0.11 mmol; synthesized according to the process described in the literature: Burrows C. J., et al., Tetrahedron Letters, 34(12), pp. 1905-1908 (1993)), with the exception that the crude product was dissolved in a dichloromethane/diethyl ether solution (5 mL; volume ratio 1:2) and the resulting solution was passed through a column charged with silica gel (5 g) to remove impurities. The brown solution obtained as an advance product was concentrated until the complex was deposited. The resulting solid was collected by filtration and dried under reduced pressure (1 mmHg) to give trans-RuCl₂[(S)-xylbinap][(S)-damen] (78.7 mg, 0.066 mmol; yield 66%) as a yellow crystal.
¹H -NMR (400 MHz, CDCl₃) δ 0.68 (d, 3H, J=6.8), 1.74 (s, 6H), 1.75 (s, 6H), 2.08 (s, 6H), 2.30 (s, 6H), 2.65 (m, 1H), 2.74 (m, 1H), 3.39 (m, 1H), 3.75 (s, 3H), 3.83 (s, 3H), 4.05 (m, 1H), 4.80 (br, 1H), 5.88 (d, 2H, J=9.5), 6.06 (t, 2H, J=8.3), 6.60-7.80 (m, 26H), 8.30 (m, 2H);
³¹P-NMR (161.6 MHz, CDCl₃) δ 43.4 (d, J=36.9), 44.0 (d, J=36.9).

Examples of ruthenium/bidentate phosphine/diamine complex which may be synthesized according to the procedure of Example 1 are indicated as the general formula (22).

### Example of Ru Complex:

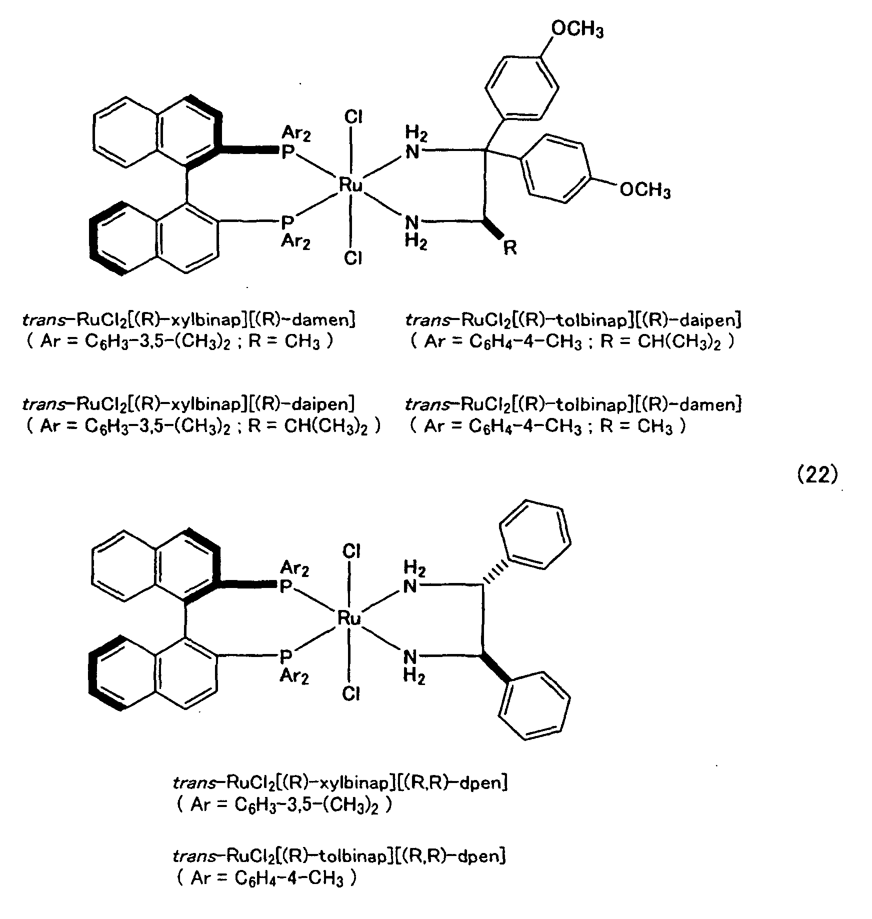

### Example 5

### Asymmetrichydrogenation of 3-dimethylaminopropiophenone

Trans-RuCl₂[(S)-xylbinap][(S)-daipen] (Complex (S,S)-1; 7.3 mg, 6 µmol) was measured into a 500 mL glass autoclave equipped with a teflon-coated stirrer. After air in the autoclave was evacuated, argon was then introduced. 2-Propanol (30 mL) and a solution of potassium tert-butoxide in 2-methyl-2-propanol (1.0 M solution, 60 µL, 60 µmol; mfd. by Aldrich), which had been previously degassed by argon-bubbling, were added with a syringe under an argon stream.

The thus obtained solution was degassed by applying five times the pressure-reducing/argon-introducing procedure to the solution with stirring. The solution was stirred on an oil bath at 60°C for 30 minutes. After the oil bath was removed and the solution was allowed to cool down to room temperature, 3-dimethylaminopropiophenone (10.6 g, 60 mmol; synthesized according to the process described in the literature: Maxwell, C. E., Org. Synthesis, 1955, Coll. Vol. 3, pp. 305-306) and 2-propanol (30 mL) which had been previously degassed by argon-bubbling, were added with a syringe under an argon stream. The thus obtained solution was degassed by applying five times the pressure-reducing/argon-introducing procedure to the solution with stirring. A hydrogen cylinder was connected to the autoclave using a hydrogen-feeding pipe and the atmosphere within the feeding pipe was replaced with hydrogen gas (2 atm) five times. After the pressure in the autoclave was increased to 5 atm, the hydrogen gas was carefully discharged until the pressure was lowered to 2 atm. This procedure was repeated seven times. The pressure of hydrogen gas was then increased to 8 atm, followed by vigorous stirring at 25°C for 5 hours. After the reaction was completed, the thus obtained solution was concentrated under reduced pressure to give (R)-1-phenyl-3-dimethylaminopropan-1-ol (97.5%ee; yield 96% as measured by ¹H-NMR using methyl propionate as the internal standard).

The obtained product was subjected to an abbreviated distillation treatment with Kugel Rohr to give (R)-1-phenyl-3-dimethylaminopropan-1-ol (9.84 g, yield 93%, purity 98%) as a colorless oil.
¹H-NMR (500 MHz, CDCl₃) δ 1.76-1.87 (m, 2H), 2.30 (s, 6H), 2.45-2.49 (m, 2H), 2.63-2.49 (m, 2H), 4.93 (dd, 1H, J=7.8, 3.9), 6.86 (s, 1H), 7.22-7.42 (m, 5H);
¹³C-NMR (125 MHz, CDCl₃) δ 34.5, 45.3, 58.5, 75.8, 125.6, 126.8, 128.2, 145.1.
The enantiomer excess was determined by means of HPLC analysis.
Chiral Column: CHIRALCEL OD (4.6 mm ID × 250 mm; mfd. by Daicel);
Mobile phase: hexane/2-propanol (9/1);
Column temperature: 30°C;
UV wave length: 254 nm;
Flow rate: 0.5 mL/min;
Retention time:
   (R)-1-phenyl-3-dimethylaminopropan-1-ol: 12.5 min (96.75%)
   (S)-1-phenyl-3-dimethylaminopropan-1-ol: 16.2 min (1.25%).
The absolute structure was determined as R-form by comparing the specific rotation with the known specific rotation disclosed in the following reference.
Actual measurement value: [α] ²⁶_{D}=+32.0 (c=1.695, CH₃OH)
Literature data of R-form: [α]_{D}=+27.6 (c=1.61, CH₃OH)
   Reference: Andrisano, R.; Angeloni, A. S.; Marzocchi, S., Tetrahedron, 1973, 29, pp. 913-916.

### Comparative Example 1

The procedure of this comparative example is different from that of Example 5. This comparative example was carried out by a procedure comprising no step of previously preparing an activated catalyst species using a base and Complex (S,S)-1 which is a catalyst precursor. In this case, the decomposition of 3-dimethylamino-propiophenone took precedence over its hydrogenation. Both purity and yield of the thus obtained (R)-1-phenyl-3-dimethylaminopropan-1-ol in the asymmetrically reduced form were lowered. The said comparative example will be described in more detail.

Complex (S,S)-1 (1.5 mg, 1.25 µmol) was measured into a 100 mL glass autoclave equipped with a teflon-coated stirrer. After air in the autoclave was evacuated, argon was then introduced. 3-Dimethylaminopropiophenone (443 mg, 2.5 mmol), 2-propanol (2.5 mL), and a solution of potassium tert-butoxide in 2-methyl-2-propanol (1.0 M solution, 25 mL, 25 mmol; mfd. by Aldrich) which had been previously degassed by argon-bubbling, were added with a syringe under an argon stream. The thus obtained solution was degassed by applying five times the pressure-reducing/argon-introducing procedure to the solution with stirring.

A hydrogen cylinder was connected to the autoclave using a hydrogen-feeding pipe and the atmosphere within the feeding pipe was replaced with hydrogen gas (2 atm) five times. After the pressure in the autoclave was increased to 5 atm, the hydrogen gas was carefully discharged until the pressure was lowered to 2 atm. This procedure was repeated seven times. The pressure of hydrogen gas was then increased to 8 atm, followed by vigorous stirring at 25°C for 3 hours. After the reaction solution was concentrated under reduced pressure, the residue was subjected to a silica gel column chromatography treatment (silica gel 20 g; solvent: dichloromethane) to give (R)-1-phenyl-3-dimethylaminopropan-1-ol (102 mg, 0.6 mmol, yield 24%, 97%ee).

Therefore, the most preferred example of the present invention is a process according to the procedure of Example 5 comprising previously activating a catalyst by reacting a complex with a base at 60°C for 30 minutes or more, and then conducting hydrogenation of a substrate by adding the substrate and pressurizing with hydrogen. In the following, Examples 6 to 24, Example 26 and Example 28 were carried out according to the procedure of Example 5.

### Example 6

### Asymmetric hydrogenation of 2-dimethylaminoacetophenone (Intermediate 7: free-form)

Complex (R,R-1) (6.1 mg, 5 µmol; synthesized using R-form ligands according to the procedure of Example 1) was measured into a 100 mL glass autoclave equipped with a teflon-coated stirrer. An asymmetric hydrogenation according to the procedure of Example 5 was carried out using 2-dimethylaminoacetophenone (1.63 g, 10 mmol; freshly obtained by neutralizing a hydrobromide salt of Intermediate 7 with a 0.1 N potassium hydroxide solution, and extracting the thus neutralized material with diethyl ether followed by drying treatment under reduced pressure), a 1.0 M solution of potassium tert-butoxide in 2-methyl-2-propanol (100 µL, 0.1 mmol; mfd. by Aldrich) and 2-propanol (10 mL) with vigorous stirring under a hydrogen atmosphere at 8 atm at 25°C for 12 hours. The resulting reaction mixture was then subjected to an abbreviated distillation treatment with Kugel Rohr to give (R)-1-phenyl-2-dimethylaminoethanol (93%ee, 1.49 g, yield 90%) as a colorless oil.
¹H-NMR (400 MHz, CDCl₃) δ 2.31-2.51 (m, 8H), 4.69 (dd, 1H, J=10.8, 3.2), 7.25-7.39 (m, 5H).
The enantiomer excess was determined by means of HPLC analysis of an ester-form obtained by benzoylating the thus obtained product.
Chiral Column: CHIRALCEL OD (4.6 mm ID × 250 mm; mfd. by Daicel);
Mobile phase: hexane/2-propanol (49/1);
Column temperature: 40°C;
UV wave length: 254 nm;
Flow rate: 0.3 mL/min;
Retention time:
   Benzoate of (S)-1-phenyl-2-dimethylaminoethanol: 16.0 min (3.3%)
   Benzoate of (R)-1-phenyl-2-dimethylaminoethanol: 18.2 min (96.7%)
The absolute structure was determined as R-form by comparing the specific rotation with the known specific rotation of (R)-1-phenyl-2-dimethylaminoethanol hydrochloride disclosed in the following reference.
Actual measurement value: [α]²⁷_{D}=-64.0 (c=0.635, C₂H₅OH)
Literature data of R-form: [α]^{23.5}_{D}=-78.9( c=0.985, C₂H₅OH) [α]_{D} reference: Chan, M. M-L.; Robinson, J. B., J. Med. Chem., 1974, 17, p. 1057.

### Example 7

### Asymmetric hydrogenation of dimethylaminoacetone

Complex (R,R)-1 (1.5 mg, 1.25 µmol; synthesized using R-form ligands according to the procedure of Example 1) was measured into a 100 mL glass autoclave equipped with a teflon-coated stirrer. An asymmetric hydrogenation according to the procedure of Example 5 was carried out using dimethylaminoacetone (253 mg, 2.5 mmol; mfd. by Aldrich), a 1.0 M solution of potassium tert-butoxide in 2-methyl-2-propanol (20 µL, 20 µmol; mfd. by Aldrich) and 2-propanol (2.5 mL) with vigorous stirring under a hydrogen atmosphere at 8 atm at 25°C for 4 hours. After the reaction was completed, the thus obtained solution was diluted with ether, and a 1.0 M solution of hydrogen chloride in ether (3 mL, 3 mmol; mfd. by Aldrich) was added. The resulting mixture was concentrated under reduced pressure to give 1-dimethylamino-2-propanol monohydrochloride. The yield was 99% as measured by ¹H-NMR analysis.
¹H-NMR (400 MHz, DMSO-d6) δ 1.10 (d, 3H, J=6.0), 2.77 (dd, 6H, J=12.8, 4.8), 2.90-2.97 (m, 1H), 3.02-3.08 (m, 1H), 4.03-4.11 (m, 1H), 9.95 (br, 1H).

To the thus obtained 1-dimethylamino-2-propanol monohydrochloride were added dichloromethane (10 mL), distilled water (200 mL) and sodium hydroxide (120 mg, 3 mmol; mfd. by Nacalai Tesque). The resulting mixture was stirred for 2 hours. dichloromethane was distilled off under reduced pressure, followed by an abbreviated distillation treatment with Kugel Rohr. The thus obtained oil was diluted with dichloromethane, dried over anhydrous sodium sulfate and then filtered. The filtrate was concentrated under reduced pressure to give (S)-1-dimethylamino-2-propanol (92%ee, 205 mg, 1.99 mmol, yield 79%) as a light yellow oil.
¹H-NMR (400 MHz, CDCl₃) δ 1.13 (d, 3H, J=6.4), 2.12-2.27 (m, 8H), 3.77-3.80 (m, 1H).
The enantiomer excess was determined based on the area ratio of the methine proton at 2-position according to ¹H NMR analysis using a shift reagent. The measurement was carried out using 1-dimethylamino-2-propanol (22 mg, 0.21 mmol) and europium tris[3-heptafluoropropylhydroxymethylene-(+)-camphorate] (200 mg, 0.17 mmol) in CDCl₃. The absolute structure was determined as S-form by comparing the specific rotation with the known specific rotation of (R)-1-dimethylamino-2-propanol disclosed in the following reference.
Actual measurement value: [α] ²⁷_{D}=+21.1 (c=1.006, CH₃OH);
Literature data of R-form: [α]_{D}=-23.7 (c=1.11 CH₃OH);
[α]_{D} reference: Tomita, L. D.; Klabunovskii, E. I.; Petrov, Yu. I.; Kretova, L. A.; Kholdyakov, N. I.; Antonova, T. A.; Cherkasova, E. M., Bull. Acad. Sci. USSR. Div. Chem, Sci. (Engl. Transl.), 1971, 20, p. 2063.

### Example 8

### Asymmetric hydrogenation of 2-[(methyl) (phenyl)amino]acetone (Intermediate 8)

Complex (R,R-1) (1.5 mg, 1.25 µmol) was measured into a 100 mL glass autoclave equipped with a teflon-coated stirrer. An asymmetric hydrogenation according to the procedure of Example 5 was carried out using Intermediate 8 (408 mg, 2.5 mmol), a solution of potassium tert-butoxide in 2-methyl-2-propanol (1.0 M solution, 25 µL, 25 µmol; mfd. by Aldrich) and 2-propanol (2.5 mL) with vigorous stirring under a hydrogen atmosphere at 8 atm at 25°C for 13 hours. After the reaction was completed, the thus obtained solution was concentrated under reduced pressure and compounds derived from the complex was removed by a silica gel chromatography treatment (silica gel 10 g; solvent: diethyl ether) to give 1-[(methyl)(phenyl)amino]-2-propanol (81%ee, 384 mg, 2.32 mmol, yield 93%) as a colorless oil.
¹H-NMR (270 MHz, CDCl₃) δ 1.23 (d, 3H, J=12.7), 2.18 (s, 1H), 2.98 (s, 3H), 3.23 (d, 2H, J=6.5), 4.06-4.18 (m, 1H), 6.74-6.85 (m, 3H), 7.20-7.29 (m, 2H).
The enantiomer excess was determined by means of HPLC analysis.
Chiral Column: CHIRALCEL OD (4.6 mm ID × 250 mm; mfd. by Daicel);
Mobile phase: hexane/2-propanol (9/1);
Column temperature: 30°C;
UV wave length: 254 nm;
Flow rate: 0.5 mL/min;
Retention time:
   (S)-1-[(methyl)(phenyl)amino]-2-propanol: 14.7 min (90.7%)
   (R)-1-[(methyl)(phenyl)amino]-2-propanol: 22.2 min (9.3%)
The absolute structure was determined by comparing the specific rotation with the specific rotation of a separately synthesized (S)-1-[(methyl)(phenyl)amino]-2-propanol.

Actual measurement value of the asymmetrically hydrogenated product: [α]23_{D}=+19.0 (c=0.545, CH₃OH)

Specific rotation of separately synthesized (S)-1-[(methyl)-(phenyl)amino]-2-propanol (>99%ee) having a known absolute structure: [α]²³_{D}=+26.3 (c=0.538, CH₃OH)

A process for the preparation of the separately synthesized (S)-1-[(methyl)(phenyl)-amino]-2-propanol will be described below.

### Synthesis of (S)-1-[(methyl)(phenyl)amino]-2-propanol

Alumina (26 g; mfd. by Merck), diethyl ether (80 mL) and N-methylaniline (1.07 g, 10 mmol; mfd. by TOKYO KASEI) were added to a 100 mL recovery flask equipped with a teflon-coated stirrer. To the resulting mixture, (S)-propylene oxide (140 µL, 2 mmol; mfd. by TOKYO KASEI) diluted with diethyl ether (2 mL) was added dropwise with vigorous stirring. After stirring at 25°C for 6 hours, the reaction liquid was filtered to give a solution, which was then concentrated under reduced pressure. The residue was subjected to a silica gel chromatography treatment (silica gel 70 g; 1:3 ethyl acetate/hexane) to give as the first fraction, (S)-1-[(methyl)(phenyl)amino]-2-propanol (123 mg, 0.746 mmol, yield 37%) as a colorless oil.
¹H-NMR (270 MHz, CDCl₃) δ 1.23 (d, 3H, J=12.7), 2.18 (s, 1H), 2.98 (s, 3H), 3.23 (d, 2H, J=6.5Hz), 4.06-4.18 (m, 1H), 6.74-6.85 (m, 3H), 7.20-7.29 (m, 2H);
[α]²³_{D}=+26.3 (c=0.538, CH₃OH) , >99%ee(S).

### Example 9

### Asymmetric hydrogenation of 2-[(benzoyl)(3,4-dimethoxyphenethyl)amino]-4'-benzyloxyacetophenone

Complex (R,R-1) (1.5 mg, 1.25 µmol) was measured into a 100 mL glass autoclave equipped with a teflon-coated stirrer. An asymmetric hydrogenation according to the procedure of Example 5 was carried out using 2-[(benzoyl)(3,4-dimethoxyphenethyl)amino]-4'-benzyloxyacetophenone (1.27 g, 2.5 mmol; prepared according to the method described in YAKUGAKU ZASSHI, 106(1), pp. 80-89(1986)), a solution of potassium tert-butoxide in 2-methyl-2-propanol (1.0 M solution, 50 µL, 50 µmol; mfd. by Aldrich) and 2-propanol (2.5 mL) with vigorous stirring under a hydrogen atmosphere at 8 atm at 25°C for 24 hours.

After the reaction was completed, the thus obtained solution was concentrated under reduced pressure and compounds derived from the complex was removed by a silica gel chromatography treatment (silica gel 5 g; solvent: ethyl acetate) to give (R)-2-[(benzoyl)-(3,4-dimethoxyphenethyl)amino]-1-(4'-benzyloxyphenyl)ethanol (97%ee, 1.28 g, 2.5 mmol, yield 100%) as a white solid.
The enantiomer excess was determined by means of HPLC analysis.
Chiral Column: CHIRALPAK AD (4.6 mm ID× 250 mm; mfd. by Daicel);
Mobile phase: hexane/2-propanol (3/1);
Column temperature: 40°C;
UV wave length: 254 nm;
Flow rate: 1 mL/min;
Retention time:
   (R)-2-[(benzoyl)(3,4-dimethoxyphenethyl)amino]-1-(4'-benzyloxyphenyl)ethanol: 11.7 min (98.6%);
   (S)-2-[(benzoyl)(3,4-dimethoxyphenethyl)amino]-1-(4'-benzyloxyphenyl)ethanol: 14.8 min (1.4%).
      The absolute structure was determined after removing the benzoyl group. The absolute structure-determining process will be described below.

### Synthesis of (R)-1-(4-benzyloxyphenyl)-2-[2-(3,4-dimethoxyphenyl)ethylamino]ethanol monohydrochloride

To a 50 mL recovery flask equipped with a teflon-coated stirrer and a reflux column, (R)-2-[(benzoyl)(3,4-dimethoxyphenethyl)amino]-1-(4'-benzyloxyphenyl)ethanol (1.28 g, 2.5 mmol; obtained in Example 9), ethanol (25 mL), distilled water (2.5 mL) and 85% potassium hydroxide (pellet, 660 mg, 10 mmol; mfd. by Nacalai Tesque). The resulting mixture was placed on an oil bath and refluxed for 8 hours. After allowing to cool down to room temperature, the mixture was extracted with distilled water and ethyl acetate. The organic layer was washed with saturated brine, dried over anhydrous sodium sulfate and then filtered. The thus obtained solution was concentrated under reduced pressure. The residue was diluted with diethyl ether (30 mL). After a solution of hydrogen chloride in ether (1 M solution, 3 mL, 3 mmol; mfd. by Aldrich) was added dropwise, the resulting mixture was concentrated under reduced pressure to give the title debenzoylated compound, (R)-1-(4-benzyloxyphenyl)-2-[2-(3,4-dimethoxyphenyl)ethylamino]ethanol monohydrochloride (1.10 g, 2.48 mmol, yield 99%).

This was recrystalized from ethanol to give an optically pure (R)-1-(4-benzyloxyphenyl)-2-[2-(3,4-dimethoxyphenyl)ethylamino]-ethanol monohydrochloride (927 mg, 2.09 mmol, yield 84%).
¹H-NMR (400 MHz, DMSO-d6) δ 2.68-2.85 (m, 6H), 3.71 (s, 3H), 3.73 (s, 3H), 4.63 (m, 1H), 5.09 (s, 2H), 6.70-6.72 (m, 1H), 6.80-6.86 (m, 2H), 6.96 (d, 2H, J=8.4), 7.24 (d, 2H, J=8.8), 7.30-7.44 (m, 5H).
The enantiomer excess was determined by means of optically active HPLC analysis of free amine form of 1-(4'-benzyloxyphenyl)-2-[(3,4-dimethoxyphenethyl)amino]ethanol.
HPLC
Column: CHIRALPAK AD (4.6 mm ID× 250 mm; mfd. by Daicel);
Solvent: hexane/2-propanol (4/1);
Temperature: 40°C;
UV wave length: 254 nm;
Flow rate: 0.5 mL/min;
Retention time:
   (R)-1-(4'-benzyloxyphenyl)-2-[(3,4-dimethoxyphenethyl)amino]ethanol:
   19.2 min (100%);
   (S)-1-(4'-benzyloxyphenyl)-2-[(3,4-dimethoxyphenethyl)amino]ethanol:
   21.2 min (0%).
Actual measurement value: [α]²⁵_{D}=-30.5 (c=0.96, CH₃OH) ;
Literature data of R-form: [α]_{D}=-30.1 (c=1, CH₃OH);
[α]_{D} reference: Kawaguchi, T.; Saito, K.; Matsuki, K.; Iwakuma, T.; Takeda, M., Chem. Pharm. Bull., 1993, 41, p. 639.

### Example 10

### Asymmetric hydrogenation of 4'-fluoro-4-[4-(5-fluoro-2-pyrimidinyl)-1-piperazinyl]butyrophenone (S/C=2000, 0.5 M)

Complex (S,S-1) (1.5 mg, 1.25 µmol) was measured into a 100 mL glass autoclave equipped with a teflon-coated stirrer. An asymmetric hydrogenation according to the procedure of Example 5 was carried out using 4'-fluoro-4-[4-(5-fluoro-2-pyrimidinyl)-1-piperazinyl]butyrophenone (866 mg, 2.5 mmol; mfd. by the process described in Yevich, J. P.; New, J. S.; Lobeck, W. G.; Dextraze, P.; Bernstein, E.; Taylor, D. P.; Yocca, F. D.; Eison, M. S.; Temple, Jr. D. L., J. Med. Chem., 1992, 35, p. 4516), a solution of potassium tert-butoxide in 2-methyl-2-propanol (1.0 M solution, 50 µL, 50 µmol; mfd. by Aldrich) and 2-propanol (5 mL) with vigorous stirring under a hydrogen atmosphere at 8 atm at 25°C for 12 hours.

After the reaction was completed, the thus obtained solution was concentrated under reduced pressure and compounds derived from the complex was removed by a silica gel chromatography treatment (silica gel 5 g; solvent: ethyl acetate) to give (R)-1-(4-fluorophenyl)-4-(5-fluoro-2-pyrimidinyl)-1-piperazinebutanol (99%ee, 843 mg, 2.42 mmol, yield 97%) as a white solid.
¹H-NMR (400 MHz, CDCl₃) δ 1.65-1.98 (m, 4H), 2.44-2.65 (m, 6H), 3.83 (m, 2H), 4.66 (dd, 1H, J=7.6, 2.4), 6.98-7.02 (m, 2H), 7.32-7.35 (m, 2H), 8.19 (s, 2H);
¹³C-NMR (100 MHz, CDCl₃) δ 23.56, 39.52, 43.81, 52.73, 58.74, 72.95, 114.8 (J_{CF}=21.0), 127.1 (J_{CF}=7.7), 141.3 (J_{CF}=2.8), 145.0 (J_{CF}=21.4), 151.6 (J_{CF}=247.1), 158.7, 161.7 (J_{CF}=243.0).
The enantiomer excess was determined by means of HPLC analysis.
Chiral Column: CHIRALPAK AD (4.6 mm ID× 250 mm; mfd. by Daicel);
Mobile phase: hexane/2-propanol (19/1);
Column temperature: 40°C;
UV wave length: 254 nm;
Flow rate: 0.5 mL/min;
Retention time:
   (S)-1-(4-fluorophenyl)-4-(5-fluoro-2-pyrimidinyl)-1-piperazinebutanol: 27.4 min (0.3%);
   (R)-1-(4-fluorophenyl)-4-(5-fluoro-2-pyrimidinyl)-1-piperazinebutanol: 29.4 min (99.7%)
The absolute structure was determined as R-form by comparing the specific rotation with the known specific rotation disclosed in the following reference.
Actual measurement value: [α]²⁵_{D}=+14.6 (c=1.05, CH₃OH);
Literature data of R-form (>99.9%ee):
   [α]²⁵_{D}=+14.3 (c=0.53, CH₃OH);
[α]_{D} reference: Yevich, J. P.; New, J. S.; Lobeck, W. G.; Dextraze, P.; Bernstein, E.; Taylor, D. P.; Yocca, F. D.; Eison, M. S.; Temple, Jr. D. L., J. Med. Chem., 1992, 35, p. 4516.

### Example 11

### Asymmetric hydrogenation of 4'-fluoro-4-[4-(5-fluoro-2- pyrimidinyl)-1-piperazinyl]butyrophenone (S/C=2000, 1 M)

The procedure of Example 10 was repeated with the exception that the reactant concentrations were altered as follows. Catalyst: Complex (R,R)-1 (1.5 mg, 1.25 µmol); Substrate: 4'-fluoro-4-[4-(5-fluoro-2-pyrimidinyl)-1-piperazinyl]butyrophenone (866 mg, 2.5 mmol); Solvent: 2-propanol (2.5 mL); Base: a solution of potassium tert-butoxide in 2-methyl-2-propanol (1.0 M solution, 50 µL, 50 µmol; mfd. by Aldrich); Hydrogen pressure: 8 atm; Temperature: 25°C; Reaction time: 32 hours; Isolated yield: 78%; Enantiomer excess: 99%; Absolute structure: S.

### Example 12

### Asymmetric hydrogenation of 4'-fluoro-4-[4-(5-fluoro-2-pyrimidinyl)-1-piperazinyl]butyrophenone (S/C=2000, 1 M)

The procedure of Example 10 was repeated with the exception that alterations were made as follows. Catalyst: trans-RuCl₂[(R)-tolbinap] [(R,R)-dpen] (1.2 mg, 1.25 µmol; synthesized from (R)-Tol-BIANP (mfd. by AZMAX), (R,R)-DPEN (mfd. by KANKYO KAGAKU CENTER) and [RuCl₂(benzene)]₂ (mfd. by Aldrich) according to the procedure of Example 1); Substrate: 4'-fluoro-4-[4-(5-fluoro-2-pyrimidinyl)-1-piperazinyl]butyrophenone (866 mg, 2.5 mmol); Solvent: 2-propanol (2.5 mL); Base: a solution of potassium tert-butoxide in 2-methyl-2-propanol (1.0 M solution, 50 µL, 50 µmol; mfd. by Aldrich); Hydrogen pressure: 8 atm; Temperature: 25°C; Reaction time: 16 hours; Isolated yield: 78%; Enantiomer excess: 74%; Absolute structure: S.

### Example 13

### Asymmetric hydrogenation of 4'-fluoro-4-[4-(5-fluoro-2-pyrimidinyl)-1-piperazinyl]butyrophenone (S/C=10000, 0.5 M)

Complex (S,S)-1 (1.2 mg, 1.0 µmol) was measured into a 500 mL glass autoclave equipped with a teflon-coated stirrer. An asymmetric hydrogenation according to the procedure of Example 5 was carried out using 4'-fluoro-4-[4-(5-fluoro-2-pyrimidinyl)-1-piperazinyl]butyrophenone (3.46 g, 10 mmol), a solution of potassium tert-butoxide in 2-methyl-2-propanol (1.0 M solution, 200 µL, 0.2 mmol; mfd. by Aldrich) and 2-propanol (20 mL) with vigorous stirring under a hydrogen atmosphere at 8 atm at 25°C for 32 hours. After the reaction was completed, the thus obtained solution was concentrated under reduced pressure and subjected to a silica gel chromatography treatment (silica gel, 170g; solvent: benzene/ethyl acetate (1/1) to ethyl acetate) to give as the second fraction (R)-1-(4-fluorophenyl)-4-(5-fluoro-2-pyrimidinyl)-1-piperazinebutanol (99%ee, 3.29 g, 9.45 mmol, yield 94.5%) as a white solid. This was recrystalized twice from ethanol to give an optically pure (R)-1-(4-fluorophenyl)-4-(5-fluoro-2-pyrimidinyl)-1-piperazinebutanol (2.55 g, 7.32 mmol, yield 73%) as a white solid.

### Example 14

### Asymmetric hydrogenation of benzyloxyacetone (Intermediate 9)

Complex (S,S)-1 (1.5 mg, 1.25 (µmol) was measured into a 100 mL glass autoclave equipped with a teflon-coated stirrer. An asymmetric hydrogenation according to the procedure of Example 5 was carried out using Intermediate 9 (411 mg, 2.5 mmol), a solution of potassium tert-butoxide in 2-methyl-2-propanol (1.0 M solution, 20 µL, 20 µmol; mfd. by Aldrich) and 2-propanol (2.5 mL) with vigorous stirring under a hydrogen atmosphere at 8 atm at 25°C for 13 hours.

After the reaction was completed, the thus obtained solution was concentrated under reduced pressure and compounds derived from the complex were removed by a silica gel chromatography treatment (silica gel 5 g; solvent: diethyl ether) to give (R)-1-benzyloxy-2-propanol (79%ee, 398 mg, 2.39 mmol, yield 96%) as a colorless oil.
¹H-NMR (400 MHz, CDCl₃) δ 1.15 (d, 3H, J=6.0), 2.45 (s, 1H), 3.29 (dd, 1H, J=9.2, 7.6), 3.47 (dd, 1H, J=9.2, 3.2), 3.96-4.04 (m, 1H), 4.56 (s, 2H), 7.27-7.38 (m, 5H);
¹³C-NMR (100 MHz, CDCl₃), d 18.60, 66.48, 73.29, 75.29, 127.71, 127.76, 128.44, 137.95.
The enantiomer excess was determined by means of HPLC analysis.
Chiral Column: CHIRALCEL OD (4.6 mm ID× 250 mm; mfd. by Daicel);
Mobile phase: hexane/2-propanol (95/5);
Column temperature: 40°C;
UV wave length: 254 nm;
Flow rate: 1 mL/min;
Retention time:
   (R)-1-benzyloxy-2-propanol: 8.1 min (89.5%);
   (S)-1-benzyloxy-2-propanol: 9.2 min (10.5%).
The absolute structure was determined as R-form by comparing the specific rotation with the known specific rotation disclosed in the following reference.
Actual measurement value: [α]²⁶_{D}=-11.8 (c=1.026, CHCl₃);
Literature data of S-form: [α]²⁴_{D}=+13.2 (c=1, CHCl₃);
   [α]_{D} reference: Naemura, K.; Asada, M.; Hirose, K.; Tobe, Y., Tetrahedron:Asymmetry, 1995, 6, pp. 1873-1876.

### Example 15

### Asymmetric hydrogenation of 2-methoxyacetophenone

Complex (R,R)-1 (1.5 mg, 1.25 µmol) was measured into a 100 mL glass autoclave equipped with a teflon-coated stirrer. An asymmetric hydrogenation according to the procedure of Example 5 was carried out using 2-methoxyacetophene (376 mg, 2.5 mmol; mfd. by Aldrich), a solution of potassium tert-butoxide in 2-methyl-2-propanol (1.0 M solution, 20 µL, 20 µmol; mfd. by Aldrich) and 2-propanol (2.5 mL) with vigorous stirring under a hydrogen atmosphere at 8 atm at 25°C for 16 hours.

After the reaction was completed, the thus obtained solution was concentrated under reduced pressure and compounds derived from the complex were removed by a silica gel chromatography treatment (silica gel 5 g; solvent: diethyl ether) to give (R)-1-phenyl-2-methoxyethanol (95%ee, 350 mg, 2.30 mmol, yield 92%) as a light yellow oil.
¹H -NMR (400 MHz, CDCl₃) δ 2.79 (s, 1H), 3.41-3.53 (m, 4H), 3.55 (dd, 1H, J-9.6, 2.8), 4.89 (dd, 1H, J=8.8, 2.8), 7.29-7.40 (m, 5H).
The enantiomer excess was determined by means of GC analysis.
Column: Chirasil-DEX CB (inner diameter (df) 0.25 mm;
   size 0.32 mm × 25 mm; mfd. by CHROMPAK);
Column temperature: 120°C;
Temperature of injection and detection: 200°C;
Helium pressure: 44 kPa;
Retention time:
   (S)-1-phenyl-2-methoxyethanol: 19.5 min (2.6%);
   (R)-1-phenyl-2-methoxyethanol: 19.8 min (97.4%).
The absolute structure was determined by comparing the specific rotation with the known specific rotation disclosed in the following reference.
Actual measurement value: [α]²⁷_{D}=-38.4 (c=2.165, acetone);
Literature data of R-form (76%ee): [α]_{D}=-34.4 (c=2, acetone);
[α]_{D} reference: Ferraboschi, P.; Grisenti, P.; Manzocchi, A.; Santaniello, E., Tetrahedron, 1994, 50, pp. 10539-10548.

### Example 16

### Asymmetric hydrogenation of phenoxyacetone

Complex (R,R)-1 (1.5 mg, 1.25 µmol) was measured into a 100 mL glass autoclave equipped with a teflon-coated stirrer. An asymmetric hydrogenation according to the procedure of Example 5 was carried out using phenoxyacetone (376 mg, 2.5 mmol; mfd. by TOKYO KASEI), a solution of potassium tert-butoxide in 2-methyl-2-propanol (1.0 M solution, 20 µL, 20 µmol; mfd. by Aldrich) and 2-propanol (2.5 mL) with vigorous stirring under a hydrogen atmosphere at 8 atm at 25°C for 1 hour.

After the reaction was completed, the thus obtained solution was concentrated under reduced pressure and compounds derived from the complex were removed by a silica gel chromatography treatment (silica gel 15 g; solvent: diethyl ether) to give 1-phenoxy-2-propanol (80%ee, 358 mg, 2.33 mmol, yield 93%) as a colorless oil.
¹H-NMR (270 MHz, CDCl₃) δ 1.29 (d, 3H, J=6.5), 2.36 (s, 1H), 3.79 (dd, 1H, J=7.8, 9.2), 3.95 (dd, 1H, J=9.5, 3.0), 4.15-4.26 (m, 1H), 6.89-7.00 (m, 3H), 7.24-7.33 (m, 2H).
The enantiomer excess was determined by means of GC analysis.
Column: Chirasil-DEX CB (inner diameter (df) 0.25 mm; size 0.32 mm × 25 mm; mfd. by CHROMPAK);
Column temperature: 100°C;
Temperature of injection and detection: 200°C;
Helium pressure: 44 kPa;
Retention time:
   (S)-1-phenoxy-2-propanol: 52.9 min (90.0%);
   (R)-1-phenoxy-2-propanol: 58.3 min (10.0%).
The absolute structure was determined as S-form by comparing the specific rotation with the known specific rotation disclosed in the following reference.

Actual measurement value : [α]²⁷_{D}=-3.05 (c=3.04, C₂H₅OH); Literature data of S-form (>99%ee)
: [α]²⁰_{D}=-2.7 (c=1.80, C₂H₅OH);
[α]_{D} reference: Waagen, V.; Partali, V.; Hollingsaeter, I.; Huang, M.S.S.; Anthonsen, T., Acta Chemica Scandinavica, 1994, 48, pp. 506-510.

### Example 17

### Asymmetric hydrogenation of 2-(tert-butyldiphenylsilyl)oxy-acetophenone (Intermediate 10)

Complex (R,R)-1 (1.5 mg, 1.25 µmol) was measured into a 100 mL glass autoclave equipped with a teflon-coated stirrer. An asymmetric hydrogenation according to the procedure of Example 5 was carried out using Intermediate 10 (936 mg, 2.5 mmol), a solution of potassium tert-butoxide in 2-methyl-2-propanol (1.0 M solution, 25 µL, 25 µmol; mfd. by Aldrich) and 2-propanol (2.5 mL) with vigorous stirring under a hydrogen atmosphere at 8 atm at 25°C for 13 hours.

After the reaction was completed, an oil obtained by a concentrating treatment under reduced pressure was subjected to a silica gel chromatography treatment (silica gel 50 g; solvent: hexane/diethyl ether=4/1) to give as the first fraction 2-(tert-butyldiphenylsilyl)oxy-1-phenylethanol (746 mg, yield 79%) as a colorless oil; ¹H-NMR (400 MHz, CDCl₃) δ 1.08 (s, 9H), 3.00 (s, 1H), 3.66 (dd, 1H, J=10.4, 8.4), 3.79 (dd, 1H, J=10.4, 3.6), 4.79-4.82 (m, 1H), 7.25-7.44 (m, 11H), 7.60-7.66(m, 4H); and as the second fraction 1-(tert-butyldiphenylsilyl)oxy-1-phenyletanol (107 mg, yield 11%) as a colorless oil: ¹H-NMR(400MHz, CDCl₃) δ 1.06 (s, 9H), 3.56-3.59 (m, 1H), 3.63-3.66 (m, 1H), 4.79 (dd, 1H, J=6.8, 4.4), 7.23-7.45 (m, 13H), 7.68-7.70 (m, 2H).

The enantiomer excess and absolute structure were determined for the diol form obtained by removing the silyl group as a protecting group as described in Example 18.

### Example 18

### Synthesis of (R)-1-phenyl-1,2-ethanediol

Complex (R,R)-1 (1.5 mg, 1.25 µmol) was measured into a 100 mL glass autoclave equipped with a teflon-coated stirrer. An asymmetric hydrogenation according to the procedure of Example 5 was carried out using Intermediate 10 (936 mg, 2.5mmol), a solution of potassium tert-butoxide in 2-methyl-2-propanol (1.0 M solution, 25 µL, 25 µmol; mfd. by Aldrich) and 2-propanol (2.5 mL) with vigorous stirring under a hydrogen atmosphere at 8 atm at 25°C for 14 hours.

After the reaction was completed, the thus obtained solution was put into a 20 mL recovery flask and concentrated under reduced pressure. THF (5 mL) and a solution of tetra-n-butylammonium fluoride in THF (1.0 M solution, 3.75 mL, 3.75 mmol; mfd. by Aldrich) were added under a argon stream, and the resulting mixture was stirred at 25°C for 8 hours. The mixture was concentrated under reduced pressure and then subjected to a silica gel chromatography treatment (silica gel 50 g; solvent: hexane/ethyl acetate=2/3) to give as the second fraction (R)-1-phenyl-1,2-ethanediol (97%ee, 320 mg, 2.32 mmol, yield 93%) as a yellow solid.
¹H-NMR (270 MHz, CDCl₃) δ 2.10 (br, 1H), 2.55 (br, 1H), 3.63-3.79 (m, 2H), 4.83 (dd., 1H, J=7.9, 3.3), 7.23-7.40 (m, 5H). The enantiomer excess was determined by means of HPLC analysis.
Chiral Column: CHIRALCEL OB (4.6 mm ID× 250 mm; mfd. by Daicel);
Mobile phase: hexane/2-propanol (9/1);
Column temperature: 40°C;
UV wave length: 254 nm;
Flow rate: 0.5 mL/min;
Retention time:
   (R)-1-phenyl-1,2-ethanediol: 12.3 min (98.7%);
   (S)-1-phenyl-1,2-ethanediol: 14.6 min (1.3%).
The absolute structure was determined as R-form by comparing the specific rotation with the known specific rotation disclosed in the following reference.
Actual measurement value: [α]²⁴_{D}=-38.4 (c=1.12, C₂H₅OH);
Literature data of S-form (99%ee): [α]²⁵_{D}=+38.91(c=3.61, C₂H₅OH) ;
[α]_{D} reference: Cho, B. T.; Chun, Y. S., J.Org.Chem., 1998, 63, pp. 5280-5282.

### Example 19

### Asymmetric hydrogenation of 1,1-dimethoxy-2-propanone

Complex (R,R)-1 (1.5 mg, 1.25 µmol) was measured into a 100 mL glass autoclave equipped with a teflon-coated stirrer. An asymmetric hydrogenation according to the procedure of Example 5 was carried out using 1,1-dimethoxy-2-propanone (595 mg, 5.0 mmol; mfd. by Aldrich), a solution of potassium tert-butoxide in 2-methyl-2-propanol (1.0 M solution, 40 µL, 40 µmol; mfd. by Aldrich) and 2-propanol (5 mL) with vigorous stirring under a hydrogen atmosphere at 8 atm at 25°C for 8 hours.

After the reaction was completed, GC analysis of the thus obtained solution showed that the product obtained was (S)-1,1-dimethoxy-2-propanol (yield 99%, enantiomer excess 98%).
GC
Column: Chirasil-DEX CB;
Column temperature: 60°C;
Temperature of injection and detection: 250°C;
Helium pressure: 44 kPa;
Retention time:
   1,1-dimethoxy-2-propanone: 10.0 min (0.3%);
   (R)-1,1-dimethoxy-2-propanol: 22.8 min (0.6%);
   (S)-1,1-dimethoxy-2-propanol: 23.6 min (99.1%).
The absolute structure was determined as S-form by comparing the specific rotation with the known specific rotation disclosed in the following reference.
Actual measurement value: [α]^{26.5}_{D}=-18.0 (c=0.16, CH₃OH) ;
Literature data of S-form (87%ee): [α]²⁵_{D}=-12.15(c=3.21, CH₃OH) ;
[α]_{D} reference: Cho, B.T.; Chun, Y.S., Tetrahedron:Asymmetry, 1994, 5, pp. 1147-1150.

### Example 20

### Asymmetric hydrogenation of 2,2-diethoxyacetophenone

Complex (R,R)-1 (3.1 mg, 2.5 µmol) was measured into a 100 mL glass autoclave equipped with a teflon-coated stirrer. An asymmetric hydrogenation according to the procedure of Example 5 was carried out using 2,2-diethoxyacetophenone (517 mg, 2.5 mmol; mfd. by TOKYO KASEI), a solution of potassium tert-butoxide in 2-methyl-2-propanol (1.0 M solution, 50 µL, 50 µmol; mfd. by Aldrich) and 2-propanol (2.5 mL) with vigorous stirring under a hydrogen atmosphere at 8 atm at 25°C for 8 hours.

After the reaction was completed, GC analysis of the thus obtained solution showed that the product obtained was (S)-2,2-diethoxy-1-phenylethanol (yield 100%, enantiomer excess 37%).
GC
Column: Chirasil-DEX CB;
Column temperature: 120°C;
Temperature of injection and detection: 250°C;
Helium pressure: 44 kPa;
Retention time:
   2,2-diethoxyacetophene: 22.7 min (0%);
   (S)-2,2-diethoxy-1-phenylethanol: 45.5 min (31.3%);
   (R)-1,1-diethoxy-2-propanol: 47.9 min (68.7%).
The absolute structure was determined as S-form by comparing the specific rotation of the hydrogenated product per se (Actual measurement value: [α]²⁷_{D}=-6.95 (c=1.995, CHCl₃)) with the specific rotation disclosed in the reference (Cho, B. T.; Chun, Y. S., Tetrahedron:Asymmetry, 1994, 5, pp. 1147-1150).

### Example 21

### Asymmetric hydrogenation of 2-[bisbenzylamino]-1-[3-[(methyl-sulfonyl)benzylamino]phenyl]ethanone (Intermediate 4)

Complex (S,S)-1 (2.6 mg, 2.12 µmol) was measured into a 100 mL glass autoclave equipped with a teflon-coated stirrer. An asymmetric hydrogenation according to the procedure of Example 5 was carried out using Intermediate 4 (1.07 g, 2.15 mmol), a solution of potassium tert-butoxide in 2-methyl-2-propanol (1.0 M solution, 172 µL, 172 µmol; mfd. by Aldrich) and 2-propanol (4.3 mL) with vigorous stirring under a hydrogen atmosphere at 8 atm, at 30°C for 14 hours.

After the reaction was completed, the thus obtained solution was concentrated under reduced pressure and compounds derived from the complex were removed by a silica gel chromatography treatment (silica gel 15 g; solvent: diethyl ether) to give (S)-2-[bisbenzylamino)-1-[3-[(methylsulfonyl)benzylamino]phenyl]ethanol (88%ee, 1.05 g, yield 97%) as a colorless oil.
¹H-NMR (300 MHz, CDCl₃) δ 2.47 (dd, 1H, J=12.9, 9.9), 2.57 (dd, 1H, J=12.9, 3.4), 2.86 (s, 3H), 3.46 (d, 2H, J=13.5), 3.89 (d, 2H, J=13.5), 4.62 (dd, 1H, J=9.9, 3.4), 4.78 (s, 2H), 7.07-7.39 (m, 19H).
The enantiomer excess was determined by means of HPLC analysis.
Chiral Column: CHIRALCEL OJ-R (4.6 mm ID× 150 mm; mfd. by Daicel);
Mobile phase: 0.5 M NaClO₄-HClO₄(pH=2.0)/acetonitrile (74/26) 1 pump;
Column temperature: 40°C;
UV wave length: 233 nm;
Flow rate: 0.5 mL/min;
Retention time:
   (R)-form: 56.1 min (5.8%);
   (S)-form: 61.9 min (94.2%);
   R_{f}=0.51 (1/1 n-hexane/ethyl acetate).

### Example 22

### Asymmetric hydrogenation of 1-[4-chloro-3-[(methylsulfonyl)benzylamino]phenyl]-2-[[2-(9H-carbazol-2-yloxy)ethyl]benzylamino]ethanone monohydrochloride (Intermediate 5 monohydrochloride salt) (s/c=10,000)

Complex (R,R)-1 (1.6 mg, 1.3 µmol) was measured into a 100 mL glass autoclave equipped with a teflon-coated stirrer. An asymmetric hydrogenation according to the procedure of Example 5 was carried out using Intermediate 5 monohydrochloride salt (8.63 g, 12.5 mmol), potassium tert-butoxide (1.71 g, 14.75 mmol; mfd. by KANTO KAGAKU) and a mixed solvent of 2-propanol (25 mL) and DMF (6.5 mL) with vigorous stirring under a hydrogen atmosphere at 8 atm at 30°C for 7 hours.

After the reaction was completed, the thus obtained solution was concentrated under reduced pressure, diluted with ethyl acetate and washed with saturated brine. The solvent was then distilled off, and compounds derived from the complex were removed from the residue by a silica gel chromatography treatment (silica gel 25 g; solvent: ethyl acetate) to give (R)-1-[4-chloro-3-[(methylsulfonyl)benzylamino]phenyl]-2-[[2-(9H-carbazol-2-yloxy)ethyl]benzylamino]ethanol (91%ee, 7.53 g, yield 92%) as a colorless amorphous solid.
¹H-NMR (300 MHz, CDCl₃) δ 2.91 (m, 2H), 3.16 (s, 3H), 3.75 (m, 2H), 4.02 (m, 2H), 4.61-4.82 (m, 4H), 5.23 (m, 1H), 6.72 (dd, 1H, J=8.5, 2.2), 6.92 (d, 1H, J=2.2), 7.07-7.44 (m, 16H), 7.93-8.01 (m, 2H), 11.07 (s, 1H).
The enantiomer excess was determined by means of HPLC analysis.
Chiral Column: CHIRALCEL OJ-R (4.6 mm ID× 150 mm; mfd. by Daicel);
Mobile phase: 0.5 M NaClO₄, aq./acetonitrile (30/70) 1 pump;
Column temperature: 40°C;
UV wave length: 233 nm;
Flow rate: 0.5 mL/min;
Retention time:
   (R)-form: 27.0 min (89.6%);
   (S)-form: 20.9 min (4.2%);
   R_{f}=0.64 (1/1 n-hexane/ethyl acetate).

### Example 23

### Asymmetric hydrogenation of 1-[3-[(methylsulfonyl)benzylamino] phenyl]-2-[[2-(9H-carbazol-2-yloxy) ethyl]benzylamino]ethanone monohydrochloride (Intermediate 6 monohydrochloride salt) (S-form)

Complex (S,S)-4 (1.49 mg, 1.25 µmol) was measured into a 100 mL glass autoclave equipped with a teflon-coated stirrer. An asymmetric hydrogenation according to the procedure of Example 5 was carried out using Intermediate 6 monohydrochloride salt (1.64 g, 2.5 mmol), potassium tert-butoxide (341.3 mg, 2.95 mmol; mfd. by KANTO KAGAKU) and a mixed solvent of 2-propanol (12.6 mL) and DMA (12.6 mL) with vigorous stirring under hydrogen atmosphere at 8 atm at 23°C for 45minutes.

After the reaction was completed, the thus obtained solution was concentrated under reduced pressure, diluted with ethyl acetate and washed with saturated brine. The solvent was then distilled off, and compounds derived from the complex were removed from the residue by a silica gel chromatography treatment (silica gel 25 g; solvent: ethyl acetate) to give (S)-1-[3-[(methylsulfonyl)benzylamino]phenyl]-2-[[2-(9H-carbazol-2-yloxy)ethyl]benzylamino]ethanol (96%ee, 1.5 g, yield 97%) as a colorless amorphous solid.
¹H -NMR (500 MHz, CDCl₃) δ 2.60 (dd, 1H, J=13.2, 10.3), 2.83 (dd, 1H, J=13.2, 2.9), 2.89 (s, 3H), 2.97-3.04 (m, 1H), 3.09-3.15 (m, 1H), 3.70 (d, 2H, J=13.7), 3.96 (d, 2H, J=13.7), 4.13 (m, 2H), 4.66 (dd, 1H, J=10.3, 3.4), 4.77 (d, 1H, J=14.7), 4.81 (d, 1H, J=14.7), 6.84 (dd, 1H, J=8.3, 2.0), 6.92 (d, 1H, J=2.0), 7.10-7.41 (m, 17H), 7.93 (d, 2H, J=8.8), 7.97 (d, 1H, J=7.8), 8.14 (s, 1H).
The enantiomer excess was determined by means of HPLC analysis.
Chiral Column: CHIRALCEL OJ-R (4.6 mm ID× 150 mm; mfd. by Daicel);
Mobile phase: 0.5 M NaClO₄ aq./acetonitrile (30/70) 1 pump;
Column temperature: 40°C;
UV wave length: 233 nm;
Flow rate: 0.5 mL/min;
Retention time:
   (S)-form: 18.6 min (97.7%);
   (R)-form: 26.3 min (2.2%);
   R_{f}=0.27 (1/1 n-hexane/ethyl acetate).

### Example 24

### (R)-2-[2-(9H-carbazol-2-yloxy)ethylamino]-1-[3-(methylsulfonylamino)phenyl]ethanol monohydrochloride

### Step 1. Asymmetric hydrogenation of 1-[3-[(methylsulfonyl)benzylamino]phenyl]-2-[[2-(9H-carbazol-2-yloxy)ethyl]benzylamino]ethanone monohydrochloride (Intermediate 6 monohydrochloride salt) (R-form)

Complex (R,R)-1 (1.6 mg, 1.25 µmol) was measured into a 100 mL glass autoclave equipped with a teflon-coated stirrer. An asymmetric hydrogenation according to the procedure of Example 5 was carried out using Intermediate 6 monohydrochloride salt (8.18 g, 12.5 mmol), potassium tert-butoxide (1.71 g, 14.75 mmol; mfd. by KANTO KAGAKU) and a mixed solvent of 2-propanol (25 mL) and DMA (6.5 mL) with vigorous stirring under a hydrogen atmosphere at 8 atm at 23°C for 4 hours.

After the reaction was completed, the thus obtained solution was concentrated under reduced pressure, diluted with ethyl acetate and washed with saturated brine. The solvent was then distilled off, and compounds derived from the complex was removed from the residue by a silica gel chromatography treatment (silica gel 25 g; solvent: ethyl acetate) to give (R)-1-[3-[(methylsulfonyl)benzylamino]phenyl]-2-[[2-(9H-carbazol-2-yloxy)ethyl]benzylamino]ethanol (91%ee, 8.65 g, yield 99%) as a colorless amorphous solid.
The enantiomer excess was determined by means of HPLC analysis.
Chiral Column: CHIRALCEL OJ-R (4.6 mm ID× 150 mm; mfd. by Daicel);
Mobile phase: 0.5 M NaClO₄ aq./acetonitrile (30/70) 1 pump;
Column temperature: 40°C;
UV wave length: 233 nm;
Flow rate: 0.5 mL/min;
Retention time:
   (S)-form: 18.2 min (3.6%);
   (R)-form: 25.0 min (94.8%);
   R_{f}=0.27 (1/1 n-hexane/ethyl acetate).

### Step. 2 Synthesis of (R)-2-[2-(9H-carbazol-2-yloxy)ethylamino]-1-[3-(methylsulfonylamino)phenyl]ethanol monohydrochloride

(R)-1-[3-[(Methylsulfonyl)benzylamino]phenyl]-2-[[2-(9H-carbazol-2-yloxy)ethyl]benzylamino]ethanol (10 g; obtained in the above step) was dissolved in a mixed solvent of tetrahydrofuran (100 mL) and methanol (100 mL), and a palladium hydroxide/carbon catalyst (1 g; mfd. by Nacalai Tesque) was added under a nitrogen atmosphere. The resulting mixture was subjected to a hydrogenolysis treatment with stirring under a hydrogen atmosphere at atmospheric pressure at room temperature through a night. The catalyst was filtered off, and the solvent was distilled off under reduced pressure. The crystal collected by suction filtration was washed with a mixed solvent of tetrahydrofuran and methanol (1:1).

The crystal was then dissolved in methanol (150 mL). After 0.1 N hydrochloric acid/ethanol was added to form hydrochloride salt, the deposited crystal was collected by suction filtration. The crystal was dried by heating (40°C) under reduced pressure to give a hydrochloride salt of the title compound (7.50 g) as a white crystal. R_{f}=0.3 (10/1 chloroform/methanol); MH⁺=441.

The thus obtained compound was shown to be identical to the title compound prepared according to the known method (WO 97/25311) by the fact that they had the same retention time in HPLC measurements. This compound is described in WO 97/25311 as being very useful for treating and preventing diabetes, obesity, hyperlipidemia and the like.

### Example 25

### Traps-RuCl₂[(R)-(2,6-dimethylanisol-4-yl)binap][(R)-damen] complex (designated as Complex (R,R)-25); abbreviated name: trans-RuCl₂[(R)-dmanylbinap][(R)-damen]; Formula (23):

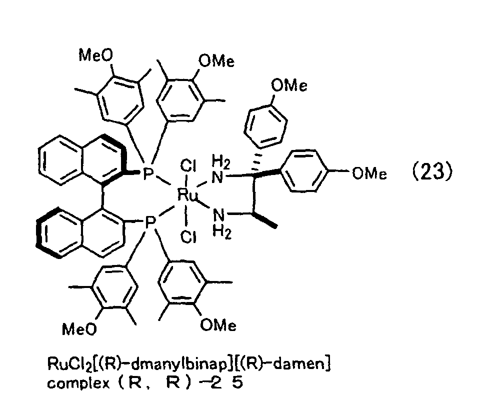

### Step 1. Synthesis of (R)-2,2'-bis[bis(3,5-dimethyl-4-methoxyphenyl)phosphino]-1,1'-binaphthyl (Intermediate 11) (Process A and Process B); abbreviated name: (R)-Dmanyl-BINAP); Formula (24):

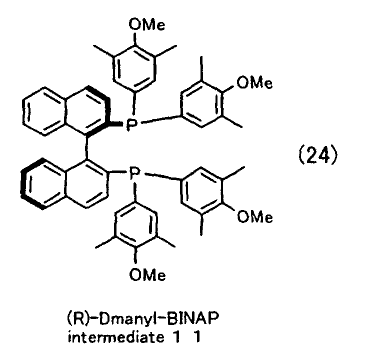

### (Process A)

5% Palladium/activated carbon (163 mg; mfd. by Aldrich) and 1,4-diazabicyclo[2.2.2]octane (415 mg, 3.53 mmol; mfd. by Aldrich) were added to a 50 mL glass Schlenk tube equipped with a teflon-coated stirrer which had been previously dried by heating under reduced pressure, and the tube was then sealed tightly. The reactor was degassed by applying the pressure-reducing/argon-introducing procedure five times. Under an argon atmosphere, dried DMF (7.5 mL) which had been previously degassed by means of vacuum freezing was added, followed by addition of bis(3,5-dimethyl-4-methoxy-phenyl)chlorophosphine (0.9 mL, ca. 3.5 mmol; synthesized by the method described in the literature: Tetrahedron:Asymmetry, Vol. 10, pp. 3341-3352 (1999)) with stirring. The procedure of reducing pressure followed by replacing the atmosphere with hydrogen at atmospheric pressure was repeated seven times. The reaction liquid was stirred under a hydrogen atmosphere at 1 atm at 100°C for 2 hours. After allowed to cool down to room temperature, the reaction liquid was filtered through dried Celite under an argon atmosphere. The filtrate was collected in a 50 mL Schlenk tube which had been previously dried by heating under reduced pressure, and then concentrated under reduced pressure. To the residue, (R)-(-)-1,1'-bi-2-naphthol bis(trifluoromethanesulfonate) (802 mg, 1.41 mmol; mfd. by FLUKA), [1,2-bis(diphenylphosphino)ethane]dichloronickel(II) (75 mg, 0.14 mmol; mfd. by Aldrich) and a solution of 1,4-diazabicyclo-[2.2.2]octane (668 mg, 5.84 mmol; mfd. by Aldrich) in dried DMF (7.5 mL) previously degassed by means of vacuum freezing were added under an argon atmosphere. The resulting mixture was stirred at 100°C for 106 hours. After cooled down to room temperature, the mixture was filtered through dried Celite under an argon atmosphere and the filtrate was concentrated under reduced pressure. The residue was extracted with ethyl acetate (22 mL) and purified water (11 mL). The organic layer was washed sequentially with 1 N hydrochloric acid (11 mL) and aqueous saturated ammonium chloride (11 mL), dried over anhydrous magnesium sulfate, and concentrated under reduced pressure. The residue was purified three times by silica gel column chromatography (20 mL, neutral spherical shape; 100/0-40/1 n-hexane/ethyl acetate) to give Intermediate 11 (334.7 mg). MS(EI)m/z 854[M]⁺.

### Process B

To a 50 mL glass Schlenk tube equipped with a teflon-coated stirrer which had been previously dried by heating under reduced pressure, 5% palladium/activated carbon (0.2 g; mfd. by Aldrich), 1,4-diazabicyclo[2.2.2]octane (1.46 g, 12.75 mmol; mfd. by Aldrich), (R)-(-)-1,1'-bi-2-naphthol bis(trifluoromethanesulfonate) (1.10 g, 1.92 mmol; mfd. by FLUKA) and [1,2-bis(diphenylphosphino)ethane]dichloronickel(II) (110 mg, 0.2 mmol; mfd. by Aldrich) were added, and the tube was then sealed tightly. The reactor was degassed by applying the pressure-reducing/argon-introducing procedure five times. Under an argon atmosphere, dried DMA (11 mL) which had been previously degassed by means of vacuum freezing was added, followed by addition of bis(3,5-dimethyl-4-methoxyphenyl)chlorophosphine (1.25 mL, ca.4.8 mmol; synthesized by the method described in the literature: Tetrahedron:Asymmetry, Vol. 10, pp. 3341-3352 (1999)) with stirring. The procedure of reducing pressure followed by replacing the atmosphere with hydrogen at atmospheric pressure was repeated seven times. The reaction liquid was stirred under a hydrogen atmosphere at 1 atm at 100°C for 110 hours. After allowed to cool down to room temperature, the reaction liquid was filtered through dried Celite under an argon atmosphere, and the filtrate was concentrated under reduced pressure. The residue was then subjected to extracting treatment, washing treatment and purifying treatment by silica gel column chromatography according to Process A to give Intermediate 11 (492.5 mg).

### Step 2. Synthesis of Complex (R,R)-25

According to the procedures of Examples 1 and 4, the title Complex (R,R)-25 (56.1 mg, yellow solid) was synthesized from [RuCl₂(benzene)]₂ (25.5 mg, 0.05 mmol), Intermediate 11 (85.5 mg, 0.10 mmol; obtained in Step 1) and (R)-DAMEN (31.5 mg, 0.11 mmol; synthesized by the method described in the literature: Burrows, C. J. et al., Tetrahedron Letters, 34(12), pp. 1905-1908 (1993)). MS(FAB)m/z 1404[MH]⁺.

### Example 26

### Asymmetric hydrogenation of 1-[3-[(methylsulfonyl)benzylamino]-phenyl]-2-[[2-(9H-carbazol-2-yloxy)ethyl]benzylamino]ethanone monohydrochloride (Intermediate 6 monohydrochloride salt) (R-form)

Complex (R,R)-25 (1.76 mg, 1.25 µmol; synthesized in Example 25) was measured into a 100 mL glass autoclave equipped with a teflon-coated stirrer. An asymmetric hydrogenation according to the procedure of Example 5 was carried out using Intermediate 6 monohydrochloride salt (1.64 g, 2.5 mmol), potassium tert-butoxide (350 mg, 3 mmol; mfd. by KANTO KAGAKU) and a mixed solvent of 2-propanol (5 mL) and DMA (1.3 mL) under a hydrogen atmosphere at 8 atm at 23°C for 4 hours with vigorous stirring. After the reaction was completed, the thus obtained solution was concentrated under reduced pressure, diluted with ethyl acetate and washed with saturated brine. The solvent was then distilled off, and compounds derived from the complex were removed from the residue by a silica gel chromatography treatment (silica gel 10 g; solvent: ethyl acetate) to give (R)-1-[3-[(methylsulfonyl)benzylamino]phenyl]-2-[[2-(9H-carbazol-2-yloxy)ethyl]benzylamino]ethanol (96%ee, 1.53 g, yield 99%) as a colorless amorphous solid. The enantiomer excess was determined by means of HPLC analysis.
Chiral Column: CHIRALCEL OJ-R (4.6 mm ID× 150 mm; mfd. by Daicel);
Mobile phase: 0.5 M NaClO₄ aq./acetonitrile (30/70) 1 pump;
Column temperature: 40°C;
UV wave length: 233 nm;
Flow rate: 0.5 mL/min;
Retention time:
   (S)-form: 18.2 min (1.9%);
   (R)-form: 25.0 min (97.9%);
   R_{f}=0.27 (1/1 n-hexane/ethyl acetate).

According to the hydrogenolysis procedure and hydrochloride salt-forming procedure in Step 2 of Example 24, the above compound can be converted into (R)-2-[2-(9H-carbazol-2-yloxy)ethylamino]-1-[3-(methylsulfonylamino)phenyl]ethanol monohydrochloride which is described in WO 97/25311 as being very useful for treating and preventing diabetes, obesity, hyperlipidemia and the like.

### Example 27

### Trans-RuCl₂[(R]-xyl-6,6'-dimethylbinap][(R)-damen] complex (designated as Complex (R,R)-27); abbreviated name: trans-RuCl₂[(R)-xyl-6mebinap][(R)-damen]; Formula (25):

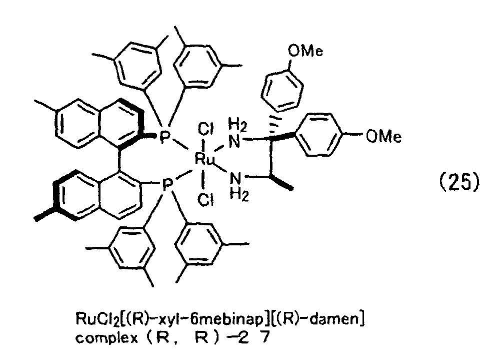

### Step 1. Synthesis of (R)-6,6'-dimethyl-1,1'-bi-2-naphthol bis(trifluoromethanesulfonate) (Intermediate 12); Formula (26):

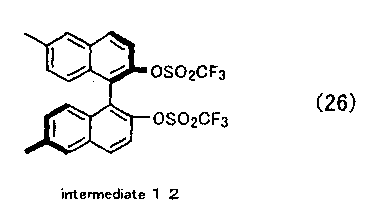

Under an argon atmosphere, (R)-6,6'-dimethyl-1,1'-bi-2-naphthol (9.432 g, 30 mmol; mfd. by KANKYO KAGAKU CENTER) was placed in a 300 mL recovery flask equipped with a teflon-coated stirrer, and then dissolved with dehydrated dichloromethane (60 mL). Under ice-cooling and stirring, dehydrated pyridine (7.2 mL, 90 mmol) was added, followed by trifluoromethanesulfonic anhydride (20 g, 70 mmol; mfd. by TOKYO KASEI). The resulting mixture was stirred with ice-cooling for 30 minutes, and then further stirred for 17 hours while its temperature was allowed to warm to room temperature. n-Hexane (60 mL) was added with vigorous stirring. The precipitate generated was removed by filtration with silica gel pad (50 g) and the silica gel pad was washed with a mixed solvent of n-hexane/dichloromethane (1/1; 200 mL). The solvent was distilled off from the combined filtrates under reduced pressure. The residue was dried under reduced pressure with vacuum pump (ca. 0.5 mmHg) for 3 hours to give Intermediate 12 (17.18 g; colorless amorphous solid).
MS(EI)m/z 578[M]⁺.

### Step 2. Synthesis of (R)-(+)-2,2'-bis[bis(3,5-dimethylphenyl)phosphino]-6,6'-dimethyl-1,1'-binaphthyl (Intermediate 13; abbreviated name: (R)-Xyl-6MeBINAP); Formula (27)

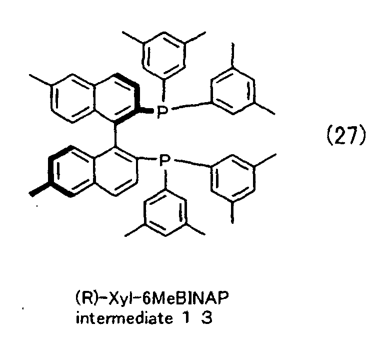

To a 50 mL glass Schlenk tube equipped with a teflon-coated stirrer which had been previously dried by heating under reduced pressure, 5% palladium/activated carbon (0.2 g; mfd. by Aldrich), 1,4-diazabicyclo[2.2.2]octane (1.49 g, 13 mmol; mfd. by Aldrich), Intermediate 12 (1.14 g, 1.96 mmol) and [1,2-bis(diphenylphosphino)ethane)dichloronickel(II) (0.1 g, 0.2 mmol; mfd. by Aldrich) were added, and the tube was then sealed tightly. The reactor was degassed by applying the pressure-reducing/argon-introducing procedure five times. Under an argon atmosphere, dried DMF (10 mL) which had been previously degassed by means of vacuum freezing was added, followed by addition of bis(3,5-dimethylphenyl)chlorophosphine (1.14 mL, ca.4.9 mmol; mfd. by Digital Specialty Chemicals, Inc.) with stirring. The procedure of reducing pressure followed by replacing the atmosphere with hydrogen at atmospheric pressure was repeated seven times. The reaction liquid was stirred under a hydrogen atmosphere at 1 atm at 100°C for 110 hours. After allowed to cool down to room temperature, the reaction liquid was filtered through dried Celite under an argon atmosphere. The filtrate was collected in a 50 mL Schlenk tube which had been previously dried by heating under reduced pressure, and then concentrated under reduced pressure. The residue was extracted with ethyl acetate (30 mL) and purified water (15 mL). The organic layer was washed sequentially with 1 N hydrochloric acid (15 mL) and aqueous saturated ammonium chloride (15 mL), dried over anhydrous magnesium sulfate, and concentrated under reduced pressure. The residue was purified three times by silica gel column chromatography (20 mL, neutral; 100/0 to 50/1 n-hexane/ethyl acetate) to give Intermediate 13 (448.6 mg). MS(EI)m/z 762[M]⁺.

### Step 3. Synthesis of Complex (R,R)-27

According to the procedures of Examples 1 and 4, the title Complex (R,R)-27 (52.0 mg, yellow solid) was synthesized from [RuCl₂(benzene)]₂ (25.5 mg, 0.05 mmol), Intermediate 13 (76.3 mg, 0.10 mmol) and (R)-DAMEN (31.5 mg, 0.11 mmol; synthesized by the method described in the literature: Burrows, C. J., et al., Tetrahedron Letters, 34(12), pp. 1905-1908 (1993)).
MS(FAB)m/z 1312[MH]⁺.

### Example 28

### Asymmetric hydrogenation of 1-[3-[(methylsulfonyl)benzylamino]- phenyl]-2-[[2-(9H-carbazol-2-yloxy)ethyl]benzylamino]ethanone monohydrochloride (Intermediate 6 monohydrochloride salt) (R-form)

Complex (R,R)-27 (1.64 mg, 1.25 µmol; synthesized in Example 27) was measured into a 100 mL glass autoclave equipped with a teflon-coated stirrer. An asymmetric hydrogenation according to the procedure of Example 5 was carried out using Intermediate 6 monohydrochloride salt (1.64 g, 2.5 mmol), potassium tert-butoxide (350 mg, 3 mmol; mfd. by KANTO KAGAKU) and a mixed solvent of 2-propanol (5 mL) and DMA (1.3 mL) under a hydrogen atmosphere at 8 atm at 25°C for 4 hours with vigorous stirring. After the reaction was completed, the thus obtained solution was concentrated under reduced pressure, diluted with ethyl acetate and washed with saturated brine. The solvent was then distilled off, and compounds derived from the complex were removed from the residue by a silica gel chromatography treatment (silica gel 10 g; solvent: ethyl acetate) to give (R)-1-[3-[(methylsulfonyl)benzyl-amino]phenyl]-2-[[2-(9H-carbazol-2-yloxy)ethyl]benzylamino]ethanol (96%ee, 1.53 g, yield 99%) as a colorless amorphous solid. The enantiomer excess was determined by means of HPLC analysis.
Chiral Column: CHIRALCEL OJ-R (4.6 mm ID× 150 mm; mfd. by Daicel);
Mobile phase: 0.5 M NaClO₄ aq./acetonitrile (30/70) 1 pump;
Column temperature: 40°C;
UV wave length: 233 nm;
Flow rate: 0.5 mL/min;
Retention time:
   (S)-form: 18.2 min (1.9%);
   (R)-form: 25.0 min (97.9%);
   R_{f}=0.27 (1/1 n-hexane/ethyl acetate).

According to the hydrogenolysis procedure and hydrochloride salt-forming procedure in Step 2 of Example 24, the above compound can be converted into (R)-2-[2-(9H-carbazol-2-yloxy)ethylamino]-1-[3-(methylsulfonylamino)phenyl]ethanol monohydrochloride which is described in WO 97/25311 as being very useful for treating and preventing diabetes, obesity, hyperlipidemia and the like.

### Reference Example 1

### 2-bromo-l-[3-[N,N-benzyl(methylsulfonyl)amino]phenyl]ethanone (Intermediate 1)

### Step 1. Synthesis of 1-[3-[N,N-benzyl(methylsulfonyl)amino]-phenyl]ethanone

1-[3-[N-(methylsulfonyl)amino]phenyl]ethanone (90.68 g; synthesized by the method described in Larsen, A. A., J. Med. Chem., 9, pp. 88-97 (1966)) was dissolved in acetone (639.6 mL) at 23°C, followed by addition of anhydrous potassium carbonate (66.0 g). Under stirring, benzyl bromide (56.6 mL; mfd. by Wako Pure Chemical Industries) was added at a stretch. Sodium iodide (13.01 g; mfd. by Wako Pure Chemical Industries) was added and the resulting mixture was vigorously stirred under heating to reflux. After 2 hours, anhydrous potassium carbonate (29.4 g) and sodium iodide (32 g) were further added and stirring was continued. After 1 hour, anhydrous potassium carbonate (29.4 g) and sodium iodide (32 g) were further added and vigorous stirring was continued at the same temperature for 13.5 hours. After allowing to cool by itself to 43°C, purified water (1090 mL) was added at a stretch and vigorously stirred. The resulting reaction liquid was extracted with ethyl acetate (495 mL). The separated organic layer was washed with saturated brine (495 mL) and dried over anhydrous magnesium sulfate (90 g) for 0.5 hour. The desiccating agent was filtered off and the solvent was then distilled off under reduced pressure.

The thus obtained crude product (139.42 g) was suspended in ethyl acetate (343 mL), then n-hexane (445 mL) was added under heating to reflux. The crude product was completely dissolved by further adding ethyl acetate (20 mL) under heating to reflux. This was recrystalized under ice-cooling and stirring to give 1-[3-[N,N-benzyl(methylsulfonyl)amino]phenyl]ethanone (71.71 g).
¹H-NMR (300 MHz, CDCl₃) δ 2.55 (s, 3H), 2.97 (s, 3H), 4.88 (s, 2H), 7.20-7.30 (m, 5H), 7.41 (m, 1H), 7.46 (dt, 1H, J=8.2, 1.9), 7.81-7.86 (m, 2H);
R_{f}=0.44 (1/1 ethyl acetate/n-hexane).

### Step 2. Synthesis of 2-bromo-1-[3-[N,N-benzyl(methylsulfonyl)amino]phenyl]ethanone (Intermediate 1)

1-[3-[N,N-benzyl(methylsulfonyl)amino]phenyl]ethanone (71.21 g; synthesized in Step 1 of Reference Example 1) was dissolved in methanol (3.94 L) and tetra-n-butylammonium tribromide (127g; mfd. by Aldrich) was added at a stretch. After stirring at room temperature for 13 hours, a mixed solution of 47% hydrobromic acid (985 mL) and purified water (985 mL) was added at a stretch. The reaction mixture was stirred for 1 hour while the internal temperature was maintained at from 35 to 40°C. Purified water (985 mL) was added, and the internal temperature was gradually lowered to 30°C with vigorous stirring over 3 hours. The reaction mixture was further cooled to 5°C and stirred for 2 hours. The precipitated crystal was collected by filtration and washed twice with a mixed liquid (2.5 L) of methanol and water. The wet crystal was dried at 50°C under reduced pressure for 24 hours to give the title compound (Intermediate 1; 73.80 g).
¹H-NMR (300 MHz, CDCl₃) δ 2.98 (s, 3H), 4.36 (s, 2H), 4.89 (s, 2H), 7.20-7.30 (m, 5H), 7.44 (t, 1H, J=8.0), 7.51 (dt, 1H, J=8.0, 1.7), 7.84-7.89 (m, 2H);
R_{f}=0.46 (1/1 ethyl acetate/n-hexane).

### Reference Example 2

### 2-bromo-1-[4-chloro-3-[N,N-benzyl(methylsulfonyl)amino]phenyl]-ethanone (Intermediate 2)

### Step. 1 Synthesis of 1-[4-chloro-3-[N,N-benzyl(methylsulfonyl)amino]phenyl]ethanone

1-[4-chloro-3-[N-(methylsulfonyl)amino]phenyl]ethanone (90.68 g; synthesized by the method described in WO 97/25311) was dissolved in DMF (50 mL) at 23°C, and anhydrous potassium carbonate (31.7 g) was then added. Under stirring, benzyl bromide (21.6 g; mfd. by Wako Pure Chemical Industries) was added at a stretch, and the resulting mixture was vigorously stirred at the same temperature for 2.5 days. Purified water (200 mL) was added at a streach. The resulting mixture was vigorously stirred and then extracted with ethyl acetate (200 mL) and n-heptane (50 mL). The separated organic layer was washed twice with purified water (100 mL), washed with saturated brine (200 mL), and dried over anhydrous sodium sulfate. The desiccating agent was filtered off and the solvent was then distilled off under reduced pressure.

The thus obtained crude product was purified by silica gel chromatography (1.5 kg) to give the title compound (27.63 g) as a light yellow oil from the fraction eluted with ethyl acetate/n-hexane (1/4 to 3/7).
¹H-NMR (300 MHz, CDCI₃) δ 2.40 (s, 3H), 3.08 (s, 3H), 4.58 (br, 1H), 5.09 (br, 1H), 7.22-7.32 (m, 5H), 7.53 (d, 1H, J=8.2), 7.56 (d, 1H, J=2.2), 7.82 (dd, 1H, J=8.2,2.2);
R_{f}=0.43 (1/1 ethyl acetate/n-hexane).

### Step 2. Synthesis of 2-bromo-1-[4-chloro-3-[N,N-benzyl(methanesulfonyl)amino]phenyl]ethanone (Intermediate 2)

1-[4-chloro-3-[N,N-benzyl(methylsulfonyl)amino]phenyl]ethanone (58.31 g; synthesized in Step 1 of Reference Example 2) was dissolved in 1,4-dioxane (583.1 mL), and tetra-n-butylammonium tribromide (91.55 g; mfd. by TOKYO KASEI) was added at a stretch. After stirring at room temperature for 15 hours, the reaction liquid was concentrated under reduced pressure. The residue was purified twice by silica gel chromatography (1 kg) to give the title compound (Intermediate 2; 54.26 g) from the fraction eluted with ethyl acetate/n-hexane (1/5 to 1/2).
¹H-NMR (300 MHz, CDCl₃) δ 3.09 (s, 3H), 4.21 (br-s, 2H), 4.60 (br, 1H), 5.11 (br, 1H), 7.22-7.32 (m, 5H), 7.57 (d, 1H, J=8.5), 7.61 (d, 1H, J=2.2), 7.87 (dd, 1H, J=8.5, 2.2);
R_{f}=0.44 (1/1 ethyl acetate/n-hexane).

### Reference Example 3

### N,N-benzyl[2-(9H-carbazol-2-yloxy)ethyl]amine (Intermediate 3)

Benzaldehyde (93.8 g; mfd. by Wako Pure Chemical Industries) was added to a solution of N-[2-(9H-carbazol-2-yloxy)ethyl]amine (200 g; synthesized by the method described in JP-A-9-249623) in methanol (5 L), and the resulting mixture was stirred at room temperature for 1 hour. Platinum oxide (10.0 g; mfd. by Wako Pure Chemical Industries) was added under an argon atmosphere, and the resulting mixture was stirred under a hydrogen atmosphere at 1 atm for 3 hours. After the atmosphere in the reaction system is replaced with argon, benzaldehyde (18.8 g) was further added. The reaction mixture was further stirred under a hydrogen atmosphere at 1 atm for 3 hours. The atmosphere in the reaction system was purged with argon, and the catalyst was then filtered off. The solvent was distilled off from the filtrate under reduced pressure. The residue was recrystalized from ethanol. The crystal collected by filtration was dried under reduced pressure to give the title compound (Intermediate 3; 252.0 g) as a slightly yellowish crystal.
R_{f}=0.60 (10/1 chloroform/methanol); MH⁺=317;
¹H-NMR (300 MHz, DMSO-d₆; free-form) δ 2.30 (s, 1H), 2.91 (t, 2H, J=5.8), 3.79 (s, 2H), 4.11 (t, 2H, J=5.8), 6.77 (dd, 1H, J=8.5, 2.2), 6.96 (d, 1H, J=2.2), 7.10 (m, 1H), 7.20-7.44 (m, 7H), 7.92-8.00 (m, 2H), 11.09 (s, 1H).

### Reference Example 4

### 2-[bisbenzylamino]-1-[3-[(benzyl)(methylsulfonyl)amino]phenyl]ethanone monohydrochloride (Intermediate 4 monohydrochloride salt)

Dibenzylamine (42.4 g; mfd. by TOKYO KASEI) was dissolved in THF (400 mL). Under stirring, Intermediate 1 (40g) was added at a stretch at room temperature. After stirring for 3 hours, the precipitated dibenzylamine hydrobromide was filtered off and the solvent was distilled off under reduced pressure. The residue was dissolved in THF (200 mL), and then converted into its hydrochloride salt by adding 36% hydrochloric acid (9.9 mL) with stirring. The precipitated hydrochloride salt was collected by filtration, washed with THF (70 mL) and dried at 40°C under reduced pressure to give the first crystal. In addition, the filtrate was concentrated and redissolved in THF (60 mL). The precipitated hydrochloride salt was collected by filtration, washed with THF (50 mL) and dried at 40°C under reduced pressure to give the second crystal. The first crystal and the second crystal were combined to give the title compound (Intermediate 4; 53.67 g).
¹H-NMR (300 MHz, DMSO-d₆; hydrochloride salt) δ 3.13 (s, 3H), 4.25-4.60 (m, 4H), 4.85 (br, 2H), 4.93 (s, 2H), 7.16-7.72 (m, 18H), 7.81 (s, 1H), 10.62 (br, 1H);
R_{f}=0.36 (1/2 ethyl acetate/n-hexane).

### Reference Example 5

### 1-[4-chloro-3-[(benzyl)(methylsulfonyl)amino]phenyl]-2-[[2-(9H-carbazol-2-yloxy)ethyl]benzylamino]ethanone monohydrochloride (Intermediate 5 monohydrochloride salt)

Intermediate 2 (34.17 g) and Intermediate 3 (54.48 g) were reacted according to the process for synthesizing Intermediate 4 in Reference Example 4. The reaction product was purified by silica gel column chromatography (eluted with ethyl acetate/n-hexane (1/4 to 3/7)), solidified as a hydrochloride salt, triturated with 2-propanol, and then dried under reduced pressure to give the title compound (Intermediate 5; 17.64 g).
¹H-NMR (300 MHz, DMSO-d₆; hydrochloride salt) δ 3.27 (s, 3H), 3.72 (m, 2H), 4.49-4.72 (m, 4H), 4.93 (m, 2H), 5.16 (br, 2H), 6.55 (d, 1H, J=8.0), 6.97 (d, 1H, J=1.9), 7.11 (t, 1H, J=7.7), 7.18-7.35 (m, 6H), 7.39-7.58 (m, 4H), 7.65-7.78 (m, 3H), 7.83 (d, 1H, J=8.5), 7.89 (d, 1H, J=8.5), 7.98 (d, 1H, J=7.7), 8.08 (s, 1H), 11.01 (br, 1H), 11.27 (s, 1H);
R_{f}=0.51 (1/1 ethyl acetate/n-hexane).

### Reference Example 6

### 1-[3-[(benzyl)(methylsulfonyl)amino]phenyl]-2-[[2-(9H-carbazol- 2-yloxy)ethyl]benzylamino]ethanone monohydrochloride (Intermediate 6 monohydrochloride salt)

Intermediate 1 (151.34 g) and Intermediate 3 (263.05 g) were reacted according to the process for synthesizing Intermediate 4 in Reference Example 4. The reaction product was solidified as a hydrochloride salt (222.58 g), triturated with 2-propanol (2L+1L), and then dried at 40°C under reduced pressure to give a crude solid (187.94 g). This was further triturated with methanol (710 mL), collected by filtration, dried at 40°C under reduced pressure, triturated again with methanol (600 mL), collected by filtration, and then dried at 40°C under reduced pressure for 12 hours to give the title compound (Intermediate 6; 159.29 g).
¹H-NMR (300 MHz, DMSO-d₆; hydrochloride salt) δ 3.12 (s, 3H), 3.72 (br, 2H), 4.46-4.68 (m, 4H), 4.95 (s, 2H), 5.12 (br, 2H), 6.52 (d, 1H, J=7.7), 6.95 (d, 1H, J=1.9), 7.11 (t, 1H, J=7.7), 7.16-7.34 (m, 6H), 7.40-7.50 (m, 4H), 7.54 (t, 1H, J=8.2), 7.66-7.78 (m, 3H), 7.83 (d, 1H, J=7.7), 7.85 (d, 1H, J=8.5), 7.95-8.02 (m, 2H), 10.79 (br, 1H), 11.23 (br, 1H);
R_{f}=0.43 (1/1 ethyl acetate/n-hexane).

### Reference Example 7

### 2-dimethylaminoacetophenone monohydrobromide (Intermediate 7 mono-HBr salt)

Phenacyl bromide (25 g, 126 mmol; mfd. by TOKYO KASEI) and diethyl ether (251 mL) were placed in a 500 mL recovery flask equipped with a teflon-coated stirrer. The flask was placed on an ice bath. An aqueous 50% dimethylamine solution (113 g, 1.26 mol; mfd. by Nacalai Tesque) was added dropwise with vigorous stirring, followed by stirring at 0°C for 8 hours. The reaction mixture was concentrated under reduced pressure. Water contained in the thus obtained residue was removed by azeotrope with toluene. The orange powder obtained by recrystallization from ethanol was then washed with n-hexane to give the title compound (Intermediate 7 mono-HBr salt; 19.2 g, 78.6 mmol, yield 63%) as a white solid.
¹H-NMR (400 MHz, DMSO-d₆) δ 2.93 (s, 6H), 5.15 (s, 2H), 7.63 (t, 2H, J=7.6), 7.77 (t, 1H, J=7.6), 8.00 (d, 2H, J=7.2), 9.88 (s, 1H).

### Reference Example 8

### Synthesis of (methyl) (phenyl)aminoacetone (Intermediate 8)

DMF (31 mL), bromoacetone (4.25 g, 31 mmol; mfd. by TOKYO KASEI), N-methylaniline (4.93 g, 46 mmol; mfd. by TOKYO KASEI) and propylene oxide (22 mL, 314 mmol; mfd. by TOKYO KASEI) were added to a 100 mL recovery flask equipped with a teflon-coated stirrer and a reflux column. The resulting mixture was stirred at 50°C for 15 hours. After allowed to cool by itself, the mixture was extracted with ethyl acetate and water. The organic layer was washed with saturated brine, dried over anhydrous sodium sulfate, filtered, and then concentrated under reduced pressure. The residue was subjected to a silica gel chromatography treatment (silica gel, 450 g; n-hexane/ethyl acetate (5/1)) to give the title compound (Intermediate 8; 2.43 g, 14.9 mmol, yield 48%) as the second fraction.
¹H-NMR (400 MHz, CDCl₃) δ 2.13 (s, 3H), 3.06 (s, 3H), 4.01 (s, 2H), 6.62 (dd, 2H, J=8.8, 0.8), 6.72-6.77 (m, 1H), 7.19-7.28 (m, 2H).

### Reference Example 9

### Synthesis of benzyloxyacetone (Intermediate 9)

THF (30 mL) and sodium hydride (60% oil suspension; 4.8 g, 120 mmol; mfd. by Nacalai Tesque) were placed in a 100 mL Schlenk type reaction tube equipped with a teflon-coated stirrer. The tube was placed on an ice bath. Benzyl alcohol (13.0 g, 120 mmol; mfd. by Nacalai Tesque) diluted with THF (10 mL) was added dropwise with a syringe. The ice bath was removed and the reaction mixture was allowed to warm to room temperature, followed by stirring for 1 hour. The tube was placed on an ice bath and ethyl 4-chloro-acetoacetate (9.88 g, 60 mmol) diluted with THF (10 mL) was added dropwise with a syringe. After removing the ice bath, the reaction mixture was stirred at 25°C for 12 hours, and then extracted with ethyl acetate and 5% hydrochloric acid. The organic layer was washed with saturated brine, dried over anhydrous sodium sulfate, filtered, and then concentrated under reduced pressure. The residue was subjected to a silica gel chromatography treatment (silica gel, 1.0 kg; hexane/ethyl acetate (5/1)) to give ethyl 4-benzyloxyacetoacetate (13.2 g, 56.0 mmol, yield 93%) as the first fraction.
¹H-NMR (400 MHz, CDCl₃) δ 1.24 (t, 3H, J=6.8), 3.54 (s, 2H), 4.11-4.20 (m, 4H), 4.59 (s, 2H), 7.31-7.37 (m, 5H).

The above obtained ethyl 4-benzyloxyacetoacetate (3.0 g, 12.7 mmol), distilled water (25 mL), ethanol (20 mL) and p-toluene-sulfonic acid (483 mg, 2.54 mmol; mfd. by KANTOU KAGAKU) were placed in a 100 mL recovery flask equipped with a teflon-coated stirrer. The flask was placed on an oil bath and the resulting mixture was refluxed for 24 hours. The oil bath was removed and the mixture was extracted with ethyl acetate and an aqueous saturated sodium hydrogencarbonate solution. The organic layer was washed with saturated brine, dried over anhydrous sodium sulfate, filtered, and then concentrated under reduced pressure. The residue was subjected to an abbreviated distillation treatment to give benzyloxyacetone (Intermediate 9; 1.59 g, 9.68 mmol, yield 76%).
¹H-NMR (400 MHz, CDCl₃) δ 2.17 (s, 3H), 4.06 (s, 2H), 4.60 (s, 2H), 7.31-7.37 (m, 5H);
¹³C-NMR (100 MHz, CDCl₃) δ 26.42, 73.31, 75.26, 76.68, 127.88, 128.02, 128.52, 137.13, 206.70.

### Reference Example 10

### Synthesis of 2-(tert-butyldiphenylsilyl)oxyacetophenone (Intermediate 10)

2-Hydroxyacetophenone (4.67 g, 34.3 mmol; mfd. by TOKYO KASEI), imidazole (3.50 g, 51.5 mmol; mfd. by Nacalai Tesque) and dichloromethane (20 mL) were placed in a 50 mL recovery flask equipped with a teflon-coated stirrer. The flask was placed on an ice bath. Tert-butyldiphenylchlorosilane (11.3 g, 41.2 mmol; mfd. by TOKYO KASEI) was added dropwise under an argon stream. The ice bath was removed and the reaction mixture was stirred at 25°C for 12 hours. The organic layer was washed with 5% hydrochloric acid and saturated brine, dried over anhydrous sodium sulfate, filtered, and then concentrated under reduced pressure. The thus obtained yellow solid was subjected to a silica gel chromatography treatment (silica gel, 700g; hexane/diethyl ether (19/1)) to give the title compound (Intermediate 10; 9.73 g, 26.0 mmol, yield 76%) as the second fraction.
¹H-NMR (270 MHz, CDCl₃) δ 1.07 (s, 9H), 4.91 (s, 2H), 7.35-7.53 (m, 9H), 7.69-7.84 (m, 6H).

All the publications, patents and patent applications cited in this specification are incorporated herein in their entities by reference.

### Industrial Utility

The present invention provides a novel and practically useful process for the preparation of optically active secondary alcohols having a nitrogenous or oxygenic functional group which comprises asymmetrically hydrogenating a ketone compound having a nitrogenous or oxygenic functional group at any of the α-, β- and γ-positions with a ruthenium/optically active bidentate phosphine/diamine complex as a catalyst either in the presence of hydrogen or in the presence of hydrogen and a base with good selectivity for the functional groups, good enantioselectivity and high efficiency.

Optically active secondary alcohols containing an amino group, optically active diols and optically active hydroxyaldehydes which are very useful in a variety of applications, for example, as an intermediate for producing medicaments and pesticides or as medicaments and pesticides per se, can be produced by sequentially or simultaneously removing the nitrogen- and/or oxygen-protecting groups of the optically active secondary alcohols obtained by the process of the present invention.

## Claims

1. A process for the preparation of an optically active secondary alcohol represented by the general formula (3) having a nitrogenous or oxygenic functional group at any of the α-, β-and γ-positions: 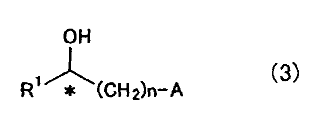 wherein
n is an integer of from 0 o 2;
R¹ represents:
(a) a straight-chain lower alkyl group,
(b) an optionally substituted monocyclic aromatic hydrocarbon ring group,
(c) an optionally substituted fused bicyclic aromatic hydrocarbon ring group,
(d) an optionally substituted fused tricyclic aromatic hydrocarbon ring group,
(e) an optionally substituted monocyclic heteroaromatic ring group,
(f) an optionally substituted fused bicyclic heteroaromatic ring group, or
(g) an optionally substituted fused tricyclic heteroaromatic ring group;
A represents:
(a) CH₂NR²R³,
(b) CH₂OR⁴, or
(c) CH(OR¹⁵)₂;
R² represents:
(a) an acyl or alkyloxycarbonyl group,
(b) an optionally substituted straight-chain alkyl group,
(c) an optionally substituted branched-chain alkyl group,
(d) an optionally substituted cyclic alkyl group,
(e) an optionally substituted alkenyl group,
(f) an optionally substituted aralkyl group,
(g) an optionally substituted aryl group,
(h) a heteroatom-containing saturated carbon chain group,
(i) a heteroatom-containing unsaturated carbon chain group,
(j) an optionally substituted heteromonocyclic group,
(k) an optionally substituted heteropolycyclic group, or
(l) a composite group consisting of members suitably selected from any of (b) to (k);
when R² is (a) an acyl or alkyloxycarbonyl group among the above groups, R³ represents:
(a) hydrogen,
(b) an optionally substituted straight-chain alkyl group,
(c) an optionally substituted branched-chain alkyl group,
(d) an optionally substituted cyclic alkyl group,
(e) an optionally substituted alkenyl group,
(f) an optionally substituted aralkyl group,
(g) an optionally substituted aryl group,
(h) a heteroatom-containing saturated carbon chain group,
(i) a heteroatom-containing unsaturated carbon chain group,
(j) an optionally substituted heteromonocyclic group,
(k) an optionally substituted heteropolycyclic group, or
(l) a composite group consisting of members suitably selected from any of (b) to (k);
alternatively, when R² is any group other than (a) an acyl or alkyloxycarbonyl group among the above groups, R³ represents:
(a) an optionally substituted straight-chain alkyl group,
(b) an optionally substituted branched-chain alkyl group,
(c) an optionally substituted cyclic alkyl group,
(d) an optionally substituted alkenyl group,
(e) an optionally substituted aralkyl group,
(f) an optionally substituted aryl group,
(g) a heteroatom-containing saturated carbon chain group,
(h) a heteroatom-containing unsaturated carbon chain group,
(i) an optionally substituted heteromonocyclic group,
(j) an optionally substituted heteropolycyclic group, or
(k) a composite group consisting of members suitably selected from any of (a) to (j); and R² and R³ may be linked to each other to form a heterocyclic group;
R⁴ represents:
(a) an optionally substituted straight-chain alkyl group,
(b) an optionally substituted branched-chain alkyl group,
(c) an optionally substituted cyclic alkyl group,
(d) an optionally substituted benzyl group,
(e) an optionally substituted aralkyl group,
(f) an optionally substituted aryl group,
(g) a heteroatom-containing saturated carbon chain group,
(h) a heteroatom-containing unsaturated carbon chain group,
(i) an optionally substituted heteromonocyclic group
(j) an optionally substituted heteropolycyclic group,
(k) a composite group consisting of members suitably selected from any of (a) to (j), or
(l) an organosilicon group represented by SiR⁵R⁶R⁷;
R⁵, R⁶ and R⁷ each independently represent:
(a) a straight-chain or branched-chain lower alkyl group, or
(b) a phenyl group;
R¹⁵ represents:
(a) a straight-chain lower alkyl group,
(b) a branched-chain lower alkyl group,
(c) a cyclic lower alkyl group,
(d) an optionally substituted phenyl group,
(e) an optionally substituted benzyl group, or
(f) alternatively, two R¹⁵ are bonded to each other to form a cyclic ketal group; and
* represents an asymmetric carbon atom,
which comprises asymmetrically hydrogenating a ketone compound represented by the general formula (1) having a nitrogenous or oxygenic functional group at any of the α-, β- and γ-positions: 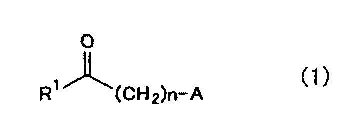 wherein R¹, A and n are as defined above, or a mineral or organic acid salt thereof, with a catalyst in the presence of hydrogen or in the presence of hydrogen and a base, the catalyst being a ruthenium/optically active bidentate phosphine/diamine complex represented by the general formula (2): 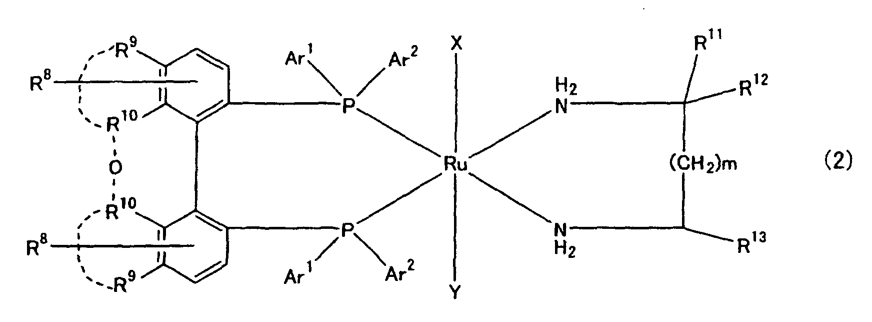 wherein
m is an integer of from 0 to 2;
X and Y may be covalently or ionically bonded to the ruthenium metal, and they independently represent:
(a) hydrogen,
(b) halogen,
(c) an alkoxy group,
(d) a carboxyl group, or
(e) other anion radical;
Ar¹ and Ar² independently represent a phenyl group substituted with from zero to five substituents selected from straight-chain or branched-chain lower alkyl group, halogen or lower alkoxy group;
R⁸ represents:
(a) hydrogen,
(b) a lower alkyl group,
(c) a lower alkoxy group, or
(d) N(R¹⁴)₂ wherein R¹⁴ represents a lower alkyl group;
R⁹ represents:
(a) hydrogen,
(b) a lower alkyl group, or
(c) a lower alkoxy group;
R¹⁰ represents:
(a) a lower alkyl group, or
(b) a lower alkoxy group;
the dashed line linking one R¹⁰ to the other R¹⁰ means that one R¹⁰ may be bonded to the other R¹⁰ via an oxygen atom;
one dashed line linking R⁹ to R¹⁰, and the other dashed line linking R⁹ to R¹⁰ independently mean that each pair of R⁹ and R¹⁰ taken together with the benzene ring to which they are attached may form a ring selected from the following rings:
(a) an optionally substituted tetralin ring,
(b) an optionally substituted naphthalene ring, and
(c) an optionally substituted 1,3-benzodioxole ring;
R¹¹ represents:
(a) hydrogen,
(b) a straight-chain lower alkyl group,
(c) a branched-chain lower alkyl group,
(d) a cyclic lower alkyl group,
(e) a phenyl group substituted with from zero to five substituents selected from lower alkyl groups or lower alkoxy groups,
(f) a 1-naphthyl group substituted with from zero to seven substituents selected from lower alkyl groups or lower alkoxy groups, or
(g) a 2-naphthyl group substituted with from zero to seven substituents selected from lower alkyl groups or lower alkoxy groups;
provided that (I) when R¹¹ is hydrogen, then R¹² and R¹³ are independently represent:
(a) hydrogen,
(b) a straight-chain lower alkyl group,
(c) a branched-chain lower alkyl group,
(d) a cyclic lower alkyl group,
(e) a phenyl group substituted with from zero to five substituents selected from lower alkyl groups or lower alkoxy groups,
(f) a 1-naphthyl group substituted with from zero to seven substituents selected from lower alkyl groups or lower alkoxy groups,
(g) a 2-naphthyl group substituted with from zero to seven substituents selected from lower alkyl groups or lower alkoxy groups, or
(h) alternatively, R¹² and R¹³ may be bonded to each other to form a ring selected from:
(h-1) a cycloalkyl ring, or
(h-2) a heteroatom-containing heterocyclic ring; or
(II) when R¹¹ is other than hydrogen, then R¹² represents:
(a) a straight-chain lower alkyl group,
(b) a branched-chain lower alkyl group,
(c) a cyclic lower alkyl group,
(d) a phenyl group substituted with from zero to five substituents selected from lower alkyl groups or lower alkoxy groups,
(e) a 1-naphthyl group substituted with from zero to seven substituents selected from lower alkyl groups or lower alkoxy groups, or
(f) a 2-naphthyl group substituted with from zero to seven substituents selected from lower alkyl groups or lower alkoxy groups; and
R¹³ represents:
(a) hydrogen,
(b) a lower alkyl group, or
(c) a benzyl group.

2. A process for the preparation of an optically active secondary alcohol as claimed in claim 1, wherein R¹ and A in the general formula (1) each comprise no acidic substituents.

3. A process for the preparation of an optically active secondary alcohol as claimed in claim 1 or 2, wherein Ar¹ and Ar² in the general formula (2) each independently represent a phenyl group having from two to five substituents selected from the group consisting of straight-chain or branched-chain lower alkyl groups, halogens, and lower alkoxy groups provided that at least two of the said substituents are straight-chain or branched-chain lower alkyl groups.

4. A process for the preparation of an optically active secondary alcohol as claimed in any of claims 1 to 3, wherein in the general formula (1), n is 0; A is CH₂NR²R³; R¹ is a phenyl group having at 3-position a nitro group, an amino group, or N(CH₂C₆H₅)SO₂R¹⁶ wherein R¹⁶ is a methyl group or a benzyl group, and at 4-position hydrogen, halogen, or a benzyloxy group; R² is an acyl group, an alkyloxycarbonyl group, or a benzyl group; and R³ is an ethyl group which is bonded at its end to the fused tricyclic ring group via an oxygen atom.
